# EUROPEAN PATENT APPLICATION

(11) **EP 3 511 425 A1**
(43) Date of publication of application: **17.07.2019**
(21) Application number: 19155189.4
(22) Date of filing: 12.07.2013
(51) Int. Cl.: C12Q 1/68, A61K 39/00, A61K 35/17, G01N 33/569, C12Q 1/6874, C12Q 1/6881, C12Q 1/6888

(54) **PERSONALIZED CANCER VACCINES AND ADOPTIVE IMMUNE CELL THERAPIES**

(30) Priority: 12.07.2012 US 201261670931 P
(62) Divisional of application: 13817114.5
(71) Applicant: Persimmune, Inc., San Diego, CA 92121 (US)
(72) Inventor: VITIELLO, Maria Antonia, La Jolla, CA 92037 (US)
(74) Representative: Gulde & Partner

(57) **Abstract**

Cancer antigens containing mutations in an expressed gene of cancer cells from a cancer patient are identified. Sequences from cancer cells obtained using a parallel sequencing platform are selected by comparing to the patient's normal genes or to normal genes from an HLA-matched individual. Sequences are further selected by identifying an HLA supertype of the cancer patient and selecting for that HLA supertype, sequences that have a particular amino acid at the mutant position and/or corresponding wide-type position in the effected gene. Peptides containing cancer antigens are optionally tested for binding to HLA antigens of the cancer patient. Peptides containing the cancer antigens are evaluated for activating cytotoxic T lymphocytes (CTL) cell lines from the cancer patient or from an HLA-matched donor. The cancer antigen(s) identified for a cancer patient are used to prepare a cancer vaccine and to treat the cancer patient.

## Description

### CROSS-REFERENCE TO RELATED APPLICATIONS

This application claims benefit of U.S. Provisional application 61/670,931, filed July 12, 2012, hereby incorporated by reference.

### FIELD OF THE INVENTION

This invention relates to the identification of mutations in expressed genes of cancer cells from cancer patients and use of the mutations to prepare cancer vaccines and adoptive immune cell therapies.

### BACKGROUND OF THE INVENTION

Cancer is the second leading cause of death in the US. The estimates for 2010 are that approximately 570,000 people will die from cancer and 1.5 million new cases will be diagnosed (1). For early stage cancers (those that have not spread to the lymph nodes and are non-metastatic) surgical removal is a very effective treatment. However, for more advanced cases, standard, non-specific cancer treatments (chemo and radiotherapy) are used. These treatments affect many healthy cells and result in elevated toxicity. One of the most important principles of medical ethics, "primum non nocere" (first do no harm), is often not applicable in the treatment of cancer, where patients are submitted to very toxic therapeutic protocols that are effective in only a percentage of treated individuals. Moreover, even individuals that initially are treated successfully are at risk for relapses, and become more difficult to treat for each succeeding relapse.

The idea of employing the adaptive immune system to kill cancer cells without harming normal cells has been a goal for many decades (for review see Dunn 2002 (2)). To become a cancer cell, a healthy cell undergoes multiple somatic mutations (3, 4). Such mutations may be targets of the adaptive immune system, which performs the function of recognizing and eliminating small variations from self. The possibility of using immunotherapy for successfully treating cancers is gaining support due to findings that (a) tumor-specific lymphocytes can be isolated from patients with tumors (6, 7); (b) the presence of tumor-specific lymphocytes infiltrating the tumor (or in circulation) correlate with good prognosis (8); and (c) antigens recognized by T lymphocytes on the tumor cell have been identified (9, 10, 12). Also related are (a) the demonstrated effectiveness of adoptive cellular immunotherapy for cytomegalovirus (CMV) infection and lymphomas associated with the Epstein Barr virus (EBV) in patients that underwent bone marrow transplantation (BMT) (11), and (b) the success of adoptive cell therapy in the treatment of patients with metastatic melanomas (7).

However, tumor antigen identification and its translation to immunotherapy still face many problems. Therefore, being able to define antigens in an easier and more efficient manner is an advantage. The process of identifying and utilizing antigens, as described herein, allows for the individualized diagnosis and treatment of patients which increase the likelihood of treatment success.

### SUMMARY OF THE INVENTION

Provided herein are methods to identify mutations in expressed genes of cancer cells from cancer patients and to use the mutations to prepare cancer vaccines and adoptive immune cell therapies for treating the cancer patients. Nucleic acid sequences from the cancer cells are obtained by a parallel sequencing platform, which employs parallel processing of the nucleic acid of cancer cells leading to sequence reads and mapping of the sequence reads to a database with reference gene sequences. In some embodiments, the parallel sequencing platform employs certain filtering of the sequencing results such as a depth of coverage less than 20 x and/or by not filtering with a base alignment quality (BAQ) algorithm.

Mutant sequences which code for all or a portion of an expressed gene are identified as those which have a mutant position amino acid which substitutes for a wildtype position amino acid located at the same position in the wildtype sequence of the protein.

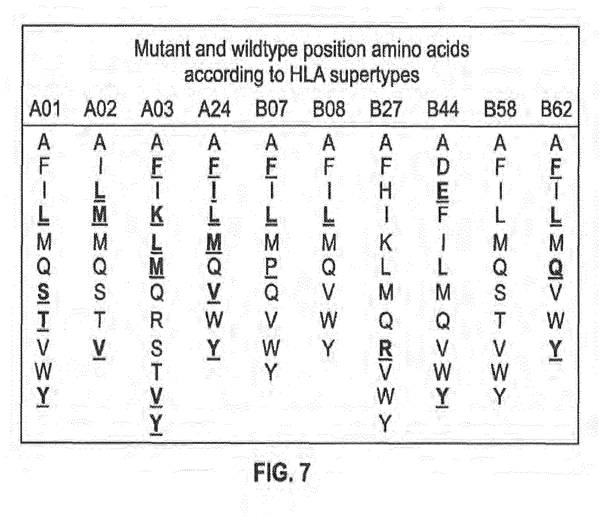

A further selection of mutant sequences can be achieved by identifying an HLA class and/or HLA supertype of the cancer patient and then selecting one or more amino acids for the particular HLA class and/or HLA supertype as the mutant position amino acid and/or wildtype position amino acid. Candidate mutant position amino acid and/or wildtype position amino acid for each HLA class and/or HLA supertype are shown in FIG. 7. Alternatively, one can ignore the HLA class and/or HLA supertype of the individual and make a further selection using one or more amino acids selected from the group consisting of tyrosine, phenylalanine, leucine, isoleucine, methionine, valine and alanine.

In accordance with the invention, peptides containing mutant sequences of interest are evaluated for their ability to bind to HLA histocompatibility antigens of the cancer patient. This can be carried out in silico using computer-based algorithm(s) for predicting HLA binding peptides. Alternatively, or in addition, the ability to bind to HLA histocompatibility antigens is carried out by synthesizing the peptides and testing them for binding to HLA histocompatibility antigens. The testing of sequences for binding to HLA histocompatibility antigens is not a requirement but may be used to narrow the set of potential cancer antigens prior to further testing.

In further embodiments, peptides containing the mutant sequences of interest may be synthesized and evaluated for activating cytotoxic T lymphocytes (CTL) cell lines prepared from the cancer patient or from an HLA-matched donor (matched for class and/or supertype). In such cases, the CTL cell lines are obtained by contacting two cell types: mononuclear cells from the cancer patient or from the HLA-matched donor and cancer cells from the cancer patient. The CTL cell lines may be prepared using mononuclear cells that are enriched in CD8⁺ cells and may further include the addition of autologous CD4⁺ T cells and/or dendritic cells from the cancer patient or autologous CD4⁺ T cells and/or dendritic cells from the HLA-matched donor. In embodiments in which only the peptides are used to stimulate the CTLs, donor matching on the HLA class and/or subtype is all that is required.

In another embodiment, methods to identify mutations in expressed genes of cancer cells from cancer patients and to use the mutations to prepare cancer vaccines and adoptive immune cell therapies for treating the cancer patients involves obtaining nucleic acid sequences from the cancer cells by the parallel sequencing platform as discussed. Mutant sequences which code for all or a portion of an expressed gene are identified as those which have a mutant position amino acid which substitutes for a wildtype position amino acid located at the same position in the wildtype sequence of the protein. Peptides containing the mutant sequences of interest and optionally the corresponding wildtype sequence peptides are synthesized and evaluated for activating cytotoxic T lymphocytes (CTL) cell lines prepared from the cancer patient or from an HLA-matched donor. In such cases, the CTL cell lines are obtained by contacting mononuclear cells from the cancer patient or from the HLA-matched donor with cancer cells from the cancer patient. The CTL cell lines may be prepared using mononuclear cells that are enriched in CD8⁺ cells and may further include the addition of autologous CD4⁺ T cells and/or dendritic cells from the cancer patient or autologous CD4⁺ T cells and/or dendritic cells from the HLA-matched donor.

Specifically, in one aspect, the invention provides method of identifying cancer antigens for preparing a cancer vaccine, comprising
a) obtaining a plurality of mutant sequences from the nucleic acid of cancer cells from a cancer patient, said mutant sequences coding for all or a portion of an expressed gene and wherein the mutant sequences each have a mutant position amino acid which substitutes for a wildtype position amino acid, or other mutation (e.g., insertion or deletion), located at the same position in the wildtype sequence of the protein, wherein said mutant sequences are obtained using a parallel sequencing platform, said parallel sequencing platform employing parallel processing of said nucleic acid of cancer cells leading to sequence reads and mapping of the sequence reads to a database with reference gene sequences; and
b) selecting mutant sequences from those identified in step a) by identifying an HLA class or supertype of the cancer patient and then selecting an amino acid for said HLA class or supertype as the mutant position amino acid and/or wildtype position amino acid using FIG. 7, wherein cancer antigens for preparing a cancer vaccine are identified.

In another aspect the invention provides a method of identifying cancer antigens for preparing a cancer vaccine, comprising
a) obtaining a plurality of mutant sequences from the nucleic acid of cancer cells from a cancer patient, said mutant sequences coding for all or a portion of an expressed gene and wherein the mutant sequences each have a mutant position amino acid which substitutes for a wildtype position amino acid, or other mutation (e.g., insertion or deletion), located at the same position in the wildtype sequence of the protein, wherein said mutant sequences are obtained using a parallel sequencing platform, said parallel sequencing platform employing parallel processing of said nucleic acid of cancer cells leading to sequence reads and mapping of the sequence reads to a database with reference gene sequences; and
b) identifying at least one mutant sequence for preparing a cancer vaccine from the plurality of mutant sequences obtained in step a) by determining that at least one peptide encoded by the at least one mutant sequence binds to an HLA class or supertype of the cancer patient.

In another aspect, the invention provides a method for predicting the effectiveness of an therapy described herein (e.g., adoptive transfer and vaccination) by (a) identifying at least one mutant nucleic acid sequence from the patient (e.g., from the cancer of the patient), wherein the mutant sequence codes for all or a portion of an expressed gene and wherein the encoded protein or peptide comprises a mutant amino acid substitution or other mutation relative to the wildtype, and (b) determining the binding capacity of the mutant peptide with the HLA class or supertype of the patient, wherein the strength, amount, and/or capacity for HLA binding is indicative of the patient's likely responsiveness to therapy, wherein greater binding indicates higher responsiveness. The binding capacity may be determined either using in silico techniques, such as the ones described herein, or by in vitro testing in which the identified mutant peptide is assess for binding to any one or more of the patient's HLA-expressing cells, as described herein, or to another cell expressing the same HLA class or supertype as the patient.

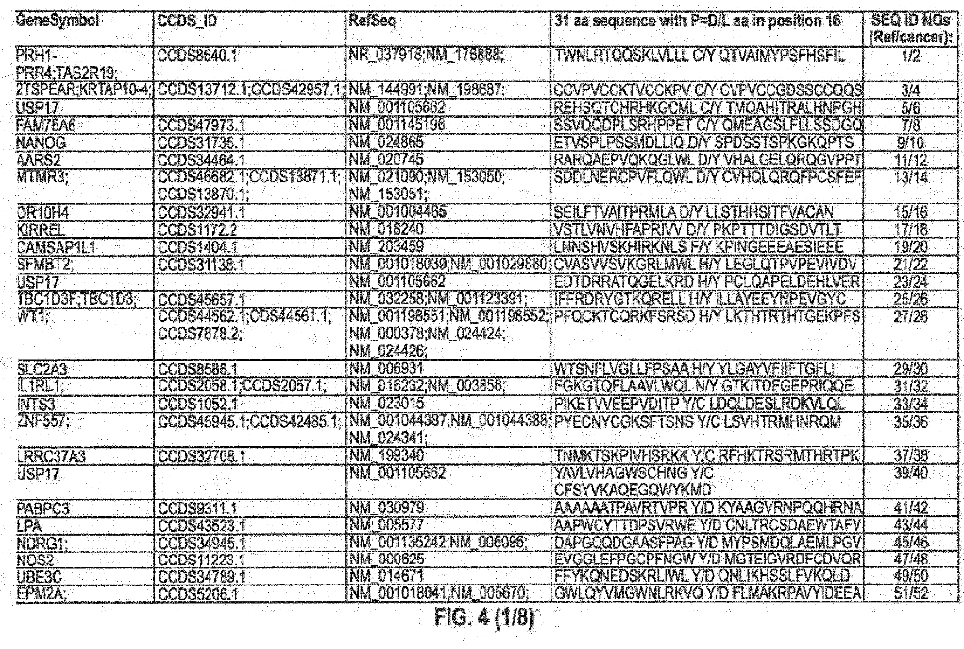

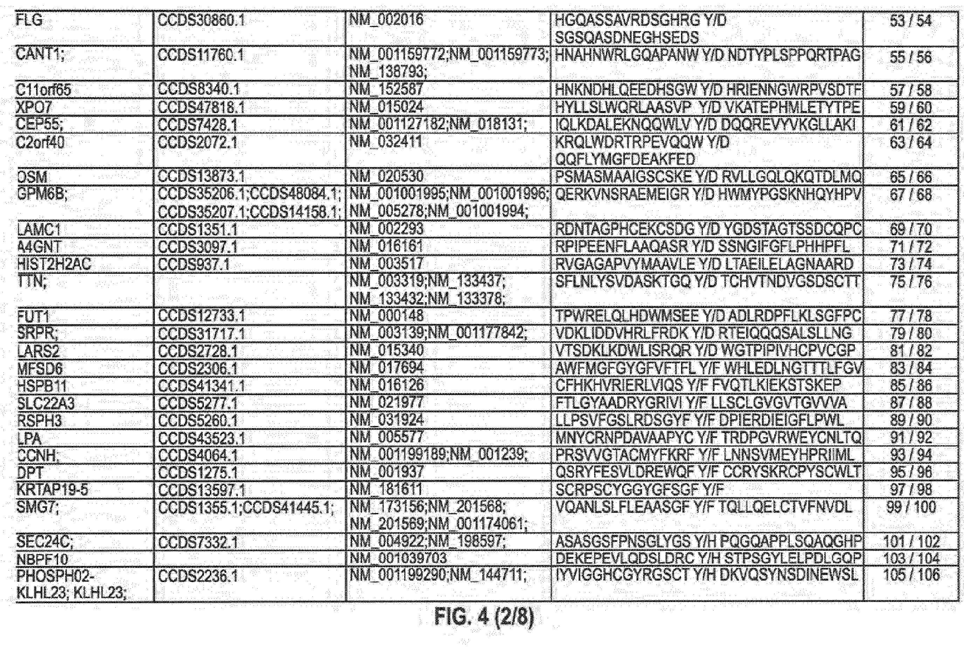

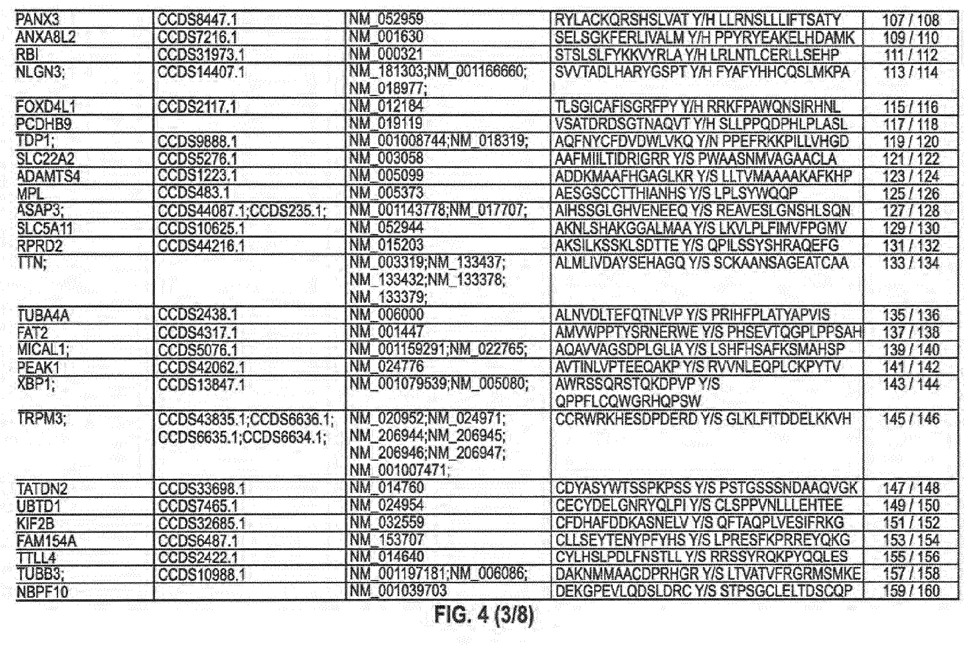

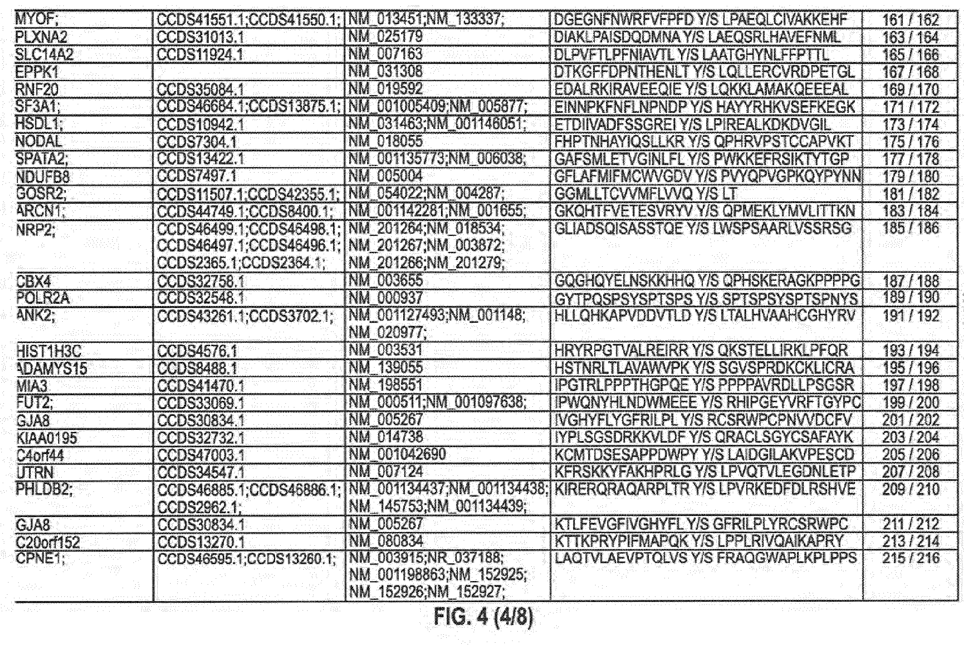

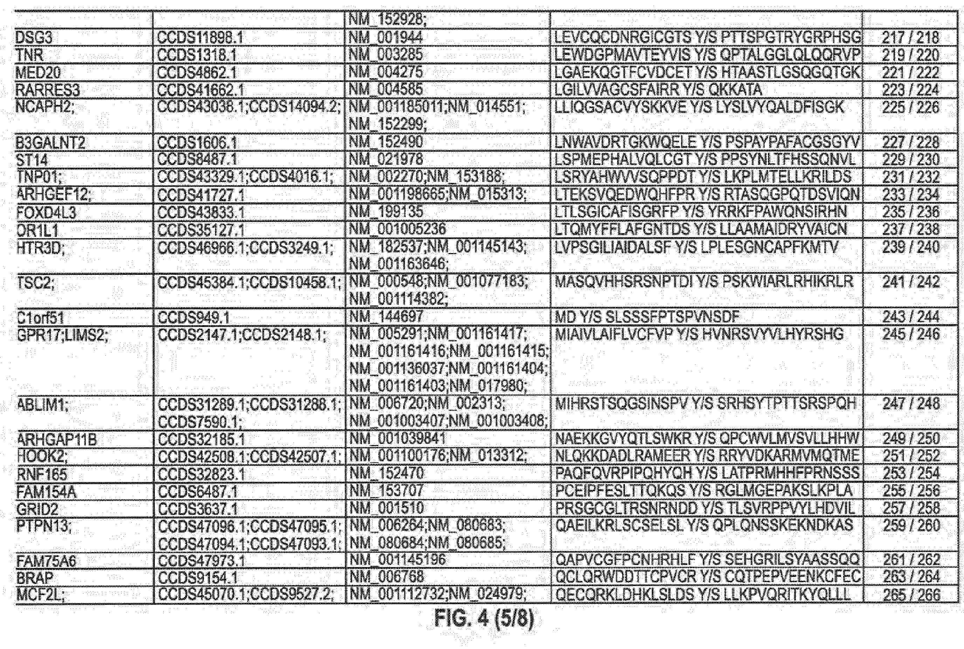

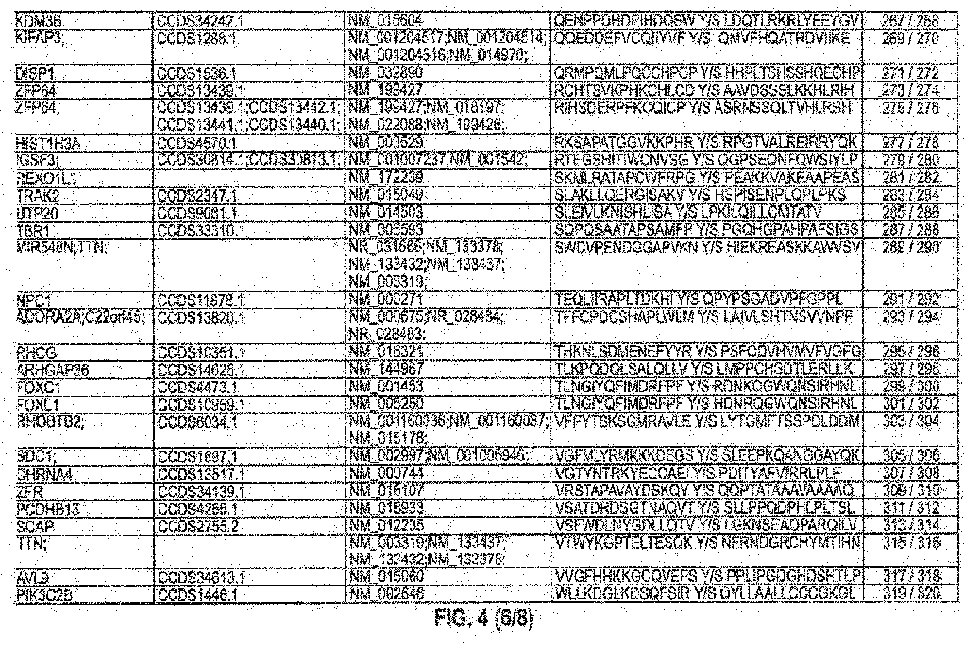

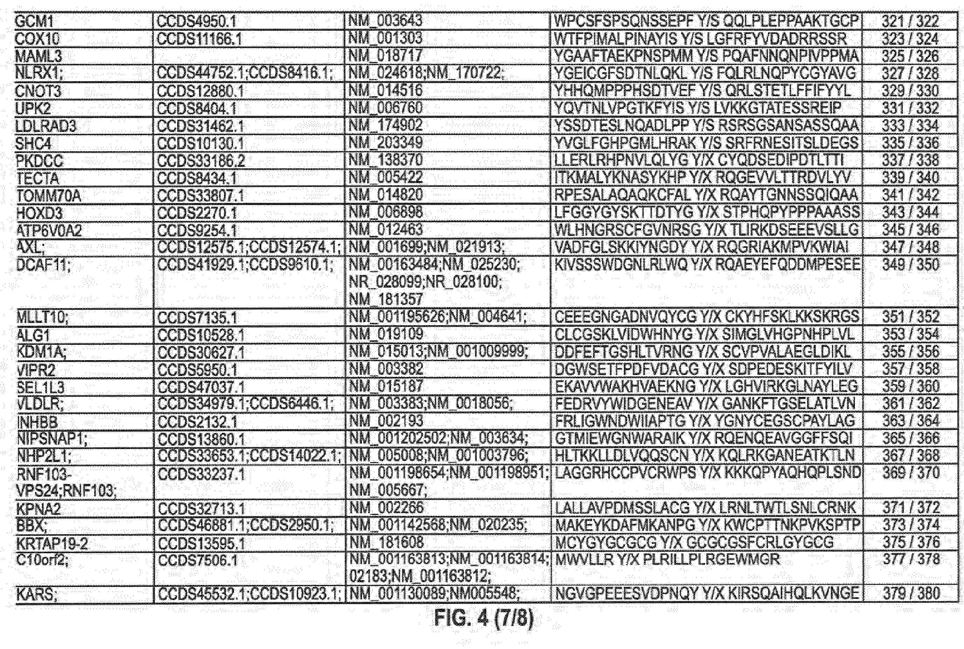

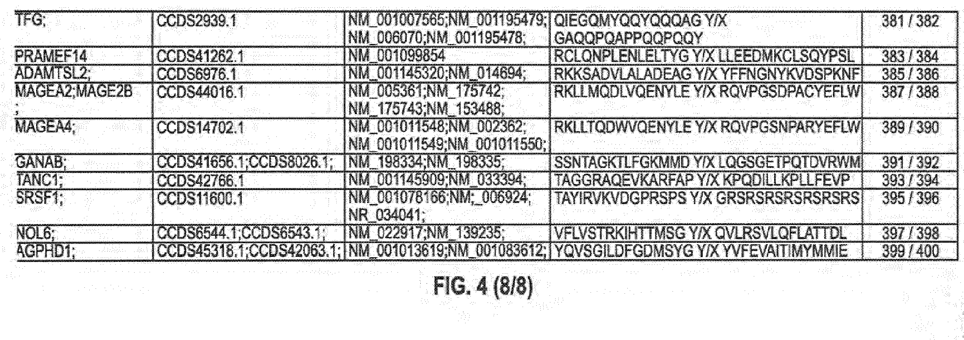

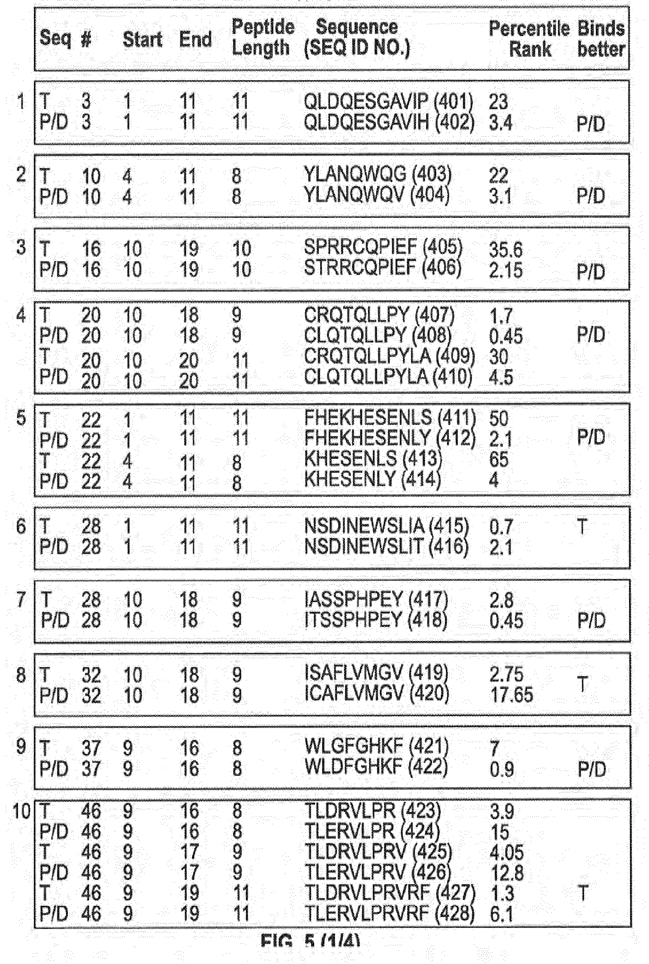

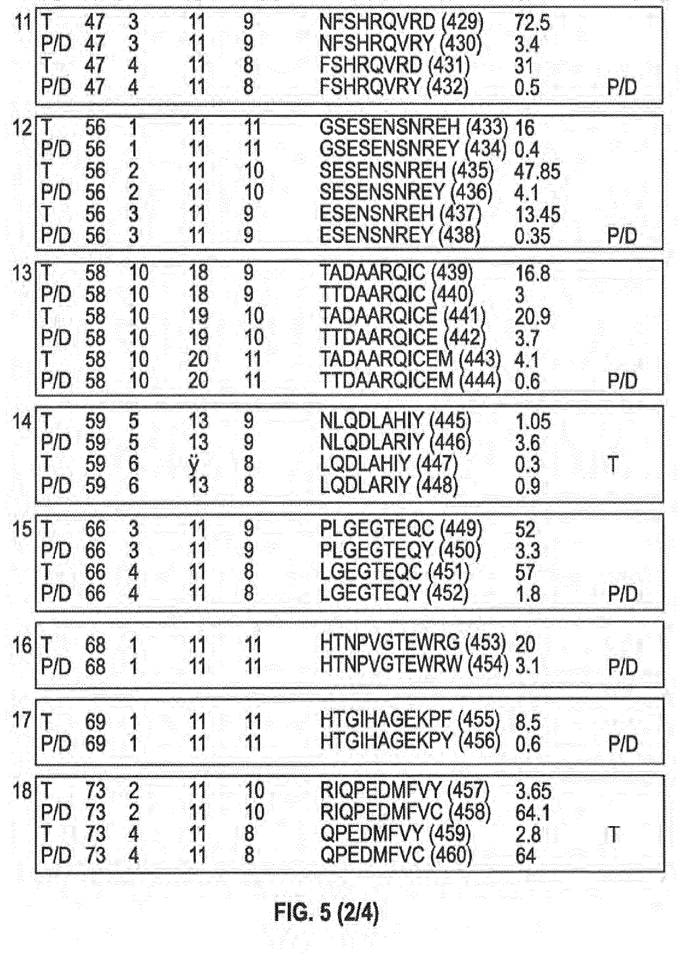

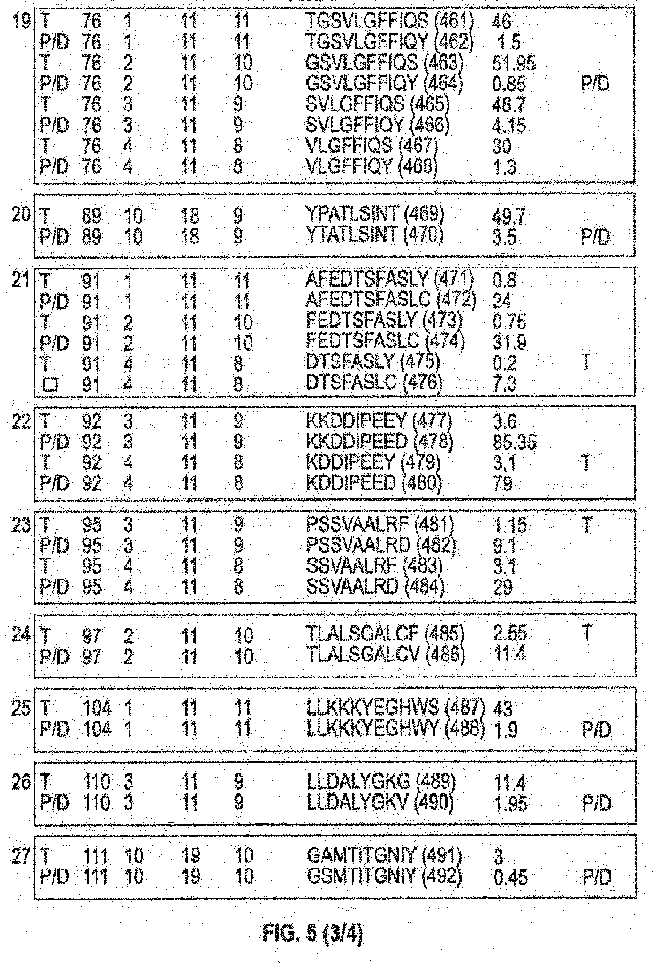

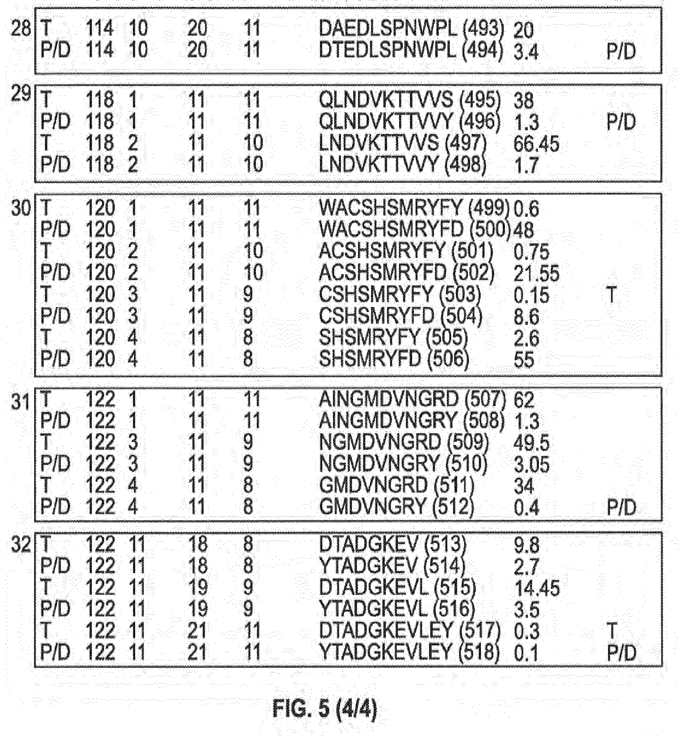

Also provided herein are mutant sequences associated with cancer, wherein the sequence is selected from any disclosed in FIGs. 4 and 5.

Further provided are cancer vaccines prepared using one or more of the cancer antigens identified by any of the above methods. The cancer vaccine may be a polypeptide that contains one or more of the cancer antigens or may be a nucleic acid that encodes for expression of one or more of the cancer antigens.

Yet further provided is a method of treating a cancer patient by identifying cancer antigens from nucleic acid obtained from cancer cells as described by any of the methods above and by preparing a vaccine with one or more of the cancer antigens. The patient is treated by administering the vaccine to generate CTLs in the patient and/or by administering CTL cell lines prepared in vitro by contacting mononuclear cells of the cancer patient or an HLA-matched donor with the cancer antigen vaccine, or by immunizing the donor with the vaccine and transferring immunized donor CTLs to the cancer patient. The contacting may include the addition of autologous CD4+ T cells and/or dendritic cells from the cancer patient or from the HLA-matched donor.

In another embodiment, cancer patients are treated by identifying cancer antigens from nucleic acids obtained from cancer cells as described by any of the methods above, by preparing a vaccine with one or more of the cancer antigens, by contacting mononuclear cells of the cancer patient or an HLA-matched donor (matched for HLA class and/or supertype) with the cancer antigen vaccine to stimulate CTL cell lines, and by administering the CTL cell lines to the cancer patient to treat the cancer . The contacting may include the addition of autologous CD4+ T cells and/or dendritic cells from the cancer patient or from the HLA-matched donor.

### BRIEF DESCRIPTION OF THE DRAWINGS

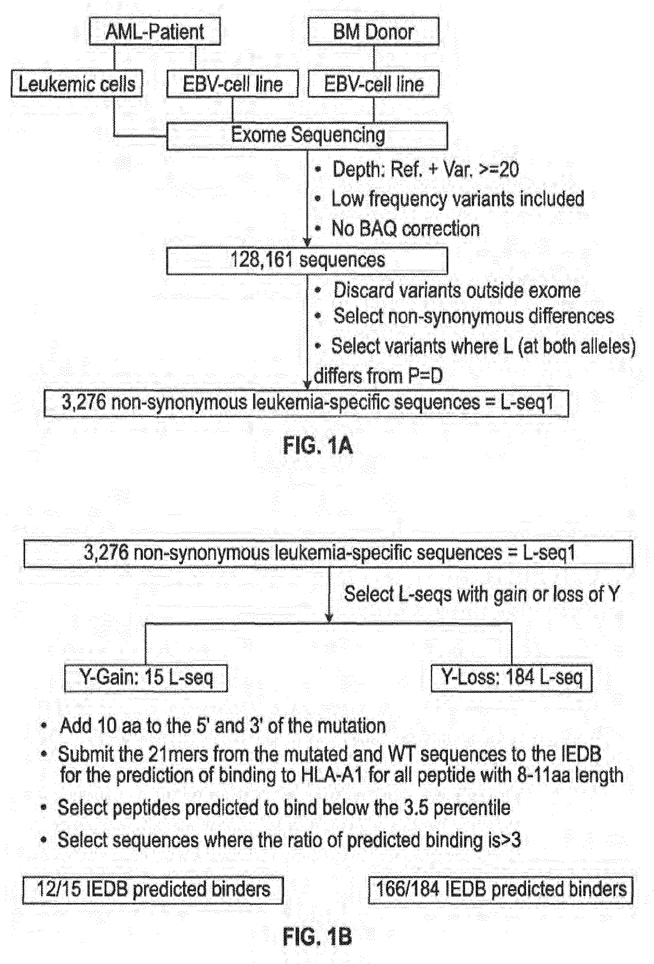

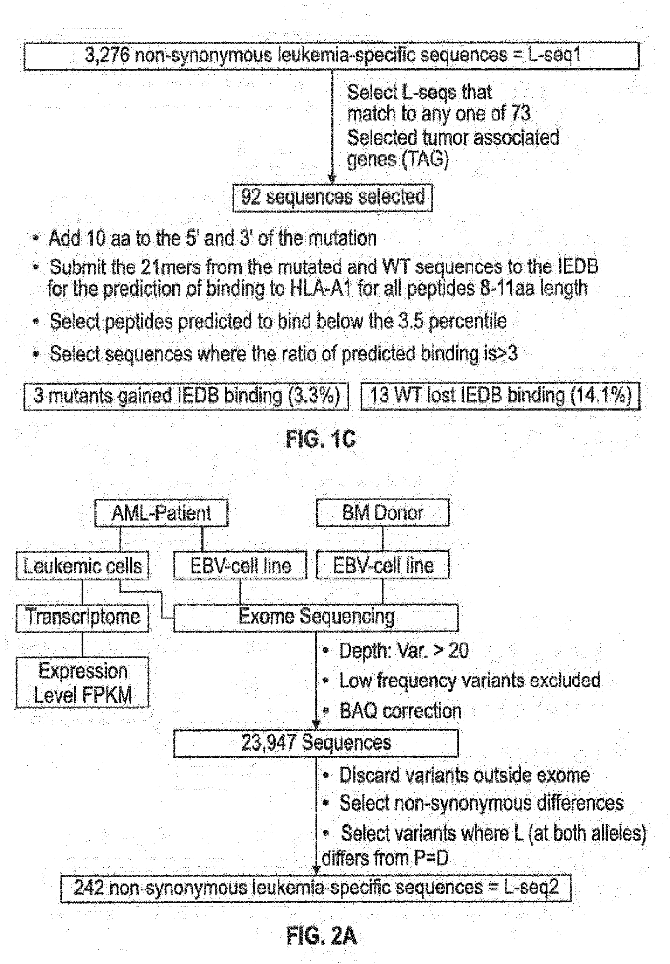

FIG. 1a-c is a set of flow diagrams relating to the identification of mutations in acute myelogenous leukemia (AML) cells from an HLA-A1 patient. FIG. 1a shows the initial selection of mutant sequences determined by applying next generation sequencing to nucleic acid prepared from leukemic cells, EBV-transformed cells from the cancer patient, and EBV-transformed cells from an HLA-matched donor. An initial set of 128,161 sequences were obtained and from that, a set of 3,276 (designated "L-seq1") were selected which have a change of a coded amino acid in a gene from the cancer cell compared to that in EBV cells from the patient and the donor. FIG. 1b shows further selection of the mutant sequences from the L-seq1 set for mutants that involve either a gain or loss of a tyrosine in the protein from the patient cancer cells. Peptide sequences containing the tyrosine involved mutant sequence were tested for binding to HLA-A1 antigens in silico using HLA peptide binding software. FIG. 1c shows selection of the mutant sequences from the L-seq1 set for proteins that have been reported to be associated with cancer. Peptide sequences containing the mutations present in genes that are associated with cancer were tested for binding to HLA-A1 antigens in silico using HLA peptide binding software. The abbreviated terms are as follows: L: leukemia; P: patient; D: donor; ref: reference; var: variant; IEDB: immune epitope database.

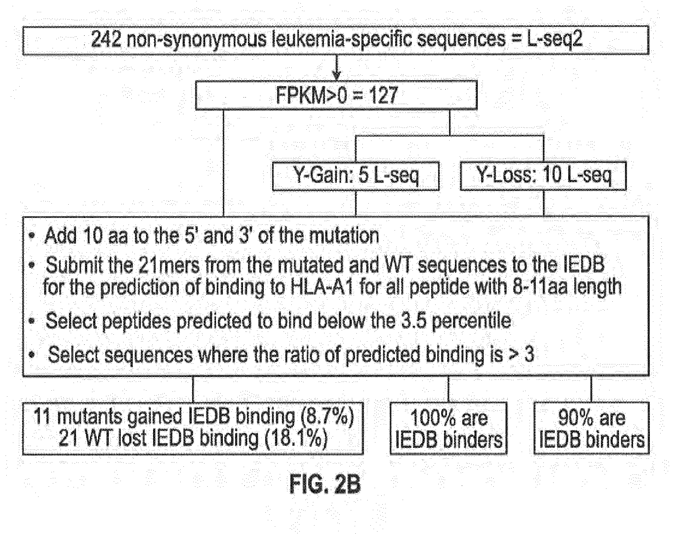

FIG. 2a-b is a second set of flow diagrams relating to the identification of mutations in the same acute myelogenous leukemia (AML) cells and donor cells used in FIG. 1a-c. FIG. 2a shows selection of an initial set of 23,947 sequences and from that, a set 242(designated "L-seq2") were selected which have a change of a coded amino acid in a gene from the leukemia cell compared to that in EBV cells from the patient and the donor. FIG. 2b shows further selection of the mutant sequences from the L-seq2 set for mutants that associated with genes expressed by the leukemia cells (FPKM> 0) or involve either a gain or loss of a tyrosine in the protein from the patient leukemia cells. Peptide sequences containing the tyrosine involved mutant sequence or containing the expressed mutant sequence were tested for binding to HLA-A1 antigens in silico using HLA peptide binding software. The acronyms referred to are as follows: L: leukemia; P: patient; D: donor; ref: reference; var: variant; IEDB: immune epitope database.
FIG. 3 shows the difference in amino acid distributions for the patient and donor (P=D) EBV cells and the leukemia cells for the 3,276 sequences in the L-seq1 set (L differs at both alleles from P=D). The highlighted amino acids are involved in binding to HLA-A1.
FIG. 4 identifies proteins in L-seq1 with amino acid changes involving a tyrosine and provides a 31 amino acid peptide sequences for patient and donor (P/D) and corresponding leukemic cell (L) of the cancer patient.
FIG. 5 identifies peptides from 32 proteins from L-seq2 and provides the sequence in leukemic cancer cells (T) and the corresponding sequence in the patient and donor (P/D). Also provided is an HLA-A1 binding ranking for each sequence.
FIG. 6 identifies 73 tumor associated genes.
FIG. 7 identifies various HLA supertypes and mutant and wildtype position amino acids that can be used for selecting mutant sequences identified by NGS.

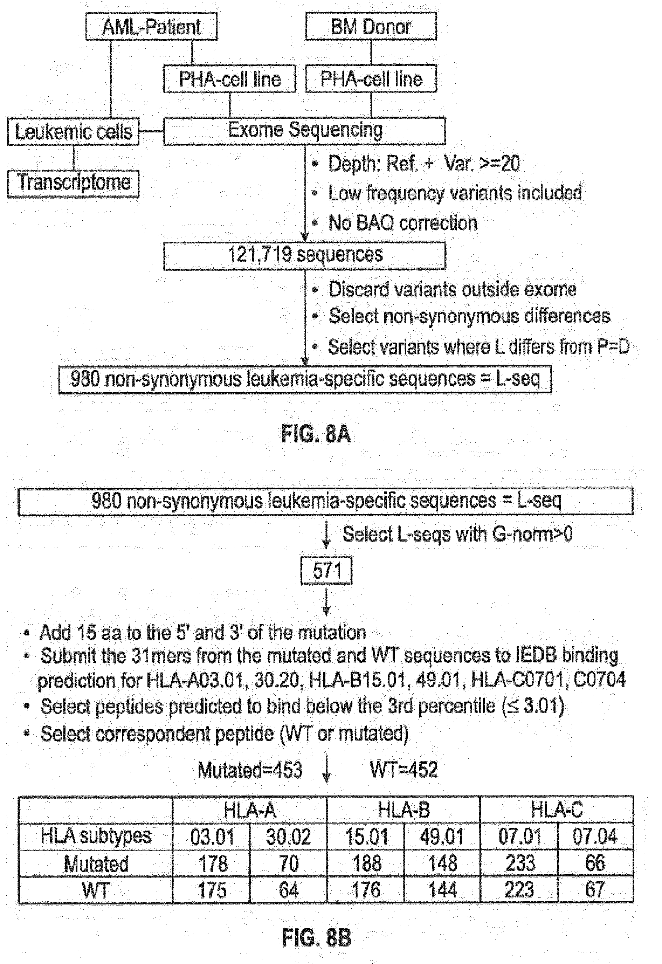

FIG 8 is a set of flow diagrams relating to the identification of mutations in acute myelogenous leukemia (AML) cells from patient #2 as described in Example 3. FIG. 8a shows the initial selection of mutant sequences determined by applying next generation sequencing to nucleic acid prepared from leukemic cells, PHA-stimulated lymphocytes from the cancer patient, and PHA-stimulated lymphocytes from an HLA-matched donor. An initial set of 121,719 sequences were obtained and from that, a set of 980 (designated "L-seq") were selected which have a change of a coded amino acid in a gene from the cancer cell compared to that in PHA-stimulated lymphocytes from the patient and the donor. FIG. 8b shows further selection of the mutant sequences from the L-seq that are expressed as measured by transcriptome analysis. The L-seq set of 980 31-mers were tested for binding to various HLA subtypes in silico using HLA peptide binding software. It was predicted that 571 of those 31-mers would exhibit HLA binding activity to at least one HLA subtype by at least one region of the peptide. There were a total of 905 predicted binding regions, of which 452 were wild-type sequences and 453 were mutated sequences. The Table insert shows the number of peptides predicted to bind to each specific HLA. The total number of HLA-binding sequences in table insert is greater than 571 because several peptides bound to more than one HLA allele.

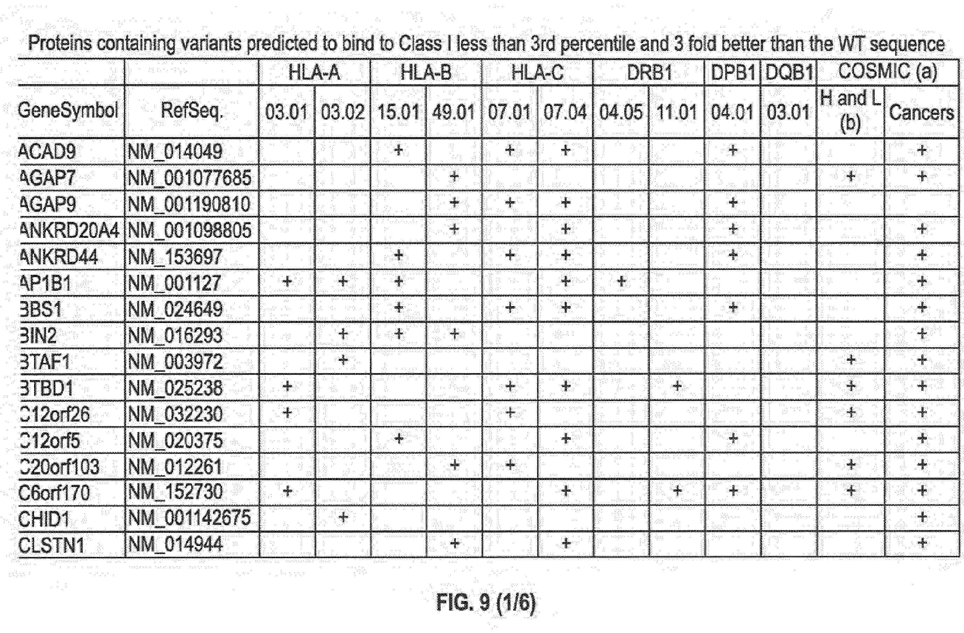

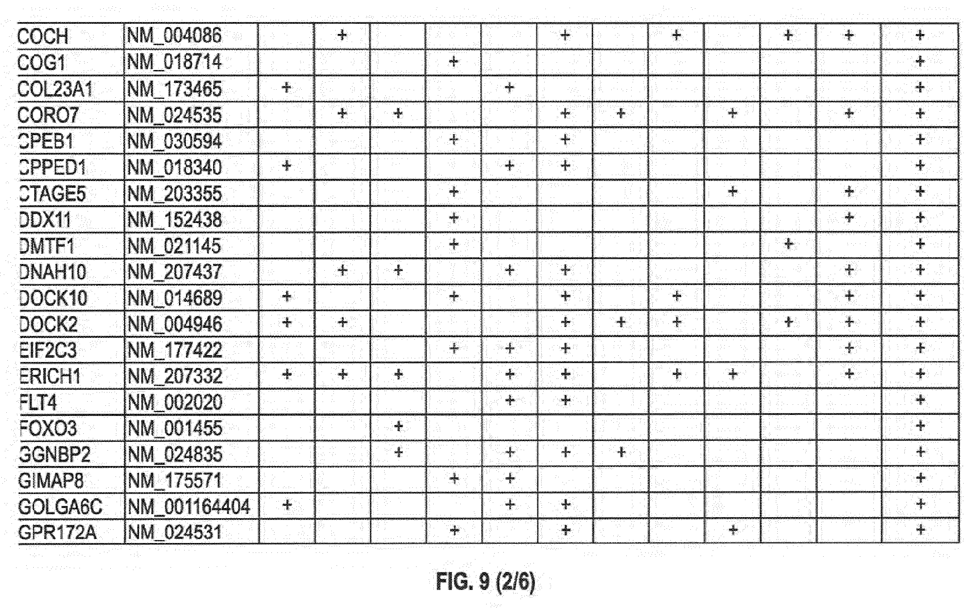

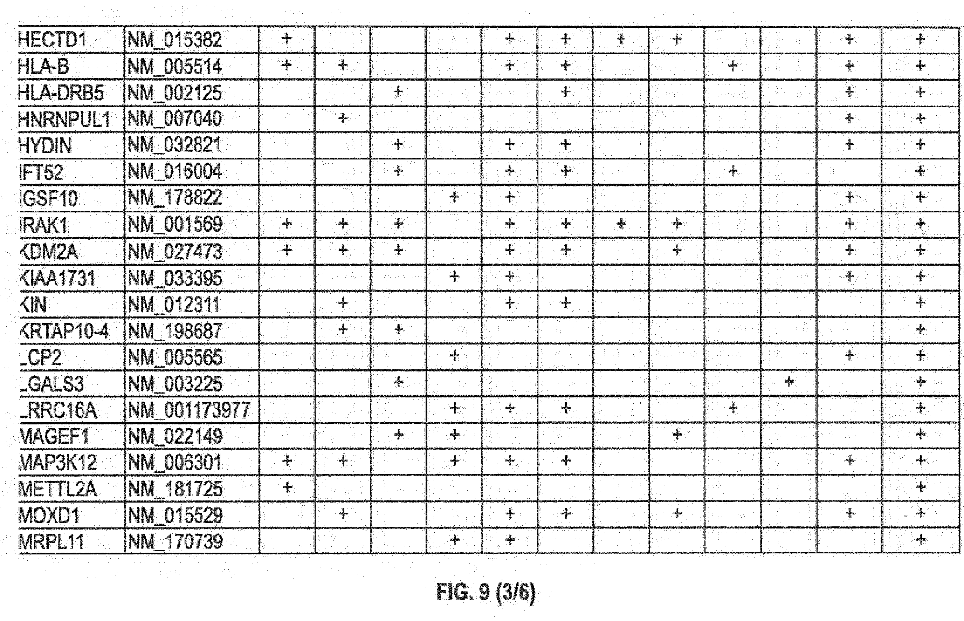

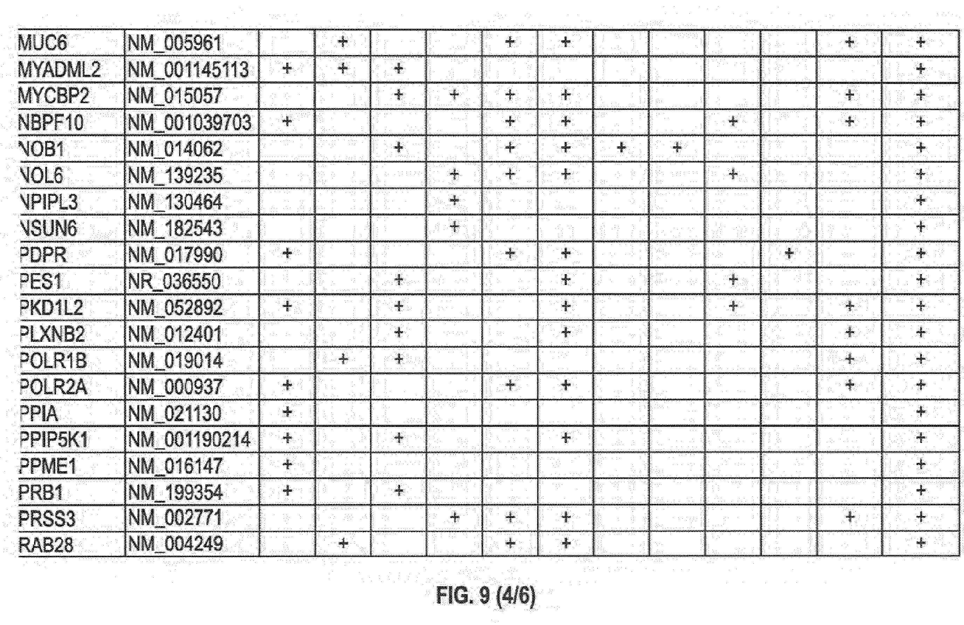

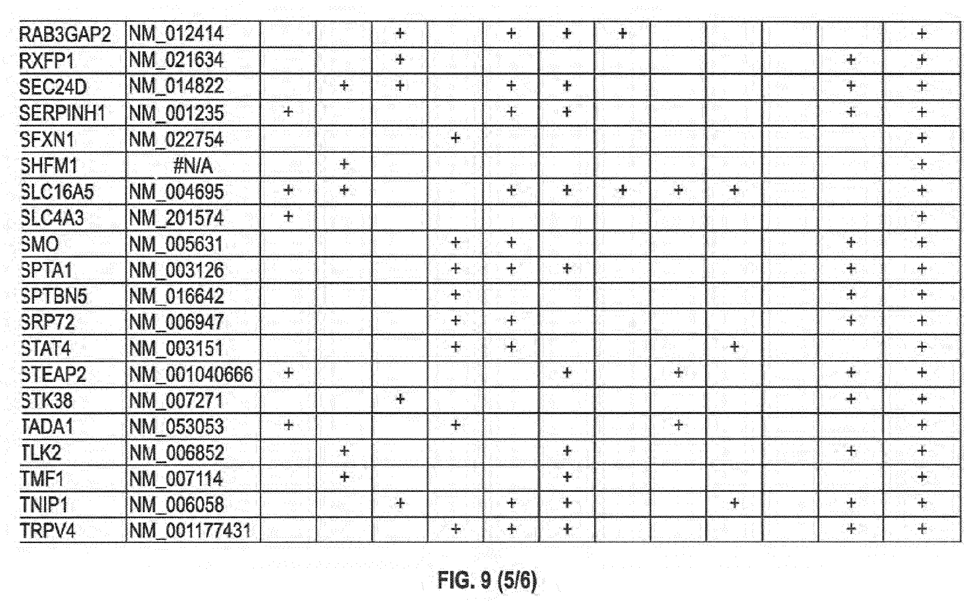

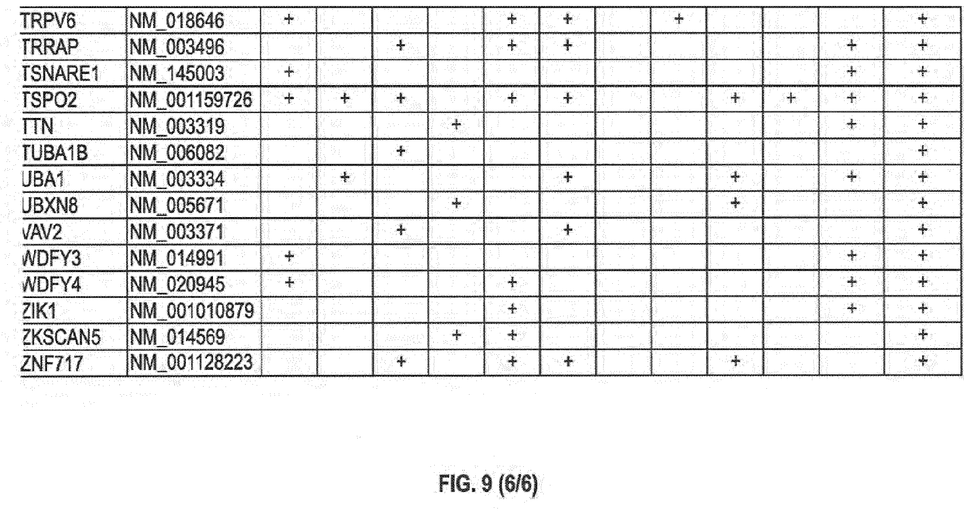

FIG 9 shows the list of mutated proteins where the mutation resulted in a predicted binding affinity of less than 3% and a 3-fold increase of binding as compared to the equivalent wild type peptide. Each line represents the binding analysis of one 31mer containing the mutation. Binding to more than one MHC might indicate that within the 31mer, more than one peptide sequence is responsible for the binding. (a) Catalog Of Somatic Mutations=COSMIC and (b) H and L= hematopoietic and lymphoid tissues.

### DETAILED DESCRIPTION OF THE INVENTION

Identification of T cell antigens that can be used for immunotherapy still faces many problems. The search for the antigens has been very laborious and after an antigen is discovered, there is a strong tendency to generalize an antigen's applicability, assuming that an antigen that works for one individual will be an antigen to treat the same kind of tumor in another individual. This tendency is based on the notion that, to be useful, an antigen needs to work in the broadest possible patient population. This practice of "generalizing" tumor antigens does not account for the fact that each tumor expresses many unique antigens and that an individual's MHC molecules restrict the T cell response. Therefore, an antigen that is good for treating one individual might not be ideal for another person. Moreover, many of the antigens identified so far are normal tissue specific antigens, raising the problem of autoimmunity.

The present paradigm for the discovery and immunotherapeutic application of tumor antigens is to look for "universal" or common tumor antigens, i.e., antigens that induce good immunity in the majority of individuals and use these antigens for vaccination purposes. The results obtained with these approaches have been disappointing. The reality is that the best immune response will differ for each patient affected by a tumor. Only after the immune repertoire is identified for many individuals, using a systematic and unbiased approach, would it be possible to ascertain common patters of immunogenic mutations. For example, a finding that some genes were affected by common clusters of mutations may lead to the application of a less individualized therapy.

Provided herein are methods to identify the available mutations that constitute tumor-associated and/or tumor-rejection antigens in cancer cells from individual cancer patients and to use the antigens to immunize T cells from the patient or HLA-matched donor T cells to recognize and kill the cancer cells. To this end, next generation sequencing will be used to sequence the transcriptome and the exome of the cancer cells as well as the exome of EBV lymphoblasts, or PHA stimulated T cells from the cancer patient. In particular cancers that employ bone marrow transplantation (e.g. in leukemia), the sequenced exome from the cancer patient cells also can be compared to the exome from EBV lymphoblasts or PHA-stimulated cells from an HLA-matched bone marrow donor.. This comparison will yield a comprehensive representation of the genes that will be translated into peptides and utilized in adoptive transfer therapy.

Next generation sequencing strategies make it possible to perform extensive molecular characterization of tumor cells in an attempt to identify genes involved in transformation. The information that can be obtained includes the copy number, level of expression, and somatic mutations.

As used herein, the terms "next generation sequencing" ("NGS"), "second generation sequencing" and "massively parallel sequencing" encompasses high-throughput sequencing methods that parallelize the sequencing process, producing thousands to millions of sequences at a time (16, 17). The number of sequences produced by parallelized sequencing is typically greater than 10,000, more typically greater than 100,000 and most typically greater than 1 million. NGS design is different from that of Sanger sequencing, also known as "capillary sequencing" or "first-generation sequencing," which is based on electrophoretic separation of chain-termination products produced in individual sequencing reactions.

Although NGS platforms differ in engineering configurations and sequencing chemistry, common to most is the use of spatially separated, clonally amplified DNA templates or single DNA molecules processed in parallel by use of a flow cell. The massive quantity of output from parallel processing is transformed from primary imaging output or detection output into sequence. A package of integrated algorithms performs the core primary data transformation steps: image analysis, intensity scoring, base calling, and alignment of subsequence reads to a reference sequence. Reference sequences include the human reference genome NCBI37/hg19 sequence, which is available from Genome Bioinformatics Group of the University of California Santa Cruz (available on the world wide web). Other sources of public sequence information include GenBank, dbEST, dbSTS, EMBL (the European Molecular Biology Laboratory), and the DDBJ (the DNA Databank of Japan). Thus, NGS refers to a parallel sequencing platform that employs parallel processing of nucleic acid leading to sequence reads and mapping of the sequence reads to a database with reference gene sequences.

The present methods for selecting cancer specific sequences using NGS have the potential to identify rare mutants which may be lost when more extensive sequence filtering is used. Even a single tumor may contain different types of cancer cells and different types of stem cells that continue to replicate themselves and also give rise to the these types of cancer cells. The exclusion of filtering between the step of sequence alignment and selection of mutant sequence will likely result in more false positives but will provide rare sequence mutations that need to be immunized against to treat the cancer.

Sequence data from NGS systems can be filtered to provide early selective criteria which aids in accuracy. A common filter is the "depth of coverage," "sequencing coverage" or "coverage depth," which is the average number of times a given DNA nucleotide is represented in sequence reads (stated differently, this is the average number of reads covering any particular base) (see, e.g., Nielsen et al., Nature Reviews Genetics 12:443 2011) The greater the coverage, the greater the likelihood of accurately calling a sequence variation. For detection of cancer mutations as disclosed herein, a depth of coverage of < 20 x is used, however, more preferable coverage is < 15 x, < 10 x, < 7 x, < 5 x, < 4 x, < 3 x, < 2 x, and 1 x. Depth of coverage also can be represented in the case of mutations as the average number of reads for the reference and variant combined for any particular base (e.g., the reference is the patient or donor EBV B cell sequences and the variant is the cancer cell sequences. A reference + variant(s) >=20 can be used to identify with confidence low depth for the cancer mutation (e.g. 18 x for the reference plus 2 x for the mutation).

Another filter is base alignment quality (BAQ), an approach that accurately measures the probability that a read base has been wrongly aligned (e.g., see Li, Bioinformatics; 27(8): 1157-1158 [2011]). Base alignment quality (BAQ) computation is turned on by default and adjusts depth of coverage values to better simulate local realignments.. BAQ is a Phred-like score representing the probability that a read base is misaligned; it lowers the base quality score of mismatched reads that are near indels. This is to help rule out false positive SNP calls due to alignment artifacts near small indels. The filter can be adjusted by utilizing its parameters. One can disable BAQ with the -B parameter, or perform a more sensitive BAQ calculation with -E.

CodonCode Corporation (58 Beech Street Dedham, MA 02026) offers Windows, Mac OS X, and Unix versions of Phrap, Phred, and Cross_match, Phil Green's programs for sequence assembly, quality base calling, and fast sequence comparisons. CodonCode also offers Unix and Linux versions of Consed, David Gordon's contig editor and automated finishing tool for Phred and Phrap. After calling bases, Phred examines the peaks around each base call to assign a quality score to each base call. Quality scores range from 4 to about 60, with higher values corresponding to higher quality. The quality scores are logarithmically linked to error probabilities, as shown in the following table:

| **Phred quality score** | **(Probability that the base is called wrong** | **Accuracy of the base call** |
|---|---|---|
| 10 | 1 in 10 | 90% |
| 20 | 1 in 100 | 99% |
| 30 | 1 in 1,000 | 99.9% |
| 40 | 1 in 10,000 | 99.99% |
| 50 | 1 in 100,000 | 99.999% |

Another sequence data filter is probabilistic modeling, which employs algorithms to filter low frequency variants.

NGS sequencing technologies include pyrosequencing, sequencing-by-synthesis with reversible dye terminators, sequencing by oligonucleotide probe ligation and real time sequencing. NGS sequencing technologies are available commercially, such as the sequencing-by-hybridization platform from Affymetrix Inc. (Sunnyvale, Calif.) and the sequencing-by-synthesis platforms from 454 Life Sciences (Bradford, Conn.), Helicos Biosciences (Cambridge, Mass.), Illumina/Solexa (Hayward, Calif.), and the sequencing-by-ligation platform from Life Technologies (San Diego, Calif.). Several companies provide NGS sequencing direct to the consumer for $10,000 or less.

The first well known example of NGS sequencing technology is the 454 (Roche) Life sequencing system (e.g. see Margulies, M. et al. Nature 437:376-380 [2005]). In 454 sequencing, the DNA is first sheared into fragments of approximately 300-800 base pairs, and the fragments are blunt-ended. Oligonucleotide adaptors, which serve as primers for amplification and sequencing of the fragments, are ligated to the ends of the fragments. The adapted fragments are attached to DNA capture beads and the beads are individually PCR amplified within droplets of an oil-water emulsion to yield multiple copies of clonally amplified DNA fragments on each bead. The beads are captured in wells where pyrosequencing is performed on each DNA fragment in parallel. Pyrosequencing makes use of pyrophosphate (PPi) which is released upon nucleotide addition, is converted to ATP by ATP sulfurylase in the presence of adenosine 5' phosphosulfate, the ATP then used to convert luciferin to oxyluciferin, generating light that is detected and measured.

Other exemplary NGS sequencing technologies include the Helicos True Single Molecule Sequencing (tSMS) (e.g. see Harris T. D. et al., Science 320:106-109 [2008]); the nanopore sequencing method (e.g. see Soni G V et al., Clin Chem 53: 1996-2001 [2007]); the chemical-sensitive field effect transistor (chemFET) array (e.g., see U.S. Patent Application Publication No. 20090026082); the Halcyon Molecular's method that uses transmission electron microscopy (TEM) (e.g., see PCT patent publication WO 2009/04644); Illumina's sequencing-by-synthesis and reversible terminator-based sequencing chemistry (e.g. see Bentley et al., Nature 6:53-59 [2009]); the SOLiD™ (Life Technologies) sequencing-by-ligation technology (e.g., see McKernan et al., Genome Research 19 (9): 1527-41 (2009]); Pacific Biosciences single molecule, real-time SMRT™ sequencing technology (e.g., see Levene et al., Science. 299 682-686 [2003]); and Life Technologies Ion Torrent single molecule sequencing on a semiconductor chip.

NGS can be used to generate a whole genome sequence or a subset of a whole genome sequence such as an exome sequence or a transcriptome sequence. As used herein, the term "genome sequence" can be referred to as a "genome library" or "genome library of sequences. Likewise, the terms "exome sequence" and "transcriptome sequence" can be referred to as an "exome library" or "exome library of sequences, or "transcriptome library" or "transcriptome library of sequences, respectively.

A whole genomic sequence is obtained by applying NGS to total genomic DNA. The exome represents the protein coding sequences of all genes in the genome. Exome sequence is obtained, for example, by preparing a genomic library and selecting exomic sequence using target-enrichment methods such as hybrid capture or in-solution capture. An exome library may contain intronic or other non-exon sequence as the enrichment may not be total. For example, exome libraries may be only about 50% pure with respect to exon sequences.

The mutant sequences from the cancer cells can be identified by whole transcriptome sequencing using known methods (13-15). The transcriptome is the set of all RNA molecules, including mRNA, rRNA, tRNA, and other non-coding RNA produced in one or a population of cells. The use of next-generation sequencing technology to study the transcriptome at the nucleotide level using cDNA libraries is known as RNA-Seq (e.g., see Wang et al., Nature Rev. Genetics 10(1): 57-63 [2009]). RNA-Seq provides insights at multiple levels into the transcription of the genome as it yields sequence, splicing, and expression-level information leading to the identification of novel transcripts and sequence alterations. Transcriptome sequence is also obtained by target-enrichment methods such as hybrid capture or in solution capture applied to RNA (e.g. oligonucleotide "bait" capture). RNA-seq does not require a reference genome to gain useful transcriptomic information. RNA-Seq approaches (e.g. see SOLiD™ Whole Transcriptome Analysis Kit from Applied Biosystems, Life Technologies Corporation) preserves strand specificity and can interrogate either polyA or ribo-depleted RNA. However, transcriptome sequences can be mapped to the RefSeq's mRNA database of the National Center for Biotechnology Information (NCBI).

The level of expression of gene sequence from NGS can be obtained by evaluation of NGS performed on the transcriptome library. The unit FPKM (expected fragments per kilobase of transcript per million fragments sequenced) provides a numerical value for the estimated proportion of each transcript.

Recent publications report that transcriptome sequencing, analyzing the complementary DNA (RNA-seq) (18), can be performed without the necessity to clone cDNA libraries or even to simply amplify the mRNA (19). The new methods eliminate steps that otherwise may incorporate errors due to RNA/DNA amplification and cloning. These new methods not only allow the detection of mutations but are also becoming an alternative to microarrays in studies involving gene expression and copy number alterations in genome-wide analysis.

Cancers suitable for analysis generally include carcinomas, leukemias or lymphomas, and sarcomas. Carcinomas may be of the breast, colon, rectum, lung, oropharynx, hypopharynx, esophagus, stomach, pancreas, liver, gallbladder and bile ducts, small intestine, urinary tract, female genital tract, male genital tract, endocrine glands, and skin. Other suitable cancers include hemangiomas, melanomas, and tumors of the brain, nerves, eyes, and meninges.

Sequence reads coming from NGS may code for all or a portion of an expressed gene in the cancer cell. Mutations of interest can result from a substitution of a wildtype amino acid but may also result from amino acid changes caused by deletions or insertions of nucleotide sequence in the encoding nucleic acid. As used herein, "mutation" means any change in a DNA sequence (or change in amino acid sequence) away from normal. Thus, when there is a normal allele that is prevalent in the population, a mutation changes this to a rare and abnormal variant. In contrast, a "polymorphism" is a DNA sequence variation that is common in the population. In this case no single allele is regarded as the standard sequence. Instead there are two or more equally acceptable alternatives for a wildtype sequence. As used herein, a cut-off point between a mutation and a polymorphism can be 1 per cent. Thus, a polymorphism arises when the least common allele has frequency of at least 1 per cent in the population. If the frequency is lower than 1%, the allele is regarded as a mutation.

Coding sequences from exome and/or transcriptome libraries from the cancer cells are compared to the exome of EBV lymphoblasts (or PHA blasts) or any other cell from the patient excluding the tumor cell from the cancer patient and in some instances from the exome from EBV lymphoblasts (or PHA blasts) from an HLA-matched bone marrow donor. Preferred mutations are those where the sequence in a coding region of a gene in the cancer is different from the same gene in normal cells or essentially wildtype cells (e.g. EBV transformed cells) from the cancer patient and, if used, from the same gene from an HLA-matched bone marrow donor. Preferably, the sequence from a gene in normal cells or essentially wildtype cells (e.g. EBV transformed cells) and from a gene from an HLA-matched bone marrow donor are the same. This approach is exemplified in FIGs 1(a) and 2(a).

Further selection of the mutations identified in coding regions of genes expressed in the cancer cells is achieved by identifying those sequences which have a particular amino acid in the cancer cell gene sequence and/or a particular amino acid at the corresponding position in the wildtype gene sequence. The amino acid at the corresponding position in the gene from the cancer cell and from the corresponding wildtype sequence can be referred to as "mutant position amino acid" and "wildtype position amino acid, respectively.

The selection of sequences based on particular mutant position amino acids and wildtype position amino acids may depend on the nature of the major histocompatibility complex (MHC) class I or class II supertype of the cancer patient. As used herein, MHC refers to a cell surface molecule encoded by a large gene family in all vertebrates. MHC molecules mediate interactions of leukocytes, also called white blood cells (WBCs), which are immune cells, with other leukocytes or body cells and determines compatibility of donors for organ transplant as well as one's susceptibility to an autoimmune disease via crossreacting immunization. In humans, MHC may also be referred to as human leukocyte antigen (HLA).

The MHC gene family is divided into three subgroups-class I, class II, and class III. Diversity of antigen presentation, mediated by MHC classes I and II, is attained in multiple ways: 1) the MHC's genetic encoding is polygenic; 2) MHC genes are highly polymorphic and have many variants; and 3) several MHC genes are expressed from both inherited alleles.

MHC functions to display peptide fragment (epitope) of protein molecules-either of the host's own phenotype or of other biologic entities-on the cell surface for recognition by T lymphocytes (T cells). MHC class II antigens generally mediate immunization-specific immunity-to an antigen while MHC class I antigens generally mediate destruction of host cells displaying that antigen.

HLA class I molecules can be clustered into groups, designated as supertypes, representing sets of molecules that share largely overlapping peptide binding specificity. Each supertype can be described by a supermotif that reflects the broad main anchor motif recognized by molecules within the corresponding supertype.

In accordance with an embodiment of the invention, the large number of mutant sequences initially identified by NGS analysis are further selected by identifying the MHC class I or class II type of the cancer patient and then choosing one or more mutant position amino acids and/or wildtype position amino acids that are changed in the cancer cells. Thus, mutant sequences are selected by identifying an HLA supertype of the cancer patient and then selecting one or more amino acids for the HLA supertype as the mutant position amino acid and/or wildtype position amino acid using FIG. 7. The amino acids in FIG. 7 constitute known amino-acid binding preferences for the HLA pockets for the specified the HLA supertypes (see, for example, Sydney et al. BMC Immunology 2008, 9:1, Ramensee et al. Immunogenetics (1999) 50:213). Amino acids highlighted by bold and with underlining are preferred binding residues. For example, where the cancer patient expresses HLA-A1 as the MHC class I antigen, the particular mutant position amino acid or wildtype position amino acid is an amino acid selected from the group consisting of tyrosine, aspartic acid, glutamic acid, leucine, serine and threonine, more preferably, leucine, serine, threonine and tyrosine, and even more preferably tyrosine. In one embodiment, the initial set of mutant and corresponding wildtype sequences obtained by NGS can be selected for those where the mutant amino acid position and the corresponding amino acid position in the wildtype gene sequence involve a gain or loss of any tyrosine. This selection step is exemplified in FIGs 1(b) and 2(b). The selection of one or more amino acids for the HLA supertype as the mutant position amino acid and/or wildtype position amino acid can be one, two, three, four, five six, seven, eight, nine or 10 amino acids. Selection based on one or two amino acids may be sufficient to narrow the library to a manageable number of candidate mutant sequences.

In some embodiments, one may ignore the HLA class and/or supertype of the individual and make a further selection of the mutants based on particular mutant position amino acids and/or wildtype position amino acids. In this instance, one or more amino acids are selected from the group consisting of tyrosine, phenylalanine, leucine, isoleucine, methionine, valine and alanine. One may select from this group one, two, three, four, five or six amino acids for the selection of mutants without regard to HLA supertype.

Further selection of mutant sequences from the cancer cells that may be potential T cell epitopes for recognition by cytotoxic T lymphocytes is achieved by evaluating peptides containing the mutation sequences for their ability to bind to MHC antigens that are expressed by the cancer patient. The terms "peptide" polypeptide," and "protein" are used interchangeably to refer to a polymer of amino acid residues. These terms also apply to amino acid polymers where all the amino acids are naturally occurring or where one or more amino acid residues is an artificial chemical analog of a corresponding naturally occurring amino acid. Amino acids can be in the L or D form as long as the binding function of the peptide is maintained.

All peptide sequences are written according to the generally accepted convention whereby the α-N-terminal amino acid residue is on the left and the α-C-terminal amino acid residue is on the right. As used herein, the term "N-terminus" refers to the free alpha-amino group of an amino acid in a peptide, and the term "C-terminus" refers to the free α-carboxylic acid terminus of an amino acid in a peptide. A peptide which is N-terminated with a group refers to a peptide bearing a group on the alpha-amino nitrogen of the N-terminal amino acid residue. An amino acid which is N-terminated with a group refers to an amino acid bearing a group on the α-amino nitrogen.

The selection of mutant sequences from the cancer cells that may be potential T cell epitopes for recognition by cytotoxic T lymphocytes can be carried out in silico using computer-based algorithm(s) for predicting IC₅₀ values for peptides binding to specific MHC molecules. Prediction tools that are readily available on-line e.g., the immune epitope database (IEDB) (24, 25), the NetMHC-3.0 (26), and the SYFPEITHI database (Ramensee Immunogenetics 1999) can be used to predict the peptide sequences. For the IEDB, a percentile rank is generated for each peptide using three methods (ANN, SMM_align and Sturniolo) by comparing the peptide's score against the scores of five million random 15mers selected from the SWISSPROT database. The percentile ranks for the three methods are then used to generate the rank for a consensus method. A small numbered percentile rank indicates a high affinity T cell epitope. A ratio between the probability of being a binder versus a non-binding also is used to evaluate binding epitopes (see, e.g., US Patent Publication 2012/0070493.

T cell epitopes presented by MHC class I molecules are typically peptides between 8 and 11 amino acids in length, whereas MHC class II molecules present longer peptides, 13-17 amino acids in length. Notwithstanding, the only practical limitation on the T cell epitopes is that they are capable of binding to the MHC molecules, which may be determined empirically, if necessary. On-line T cell epitope prediction programs are extremely accurate, with peptide sequences predicted to an accuracy of 95% (26).

The selection of peptides with useful T cell epitopes may be determined by synthesizing the peptides and testing them for binding to antigen-presenting cells that express the MHC antigens (see, for example, Peters et al. June 2006. PLoS Computational Biology 2:6 e65). Prioritization of the peptides used to test specificity of the cancer specific T cell lines can be done according to their involvement with oncogenes and then by their affinity to the patients' MHC.

Predicting peptides that bind to Class II MHC antigens can be more difficult than for Class I MHC antigens. This can be addressed for class II MHC by synthesizing longer peptides (e.g. 31mers) and selecting for binding to class II expressing cells by in vitro experiments. For example, for the ability of the peptide to induce class II restricted T cells or the ability to serve as helper epitopes and help in the induction of Class I restricted T cells. Because of their length, the Class II peptides may be more promiscuous and bind to multiple Class II alleles. Within the 31mer, epitopes that bind to multiple Class I alleles can be found.

Peptides that are predicted or shown to bind to MHC class I or class II antigens expressed by the cells of the cancer patient can be tested to determine if they are recognized by cytotoxic T lymphocytes (CTL) cell lines prepared from the cancer patient or from an HLA-matched donor. This can be determined in standard assays. For example, CTL lines prepared from the patient can be tested for cytotoxicity against patient leukemic blasts (LB), PHA-induced T lymphocyte cell lines as well as skin fibroblasts (FB) using published methods (23). CTL lines from the patient also can be tested to determine if they inhibit the growth of non-leukemic hematopoietic progenitor cells (22, table 1). Polyclonal CTLs preparations are enriched for CD8⁺ but may contain some CD4⁺ cells (22). Activation of CTL lines can be measured by determining the level of interferon gamma (IFNγ) secretion using the ELISpot assay, or an ELISA which are readily adaptable to high throughput screening.

These methods are useful to obtain polyclonal, leukemic-specific T cell lines from the cancer patient and also provide for their enrichment or cloning. With these methods, one determines whether particular mutations selected by NGS can activate CTL cell lines from the patient so as to kill the patient's cancer cells but spare normal cells (e.g. EBV lymphoblast) from the cancer patient or from an MHC matched normal donor. The peptides with mutant sequence that activate CTL lead to the identification of mutant genes in the patient cancer cells that may be essential to maintain the leukemic phenotype (driver mutations) or lead to identification of mutations in any other genes not involved in the cancer phenotype (passenger mutations).

Peptides with mutant sequence from the cancer cells that activate the patient's (or HLA-matched individuals) CTL lines specific for the cancer cells can be evaluated for their MHC restriction by using one or more of the following methods: blocking by MHC-specific antibodies, recognition of paired EBV-transformed cell lines that differ by one allele, and/or recognition of a single allele transfectant of the .221 cell line (EBV-transformed cell line with no Class I MHC) (22). Mutant peptides with broad MHC restriction may have application to activate cancer specific CTL from cancer patients that have different MHC class I and class II antigens.

Mutant peptides that activate CTL lines from the cancer patients or that have been selected by other methods, i.e., is predicted to bind to one of the HLA of the cancer patient can be used to induce leukemia-specific CTL in vitro from mononuclear cells from the patient or from an HLA-matched subject. It is desirable to directly stimulate mononuclear cells in vitro using the mutant peptides to induce T cell lines that recognize and kill tumor cells. This would avoid the need to use cancer cells to stimulate T cell induction (a very desirable feature in the case of solid tumors) and will make the CTL lines available in a more rapid and less expensive manner. Effector cells generated with this method can be tested for recognition of the peptide and the leukemic cell by the ELISpot assay. One can also test the affinity of the CTL lines by determining the number of cells necessary to kill the tumor.

Mutant peptide containing sequence that contains an epitope recognized by cancer specific CTL lines derived from the patient or from an HLA-matched donor or that have been selected by other methods, i.e., is predicted to bind to one of the HLA of the cancer patient, can be used in the preparation of a cancer vaccine. Such vaccine represents an immunogenic composition that can be administered to an individual with cancer in order to elicit CTLs that specifically recognize the mutant sequence expressed by cancer cells and result in cancer cell killing. The vaccine composition thus comprises mutant peptides or mutant polypeptides corresponding to tumor specific neoantigens identified by the methods described herein.

A suitable vaccine will preferably contain at least one mutant peptide sequence, and more preferably multiple mutant peptide sequences such as 2, 3, 4, 5, 6, 7, 8, 9, 10, or more. The mutant peptide antigens that are used in the vaccine are chosen for their ability to bind to MHC antigens expressed by the cancer patient who is to receive the vaccine. Better, they are peptides that have been recognized by CTL lines specific for the tumor or can induce CTL lines that are specific for the tumor.

The vaccine can comprise a mixture of different peptide sequences or a single polypeptide that comprises a number of mutant sequences, the latter also referred to as a polyprotein. The peptides or polyprotein can be prepared by peptide synthesis chemistry. For proteins that exceed about 50 amino acids in length, the cost of efficiency of peptide synthesis may require that the polypeptide or polyprotein be produced by recombinant DNA expression methods well known in the art such as expression systems in bacteria and yeast as described previously (see, e.g., U.S. Pat. No. 5,116,943). In general, nucleic acid encoding the mutant peptide sequence can be cloned into an expression vector for high yield expression of the encoded product. The expression vector can be part of a plasmid, virus, or may be a nucleic acid fragment. The expression vector includes an expression cassette into which the nucleic acid encoding the mutant peptide sequence is cloned in operable association with a promoter. The expression cassette may also include other features such as an origin of replication, and/or chromosome integration elements such as retroviral LTRs, or adeno associated viral (AAV) ITRs. If secretion of the mutant peptide sequence is desired, DNA encoding a signal sequence may be placed upstream of the nucleic acid encoding the mature amino acids.

Cells suitable for replicating and for supporting expression of the mutant peptide sequences are well known in the art. Such cells may be transfected or transduced as appropriate with the particular expression vector and large quantities of vector containing cells can be grown for seeding large scale fermenters to obtain sufficient quantities of the mutant peptide sequences for clinical applications. Such cells may include prokaryotic microorganisms, such as E. coli, or various other eukaryotic cells, such as Chinese hamster ovary cells (CHO), insect cells, or the like. Standard technologies are known in the art to express foreign genes in these systems.

The vaccine can also be administered in the form of a nucleic acid vector that encodes the mutant sequence and can express the sequence upon entry of the vector into appropriate cells. A variety of regulatory sequences well known to those of skill in the art are included in the vector to ensure expression of the mutant sequence in the target cells. An exemplary vector can be from a virus such as vaccinia or adenovirus Upon entry into a suitable host cell, the mutant peptide sequences are expressed from the vector and can elicit a host CTL response.

The vector may encode a polyprotein sequence by a "minigene" approach where the sequence encoding multiple mutant peptide sequences (i.e., multiple CTL epitopes) are contained in a single open reading frame with or without linker sequence between the epitopes. Thus, these epitope-encoding DNA sequences are directly adjoined, creating a continuous polypeptide sequence. Additional vector modifications required for efficient gene expression may include the use of introns. The inclusion of mRNA stabilization sequences can also be considered for increasing minigene expression as well as immunostimulatory sequences (e.g., CpGs). An alternative to construction of a minigene is to have the different mutant epitopes under separate expression control such as under a multi-cistronic system or with entirely separate controls such as with separate promoters and the related expression elements.

In some embodiments, the vector encoding for the various mutant peptide sequences also may encode a second protein included to enhance immunogenicity. Examples of proteins or polypeptides that could beneficially enhance the immune response include cytokines (e.g., IL2, IL12, GM-CSF), cytokine-inducing molecules (e.g. LeIF) or costimulatory molecules and helper T cells (see, for example, Vitiello et al. 1995. Journal of Clinical Investigation 95, 341). Expression of these immune enhancing proteins can be achieved by full regulation, partial regulation (e.g., bicistronic expression vector) and by use of separate vectors.

The vaccine can comprise a carrier to enhance the resulting immune response to the peptide mutant sequence. A "carrier" as used herein is a molecule that increases the molecular weight of an antigen thereby rendering the antigen immunogenic. A carrier may be any suitable protein e.g., keyhole limpet hemocyanin, serum proteins such as transferrin, serum albumin, and the like, scaffolding structures such as polysaccharide or antigen-presenting cells such as dendritic cells.

The vaccine can be administered in conjunction with an adjuvant. As used herein the term "adjuvant" refers to any substance that enhances an immune response to an antigen. Thus, an adjuvant is used to modify or augment the effects of a vaccine by stimulating the immune system to respond to the vaccine more vigorously. Adjuvants can include liposomes, lipopolysaccharide (LPS), molecular cages for antigen, components of bacterial cell walls, and endocytosed nucleic acids such as double-stranded RNA (dsRNA), single-stranded DNA (ssDNA), interleukins (e.g., IL-12) and unmethylated CpG dinucleotide-containing DNA (see, e.g., U.S. Pat. No. 6,406,705). Adjuvants may be mixed with the vaccine or may be covalently or non-covalently linked to the mutant sequence peptides or polypeptides.

The mutant peptide vaccine (polypeptide or polypeptide expression vector) can be administered in a sufficient amount to treat a cancer patient that has cancer cells expressing the mutant peptide sequence. Mutant peptide sequences are chosen that will bind to and be presented by MHC antigens expressed by the cancer patient. The administered vaccine will generate CTL in the patient against the cancer cells, which cells will kill the cancer cells thereby treating the patient. Alternatively, or in addition, the patient can be administered CTL cells lines prepared from the cancer patient or from an HLA-matched donor that can specifically kill the cancer cells in the patient. These CTL cell lines can be prepared by contacting in vitro mononuclear cells from the cancer patient or from the HLA-matched donor with the vaccine or with cancer cells from the patient.

As employed herein, the phrase "an effective amount," refers to a dose sufficient to provide concentrations high enough to impart a beneficial effect on the recipient thereof. The specific therapeutically effective dose level for any particular subject will depend upon a variety of factors including the disorder being treated, the severity of the disorder, the activity of the specific compound, the route of administration, the rate of clearance of the compound, the duration of treatment, the drugs used in combination or coincident with the compound, the age, body weight, sex, diet, and general health of the subject, and like factors well known in the medical arts and sciences. Various general considerations taken into account in determining the "therapeutically effective amount" are known to those of skill in the art and are described, e.g., in Gilman et al., eds., Goodman And Gilman's: The Pharmacological Bases of Therapeutics, 8th ed., Pergamon Press, 1990; and Remington's Pharmaceutical Sciences, 17th ed., Mack Publishing Co., Easton, Pa., 1990. Dosage levels typically fall in the range of about 0.001 up to 100 mg/kg/day; with levels in the range of about 0.05 up to 10 mg/kg/day are generally applicable. A composition can be administered parenterally, such as intravascularly, intravenously, intraarterially, intramuscularly, subcutaneously, orally or the like. The composition may be administered as a bolus, or slowly infused.

A therapeutically effective dose can be estimated initially from cell culture assays by determining an IC₅₀. A dose can then be formulated in animal models to achieve a circulating plasma concentration range that includes the IC₅₀ as determined in cell culture. Such information can be used to more accurately determine useful initial doses in humans. Levels of the active ingredient in plasma may be measured, for example, by HPLC. The exact formulation, route of administration and dosage can be chosen by the individual physician in view of the patient's condition.

Cancer patients are treated by the methods of the invention if the patient is cured from the cancer or if the cancer is in remission. As used herein, remission is the state of absence of disease activity in patients with a chronic illness. Thus, a cancer patient in remission is cured of their cancer or the cancer is under control. Thus, cancer may be in remission when the tumor fails to enlarge or to metastasize. Complete remission is the absence of disease active with no evidence of disease as indicated by diagnostic methods, such as imaging, such as CT and PET, and sometimes by bone marrow biopsy. When a cancer patient is put into remission, this may be followed by relapse, which is the reappearance of the cancer. Cancer patients can also be treated by adoptive transfer during relapse.

The mutant (and optionally wildtype) peptide vaccine (polypeptide or polypeptide expression vector) can be contacted in vitro by T cells from the patient or from an HLA-matched donor to stimulate cancer specific CTL cell lines. The CTL cell lines can be expanded to yield sufficient numbers of cells and then administered to the cancer patient to treat the cancer. The in vitro contacting may further include the addition of autologous CD4⁺ T cells and/or dendritic cells from the cancer patient or from the HLA-matched donor. During or after administration of the CTL, the patient may be administered the vaccine to further stimulate CTL activity against the cancer cells in the patient.

The term "CD4⁺ T cells" refers to lymphocytes that produce the CD4 protein and interact with dendritic cells to induce antigen presentation by or maturation of the dendritic cells. CD4⁺ T cells may be isolated from natural sources such as blood, cell lines grown in culture, and CD4⁺ T cell clones.

The term CD8+ T cell refers to lymphocytes that produce the CD8 protein. CD8⁺ T cells that can kill target cells are known as CD8+ cytotoxic T lymphocytes (CTL). CD8⁺ T cells may be isolated from natural sources such as blood, cell lines grown in culture, and CD8⁺ T cell clones.

The term "selective" or "specific", when used in reference to CD8+ CTL means a CD8⁺ CTL that preferentially recognizes and has cytotoxic activity toward a cancer cell, compared to a normal cell. A selective CTL can distinguish, or can be made to distinguish, a target pathologically aberrant cell from a population of non-target cells, and does not substantially cross-react with non-target cells. A pathologically aberrant cell refers to a cell that is altered from the normal due to changes in physiology or phenotype associated with a disease or abnormal condition. A cancer cell is an example of a pathologically aberrant cell.

The term "ex vivo" when used in reference to a cell is intended to mean a cell outside of the body. Therefore, an ex vivo cell culture method involves harvesting cells from an individual. Ex vivo culture methods are applicable to a cell harvested from any tissue or organ of an individual.

The term "in situ" when used in reference to selective CTL activity is intended to mean that selective CTLs can destroy a target pathologically aberrant cell in an intact structure of the body. For example, a selective CTL can destroy a target cell in a heterogeneous population of cells. Specifically, a selective CTL can eliminate a pathologically aberrant cell, such as a tumor cell, from a tissue, such as blood or bone marrow.

The term "sufficient time" when used in the context of inducing the generation of boosting the activity of CD8⁺ CTL refers to the time for processing and presenting of an antigen by dendritic cells, recognition by CD8⁺ CTL of an antigen, and activation of cytotoxic activity. A sufficient time that allows for the completion of this process can vary due to differences in the various cell populations of the methods will result in differences in rates of antigen uptake. Factors that can affect the sufficient time for CD8⁺ CTL induction can include the types of cells in a culture, the purity of various cell types, concentrations of cell types, and whether dendritic cells are immature or are presenting antigen at the time of culture.

As used herein, term "isolated" in reference to a cell refers to when the cell is separated from one or more components with which it is associated in nature. An isolated cell also includes a cell purified from non-cellular tissue components, such as connective tissue fibers. An isolated cell can be, for example, a primary cell, either freshly purified from non-cellular tissue components, or cultured for one or more generation. An example of an isolated cell is a cell that has been separated from blood, such as a cell of a preparation of peripheral blood mononuclear cells (PBMCs).

The term "substantially" unless indicated otherwise means greater than 90%, more preferably greater than 95% and more preferably greater than 99%.

As used herein, term "antigen" means a molecule that can be processed and presented by an antigen-presenting cell and subsequently recognized by a T cell receptor. Such a molecule can be for example, a polypeptide or a peptide.

The term "target", when used in reference to the immune reactivity of a CD8⁺ T cell is any predetermined antigen. A predetermined antigen can be, for example, a cell or polypeptide.

As used herein, the term "naive" when used in reference to a CD8⁺ T cell is intended to mean that a CD8⁺ T cell, has either not been exposed to a particular target cell or antigen in vivo. Therefore, a naive CD8⁺ T cell is exposed to a particular target cell or antigen ex vivo in order for it to be capable of CTL activity selective for the particular target cell or antigen.

As used herein the term mononuclear cell refers to a cell with a single nucleus. Mononuclear cells may be immune cells and may be obtained from any of various sites within the body such as blood, lymph, spleen, lymphnode, thymus and bone marrow.

As used herein, the term "treating" is intended to mean reduction in severity or prevention of a pathological condition mediated by a pathologically aberrant cell. Reduction in severity includes, for example, an arrest or decrease in clinical symptoms, physiological indicators, biochemical markers or metabolic indicators. Prevention of disease includes, for example, precluding the occurrence of the disease or restoring a diseased individual to their state of health prior to disease. Treatment of cancer can reflect a maintenance or reduction in tumor size, the absence of metastases or absence of additional metastases, increased disease free interval or extended survival.

As used herein, the term "effective amount" is intended to mean an amount of CD8⁺ CTL required to effect a decrease in the extent, amount or rate of spread of a pathological condition when administered to an individual. The dosage of a CTL preparation required to be therapeutically effective will depend, for example, on the pathological condition to be treated and the level of abundance and density of the target antigens as well as the weight and condition of the individual, and previous or concurrent therapies. The appropriate amount considered as an effective dose for a particular application of selective CTLs provided by the method can be determined by those skilled in the art, using the guidance provided herein. One skilled in the art will recognize that the condition of the patient needs to be monitored throughout the course of therapy and that the amount of the composition that is administered can be adjusted according to the individual's response to therapy.

The following examples serve to illustrate the present invention. These examples are in no way intended to limit the scope of the invention.

### EXAMPLES

### Example 1: Identifying cancer mutations from Exomic and transcriptomic libraries using NGS.

A scheme for identifying mutations in cancer cells that can be the target for immune recognition is shown in FIG. 1a-c. Leukemic cells (L) and Epstein Barr-transformed B cells (patient EBV-Cell line) (P) were obtained from patient #1 prior to hematopoietic stem cell transplantation (HSCT). Previously published methods were used to isolate from blood the leukemic cells (22, 27) and to prepare EBV cells (see, for example, Caputo JL, et al., J. Tissue Culture Methods 13: 39-44, 1991). An EBV-Cell line also was similarly produced from the bone marrow donor (D). The cells were frozen and maintained in liquid nitrogen.

DNA exome libraries were prepared from the patient #1 leukemic cells, the patient #1 EBV-cell line and from the donor EBV-cell line using NGS methods conducted under contract with Expression Analysis, Inc. (Durham, North Carolina). An RNA transcriptome library was prepared by Expression Analysis, Inc., using the patient leukemic cells. In addition to sequencing, the leukemic cell sample was evaluated for the expression level of known genes using the transcriptome library.

The sequencing results from each library were initially subjected to filtering using depth of coverage for reference + variant >= 20 x. No base alignment quality (BAQ) correction and no probabilistic modeling was used to filter out low frequency variants. Under this approach, the sequences from each cell source which differ from the reference gene sequences (human reference genome NCBI37/hg19 sequence (Genome Bioinformatics Group of the University of California Santa Cruz, available on the world wide web) resulted in an initial set of 128,161 mutations ("the 128K set") (FIG. 1).

The 128K mutation set was further selected using the criteria set below:
1. Discard all variants that arise outside of an exon;
2. Select sequences where the base difference results in a non-synonymous amino-acid (aa) change; and
3. Select sequences where the amino acid at a particular position in a gene of the leukemic cell is different for both alleles versus that in the EBV-cell line from the patient and the donor, and where the amino acid is the same (homozygous or heterozygous) in the EBV-cell line of the patient and the donor. This can be summarized as the amino acid (aa) for L (at both alleles) is different from P and D (L differs at both alleles from P=D).

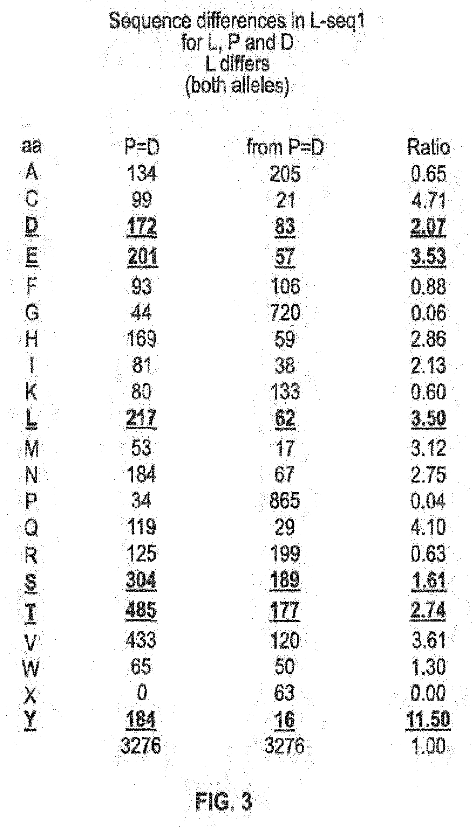

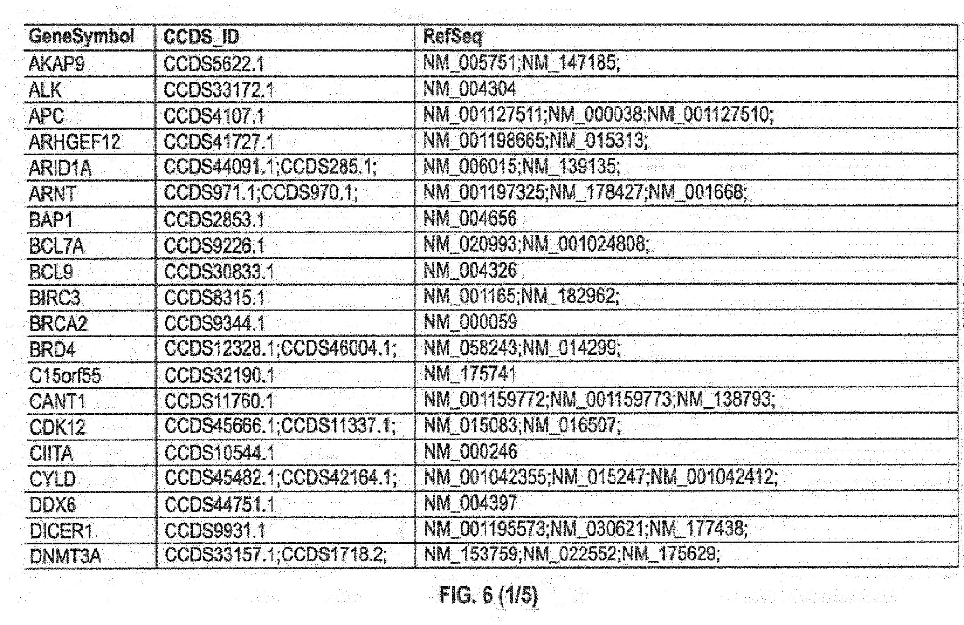

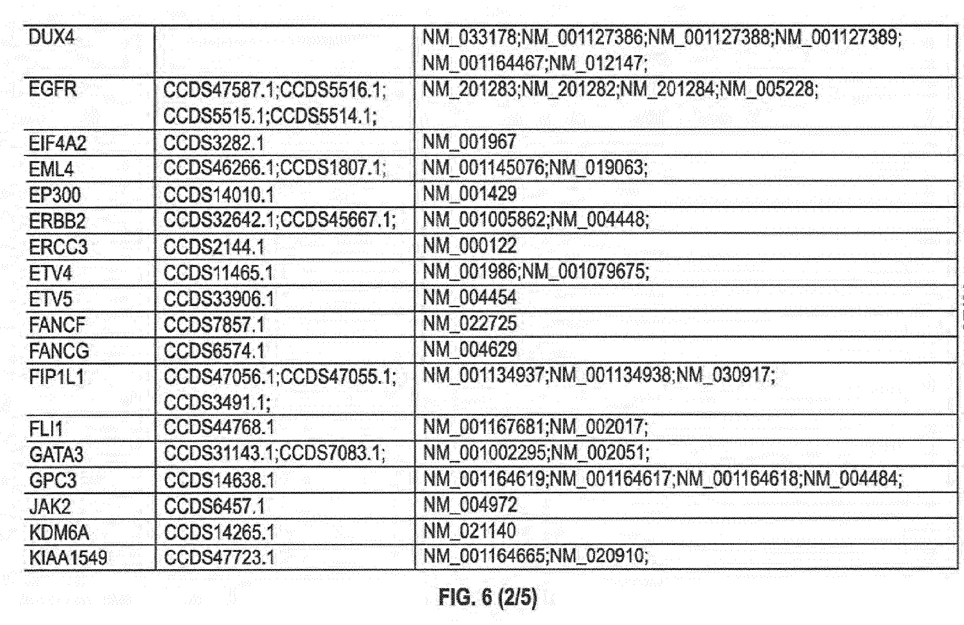

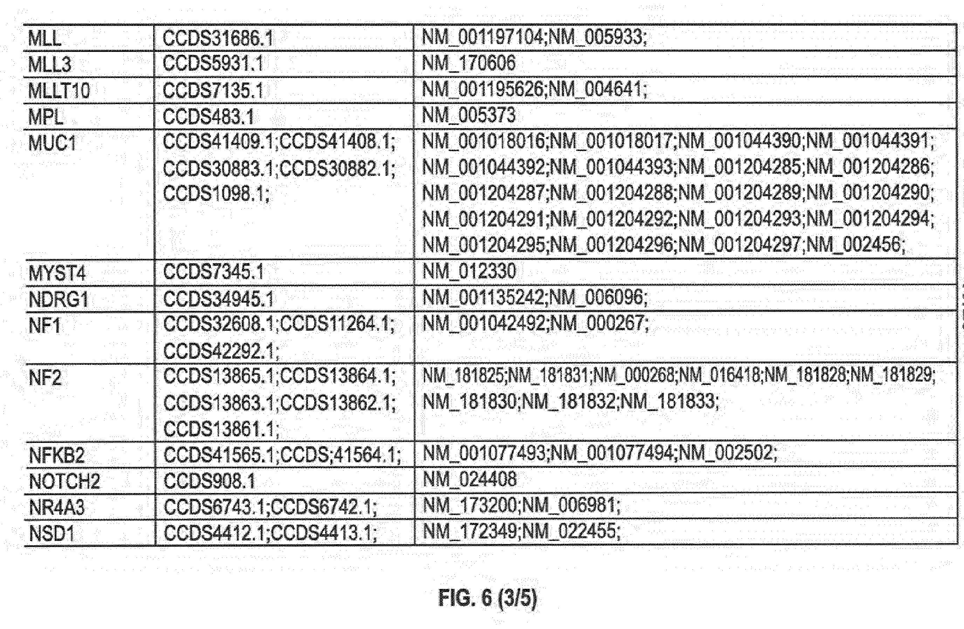

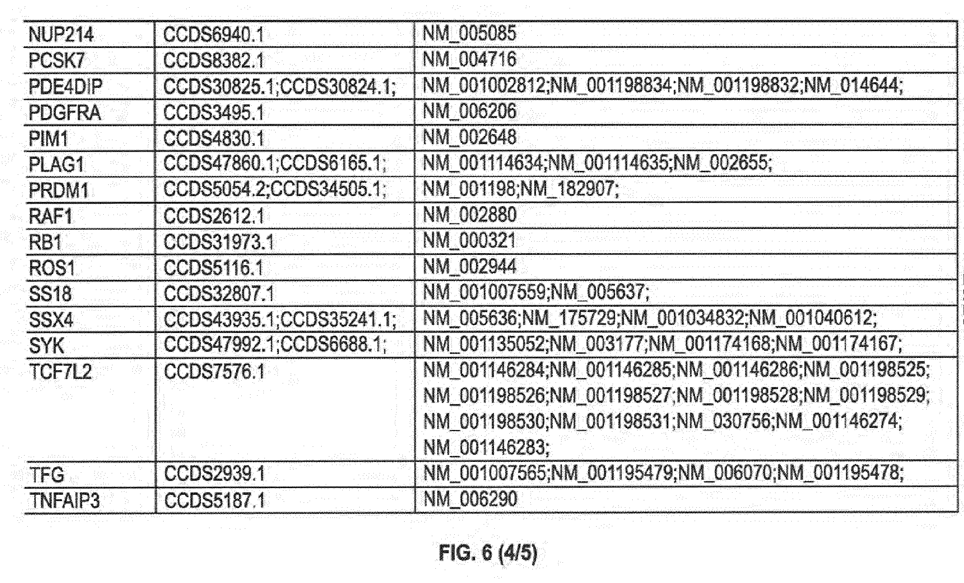

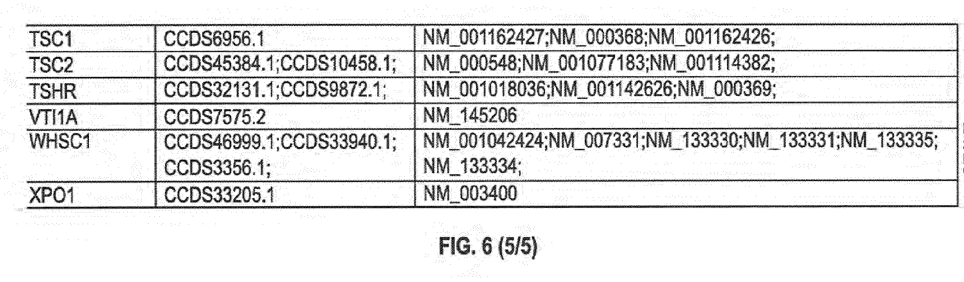

The result of these additional selections applied to the 128K set yielded a smaller set of 3,276 non-synonymous leukemic specific sequences (L-seq1). A further selection of the 3,276 set was obtained by selecting those sequences that are associated with any of a library of 73 known tumor associated genes (TAG) (see FIG. 6). This set of mutations connected with a TAG was then reduced to 92 (FIG. 3).

FIG. 2a-b shows a second approach used to process the raw sequencing information obtained from patient #1. The initial sequences were filtered to a depth of at least 20 reads (>= 20 x), infrequent variants were excluded and BAQ correction was applied. Under this approach, the sequences from each cell source which differ from the reference gene sequences yielded a set of 23,947 (24K) mutants.

The 24K mutation set was further selected using the criteria set below:
1. Discard all variants that arise outside of an exon;
2. Select sequences where the base difference results in a non-synonymous amino-acid (aa) change;
3. Select sequences where the amino acid at a particular position in a gene of the leukemic cell is different for both alleles versus that in the EBV-cell line from the patient and the donor, and where the amino acid is the same (homozygous or heterozygous) in the EBV-cell line of the patient and the donor. This can be summarized as the amino acid (aa) for L (at both alleles) is different from P and D (L differs at both alleles from P=D).

The result of these additional selections applied to the 24K set yielded a smaller set of 242 non-synonymous leukemic specific sequences (L-seq2). This set was further reduced to 127 sequences by selecting only those where the level of expression (FPKM) determined from a transcriptome library was above zero (FIG. 2b).

### Example 2: Selecting cancer specific mutations with potential HLA binding motifs.

The set of mutations from L-seq1 and Lseq2 were further selected to identify a smaller subset with prospects for binding to HLA antigens of the cancer patient. To this end, each L-seq was evaluated for mutants that involve either a gain or loss of a tyrosine. For L-seq1, there were 15 sequences with a tyrosine gain and 184 sequences with a tyrosine loss (FIG. 1b). From the 127 sequences from L-seq2 which were from genes expressed by the cancer cells, there were 5 sequences with a gain of tyrosine and 10 with a loss of tyrosine (FIG. 2b).

Peptide sequences containing the tyrosine involved mutant sequences (both gain and loss) and a corresponding wildtype peptide were transcribed (in silico) as 21mer peptides with 10 amino acids located on each side of the tyrosine involved position. The 21mer peptides were then evaluated for having an 8-11 aa epitope that would exhibit binding to HLA-A1 under the T cell epitope prediction program of IEDB. Peptide sequences were identified that bound below the 3.5% percentile and that showed a ratio of predicted binding greater than 3.

From L-seq1, the gain of tyrosine group showed 12/16 IEDB predicted binders and the loss of tyrosine group showed 166/184 IEDB predicted binders (FIG. 1b). From L-seq2, the gain of tyrosine group showed 5/5 IEDB predicted binders and the loss of tyrosine group showed 9/10 IEDB predicted binders (FIG. 2b). Thus, there is a greater percentage of predicted HLA-A1 binders coming from the tyrosine selected group than from the unselected group screened for tyrosine involved changes. However, the reduced upfront filtering that resulted in the 128K set (versus the 24K set) of prospective mutations resulted in a greater number of mutations which are prospective HLA-A1 binders.

The set of L-seq2 containing 127 expressed cancer specific mutations but not involving a tyrosine change selection showed a lower number of HLA-A1 binders, with 11 acquiring HLA-A1 binding and 21 losing HLA-A1 binding (FIG. 2b). Finally, for the 92 sequences of L-seq1 which were associated with a tumor associated gene, 3/92 acquired HLA-A1 binding while 13/92 lost HLA-A1 binding (FIG. 1c).

FIG. 3 is a listing of the amino acids in patient and donor and corresponding cancer cell of the cancer patient for 3,276 sequences in the L-seq1 set. The highlighted amino acids are those known to be involved in T cell epitopes binding to HLA-A1. The highest P=D to tumor ratio for amino acid changes (gain or loss) was obtained for tyrosine. The other highlighted amino acids also showed significant P=D tumor amino acid change ratios.

FIG. 4 identifies proteins in L-seq1 containing mutant peptide sequence involving a tyrosine and providing a 31mer peptide with mutation involved amino acid located at position 16.

FIG. 5 identifies peptides from 32 proteins from L-seq2 and provides the sequence in cancer cells (T) and the corresponding sequence in the ref (P/D). Also provided is an HLA-A1 binding ranking for each sequence.

### Example 3: Identifying Cancer Mutations - Patient #2

A modified scheme for identifying mutations in cancer cells that can be the target for immune recognition was used on samples obtained from patient #2 and is shown in FIG. 8a. Leukemic cells (L) and Epstein Barr-transformed B cells (patient PHA-Cell line) (P) were obtained from a patient prior to hematopoietic stem cell transplantation (HSCT). Previously published methods were used to isolate from blood the leukemic cells (22, 27) and to prepare EBV cells (see, for example, Caputo JL, et al., J. Tissue Culture Methods 13: 39-44, 1991). An EBV-Cell line also was similarly produced from the bone marrow donor (D). The cells were frozen and maintained in liquid nitrogen.

DNA exome libraries were prepared from the patient leukemic cells, the patient EBV-cell line and from the donor EBV-cell line using NGS methods conducted under contract with Expression Analysis, Inc. (Durham, North Carolina). An RNA transcriptome library was prepared by Expression Analysis, Inc., using the patient leukemic cells. In addition to sequencing, the leukemic cell sample was evaluated for the expression level of known genes using the transcriptome library.

The sequences obtained by the exome sequencing were aligned using BWA (version 0.5.9). with the default parameters except for the seed length (-1) to be 12 bp to aggregate as many alignments as possible. Pileups to use in further downstream processing were generated with Samtools mpilup (http://samtools.sourceforge.net/samtools.shtm) using default parameters, variants were then called using a procedure described previously (Holbrok at al, 2011). The sequences obtained by the transcriptome sequencing were aligned using Bowtie2 and the transcript annotated using the UCSC hg19 reference genome. The RNA transcripts were quantified using RSEM (Ref.: Li, B and Dewey, CN . RSEM: accurate transcript quantification from RNA-Seq data with or without a reference genome. 2011, BMC Bioinformatics 12:323). An initial set of 121,719 mutations ("the 121K set") was obtained.

As described for the patient #1 data, the 121K mutation set from patient #2 was further selected by (1) discarding all variants that arise outside of an exon, (2) select sequences where the base difference results in a non-synonymous amino acid change, and (3) select sequences where the amino acid at a particular position in a gene of the leukemic cell is different for both alleles versus that in the EBV-cell line from the patient and the donor, and where the amino acid is the same (homozygous or heterozygous) in the EBV-cell line of the patient and the donor which can be summarized as the amino acid (aa) for L (at both alleles) is different from P and D (L differs at both alleles from P=D).

The result of these additional selections applied to the 121K set yielded a smaller set of 980 non-synonymous leukemic specific sequences ("L-seq"; FIG. 8a). A further selection of the mutant sequences from the L-seq that are expressed as measured by transcriptome analysis. The L-seq set of 980 31-mers were tested for binding to various HLA subtypes in silico using HLA peptide binding software. It was predicted that 571 of those 31-mers would exhibit HLA binding activity to at least one HLA subtype by at least one region of the peptide. There were a total of 905 predicted binding regions, of which 452 were wild-type sequences and 453 were mutated sequences. The Table insert shows the number of peptides predicted to bind to each specific HLA. The total number of HLA-binding sequences in table insert is greater than 571 because several peptides bound to more than one HLA allele.

### Partial Listing of Cited References

1. 2010. Cancer Facts and Figures 2010. American Cancer Society
2. Dunn, G.P., et al. 2002. Cancer immunoediting: from immunosurveillance to tumor escape. Nat Immunol 3:991-998.
3. Greenman, C., et al. 2007. Patterns of somatic mutation in human cancer genomes. Nature 446:153-158.
4. Kaye, F.J. 2009. Mutation-associated fusion cancer genes in solid tumors. Mol Cancer Ther 8:1399-1408.
6. Muul, L.M., et al. 1987. Identification of specific cytolytic immune responses against autologous tumor in humans bearing malignant melanoma. J Immunol 138:989-995.
7. Rosenberg, S.A., et al. 2008. Adoptive cell transfer: a clinical path to effective cancer immunotherapy. Nat Rev Cancer 8:299-308.
8. Montagna, D., et al. 2006. Emergence of antitumor cytolytic T cells is associated with maintenance of hematologic remission in children with acute myeloid leukemia. Blood 108:3843-3850.
9. van der Bruggen, P., et al. 1991. A gene encoding an antigen recognized by cytolytic T lymphocytes on a human melanoma. Science 254:1643-1647.
10. Boon, T., et al. 1996. Human tumor antigens recognized by T lymphocytes. J Exp Med 183:725-729.
11. Riddell, S.R., et al. 1995. Principles for adoptive T cell therapy of human viral diseases. Annu Rev Immunol 13:545-586.
12. Boon, T., et al. 1994. Tumor antigens recognized by T lymphocytes. Annu Rev Immunol 12:337-365.
13. Haas, B.J., et al. Advancing RNA-Seq analysis. Nat Biotechnol 28:421-423.
14. Lao, K.Q., et al. 2009. mRNA-sequencing whole transcriptome analysis of a single cell on the SOLiD system. J Biomol Tech 20:266-271.
15. Mane, S.P., et al. 2009. Transcriptome sequencing of the Microarray Quality Control (MAQC) RNA reference samples using next generation sequencing. BMC Genomics 10:264.
16. Costa, V., et al. 2010. Uncovering the complexity of transcriptomes with RNA-Seq. J Biomed Biotechnol. 853916.
17. van der Brug, M.P., et al.. Navigating genomic maps of cancer cells. Nat Biotechnol 28:241-242.
18. Nagalakshmi, U., et al. 2008. The transcriptional landscape of the yeast genome defined by RNA sequencing. Science 320:1344-1349.
19. Mamanova, L., et al. FRT-seq: amplification-free, strand-specific transcriptome sequencing. Nat Methods 7:130-132.
22. Montagna, D., et al. 2001. Ex vivo priming for long-term maintenance of antileukemia human cytotoxic T cells suggests a general procedure for adoptive immunotherapy. Blood 98:3359-3366.
23. Montagna, D., et al. 2006. Single-cell cloning of human, donor-derived antileukemia T-cell lines for in vitro separation of graft-versus-leukemia effect from graft-versus-host reaction. Cancer Res 66:7310-7316.
24. Sette, A. 2004. The immune epitope database and analysis resource: from vision to blueprint. Genome Inform 15:299.
25. Zhang, Q., et al. 2008. Immune epitope database analysis resource (IEDB-AR). Nucleic Acids Res 36:W513-518.
26. Lundegaard, C et al. 2008. NetMHC-3.0: accurate web accessible predictions of human, mouse and monkey MHC class I affinities for peptides of length 8-11. Nucleic Acids Res 36:W509-512.
27. Montagna, D., et al. 2003. Generation and ex vivo expansion of cytotoxic T lymphocytes directed toward different types of leukemia or myelodysplastic cells using both HLA-matched and partially matched donors. Exp Hematol 31:1031-1038.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising," "consisting essentially of' and "consisting of' may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims. Other embodiments are set forth within the following claims.

## Claims

1. A method for producing cancer cell-specific cytotoxic T lymphocytes (CTLs), comprising:
contacting peptides encoding for cancer cell-specific mutations specific to a subject's cancer cells, T cells and dendritic cells to generate cancer cell-specific CTLs, wherein the T cells and the dendritic cells are from an HLA-matched donor.

2. The method of claim 1, wherein the contacting occurs *in vitro.*

3. The method of claim 1, wherein the contacting occurs in the HLA-matched donor.

4. The method of claim 1, wherein the subject has a cancer selected from the group consisting of: carcinoma, leukemia, lymphoma and sarcoma.

5. The method of claim 2, wherein the leukemia is acute myelogenous leukemia.

6. The method of claim 1, wherein the peptides are 21 amino acids in length or 31 amino acids in length.

7. The method of claim 1, wherein the peptides comprise an amino acid sequence of up to fifteen amino acids in each of the N-terminal and C-terminal direction flanking a mutant amino acid of the cancer cell-specific mutations.

8. The method of claim 1, wherein each of the cancer cell-specific mutations provides for a gain or loss of a tyrosine.

9. The method of claim 1, wherein T cells are enriched for CD8⁺ T cells and CD4⁺ T cells.

10. Cancer-cell specific CTLs for use as a medicament in the treatment of cancer, wherein the cancer-cell specific CTLs are prepared by the method of any one of claims 1-9.

11. The cancer-cell specific CTLs of claim 10, wherein the cancer is selected from the group consisting of: carcinoma, leukemia, lymphoma and sarcoma.

12. The cancer-cell specific CTLs of claim 11, wherein the leukemia is acute myelogenous leukemia.


SEQUENCE LISTING

<110> PERSIMMUNE, INC.

<120> PERSONALIZED CANCER VACCINES AND ADOPTIVE IMMUNE CELL THERAPIES

<130> P12812EP02

<140> PCT/US2013/050362
<141> 2013-07-12

<150> 61/670,931
<151> 2012-07-12

<160> 518

<170> PatentIn version 3.5

<210> 1
<211> 31
<212> PRT
<213> Homo sapiens

<400> 1
Thr Trp Asn Leu Arg Thr Gln Gln Ser Lys Leu Val Leu Leu Leu Cys
1               5                   10                  15

Gln Thr Val Ala Ile Met Tyr Pro Ser Phe His Ser Phe Ile Leu
            20                  25                  30

<210> 2
<211> 31
<212> PRT
<213> Homo sapiens

<400> 2
Thr Trp Asn Leu Arg Thr Gln Gln Ser Lys Leu Val Leu Leu Leu Tyr
1               5                   10                  15

Gln Thr Val Ala Ile Met Tyr Pro Ser Phe His Ser Phe Ile Leu
            20                  25                  30

<210> 3
<211> 31
<212> PRT
<213> Homo sapiens

<400> 3
Cys Cys Val Pro Val Cys Cys Lys Thr Val Cys Cys Lys Pro Val Cys
1               5                   10                  15

Cys Val Pro Val Cys Cys Gly Asp Ser Ser Cys Cys Gln Gln Ser
            20                  25                  30

<210> 4
<211> 31
<212> PRT

<213> Homo sapiens

<400> 4
Cys Cys Val Pro Val Cys Cys Lys Thr Val Cys Cys Lys Pro Val Tyr
1               5                   10                  15

Cys Val Pro Val Cys Cys Gly Asp Ser Ser Cys Cys Gln Gln Ser
            20                  25                  30

<210> 5
<211> 31
<212> PRT
<213> Homo sapiens

<400> 5
Arg Glu His Ser Gln Thr Cys His Arg His Lys Gly Cys Met Leu Cys
1               5                   10                  15

Thr Met Gln Ala His Ile Thr Arg Ala Leu His Asn Pro Gly His
            20                  25                  30

<210> 6
<211> 31
<212> PRT
<213> Homo sapiens

<400> 6
Arg Glu His Ser Gln Thr Cys His Arg His Lys Gly Cys Met Leu Tyr
1               5                   10                  15

Thr Met Gln Ala His Ile Thr Arg Ala Leu His Asn Pro Gly His
            20                  25                  30

<210> 7
<211> 31
<212> PRT
<213> Homo sapiens

<400> 7
Ser Ser Val Gln Gln Asp Pro Leu Ser Arg His Pro Pro Glu Thr Cys
1               5                   10                  15

Gln Met Glu Ala Gly Ser Leu Phe Leu Leu Ser Ser Asp Gly Gln
            20                  25                  30

<210> 8
<211> 31
<212> PRT
<213> Homo sapiens

<400> 8
Ser Ser Val Gln Gln Asp Pro Leu Ser Arg His Pro Pro Glu Thr Tyr
1               5                   10                  15

Gln Met Glu Ala Gly Ser Leu Phe Leu Leu Ser Ser Asp Gly Gln
            20                  25                  30

<210> 9
<211> 31
<212> PRT
<213> Homo sapiens

<400> 9
Glu Thr Val Ser Pro Leu Pro Ser Ser Met Asp Leu Leu Ile Gln Asp
1               5                   10                  15

Ser Pro Asp Ser Ser Thr Ser Pro Lys Gly Lys Gln Pro Thr Ser
            20                  25                  30

<210> 10
<211> 31
<212> PRT
<213> Homo sapiens

<400> 10
Glu Thr Val Ser Pro Leu Pro Ser Ser Met Asp Leu Leu Ile Gln Tyr
1               5                   10                  15

Ser Pro Asp Ser Ser Thr Ser Pro Lys Gly Lys Gln Pro Thr Ser
            20                  25                  30

<210> 11
<211> 31
<212> PRT
<213> Homo sapiens

<400> 11
Arg Ala Arg Gln Ala Glu Pro Val Gln Lys Gln Gly Leu Trp Leu Asp
1               5                   10                  15

Val His Ala Leu Gly Glu Leu Gln Arg Gln Gly Val Pro Pro Thr
            20                  25                  30

<210> 12
<211> 31
<212> PRT
<213> Homo sapiens

<400> 12
Arg Ala Arg Gln Ala Glu Pro Val Gln Lys Gln Gly Leu Trp Leu Tyr
1               5                   10                  15

Val His Ala Leu Gly Glu Leu Gln Arg Gln Gly Val Pro Pro Thr
            20                  25                  30

<210> 13
<211> 31
<212> PRT

<213> Homo sapiens

<400> 13
Ser Asp Asp Leu Asn Glu Arg Cys Pro Val Phe Leu Gln Trp Leu Asp
1               5                   10                  15

Cys Val His Gln Leu Gln Arg Gln Phe Pro Cys Ser Phe Glu Phe
            20                  25                  30

<210> 14
<211> 31
<212> PRT
<213> Homo sapiens

<400> 14
Ser Asp Asp Leu Asn Glu Arg Cys Pro Val Phe Leu Gln Trp Leu Tyr
1               5                   10                  15

Cys Val His Gln Leu Gln Arg Gln Phe Pro Cys Ser Phe Glu Phe
            20                  25                  30

<210> 15
<211> 31
<212> PRT
<213> Homo sapiens

<400> 15
Ser Glu Ile Leu Phe Thr Val Ala Ile Thr Pro Arg Met Leu Ala Asp
1               5                   10                  15

Leu Leu Ser Thr His His Ser Ile Thr Phe Val Ala Cys Ala Asn
            20                  25                  30

<210> 16
<211> 31
<212> PRT
<213> Homo sapiens

<400> 16
Ser Glu Ile Leu Phe Thr Val Ala Ile Thr Pro Arg Met Leu Ala Tyr
1               5                   10                  15

Leu Leu Ser Thr His His Ser Ile Thr Phe Val Ala Cys Ala Asn
            20                  25                  30

<210> 17
<211> 31
<212> PRT
<213> Homo sapiens

<400> 17
Val Ser Thr Leu Val Asn Val His Phe Ala Pro Arg Ile Val Val Asp
1               5                   10                  15

Pro Lys Pro Thr Thr Thr Asp Ile Gly Ser Asp Val Thr Leu Thr
            20                  25                  30

<210> 18
<211> 31
<212> PRT
<213> Homo sapiens

<400> 18
Val Ser Thr Leu Val Asn Val His Phe Ala Pro Arg Ile Val Val Tyr
1               5                   10                  15

Pro Lys Pro Thr Thr Thr Asp Ile Gly Ser Asp Val Thr Leu Thr
            20                  25                  30

<210> 19
<211> 31
<212> PRT
<213> Homo sapiens

<400> 19
Leu Asn Asn Ser His Val Ser Lys His Ile Arg Lys Asn Leu Ser Phe
1               5                   10                  15

Lys Pro Ile Asn Gly Glu Glu Glu Ala Glu Ser Ile Glu Glu Glu
            20                  25                  30

<210> 20
<211> 31
<212> PRT
<213> Homo sapiens

<400> 20
Leu Asn Asn Ser His Val Ser Lys His Ile Arg Lys Asn Leu Ser Tyr
1               5                   10                  15

Lys Pro Ile Asn Gly Glu Glu Glu Ala Glu Ser Ile Glu Glu Glu
            20                  25                  30

<210> 21
<211> 31
<212> PRT
<213> Homo sapiens

<400> 21
Cys Val Ala Ser Val Val Ser Val Lys Gly Arg Leu Met Trp Leu His
1               5                   10                  15

Leu Glu Gly Leu Gln Thr Pro Val Pro Glu Val Ile Val Asp Val
            20                  25                  30

<210> 22
<211> 31
<212> PRT

<213> Homo sapiens

<400> 22
Cys Val Ala Ser Val Val Ser Val Lys Gly Arg Leu Met Trp Leu Tyr
1               5                   10                  15

Leu Glu Gly Leu Gln Thr Pro Val Pro Glu Val Ile Val Asp Val
            20                  25                  30

<210> 23
<211> 31
<212> PRT
<213> Homo sapiens

<400> 23
Glu Asp Thr Asp Arg Arg Ala Thr Gln Gly Glu Leu Lys Arg Asp His
1               5                   10                  15

Pro Cys Leu Gln Ala Pro Glu Leu Asp Glu His Leu Val Glu Arg
            20                  25                  30

<210> 24
<211> 31
<212> PRT
<213> Homo sapiens

<400> 24
Glu Asp Thr Asp Arg Arg Ala Thr Gln Gly Glu Leu Lys Arg Asp Tyr
1               5                   10                  15

Pro Cys Leu Gln Ala Pro Glu Leu Asp Glu His Leu Val Glu Arg
            20                  25                  30

<210> 25
<211> 31
<212> PRT
<213> Homo sapiens

<400> 25
Ile Phe Phe Arg Asp Arg Tyr Gly Thr Lys Gln Arg Glu Leu Leu His
1               5                   10                  15

Ile Leu Leu Ala Tyr Glu Glu Tyr Asn Pro Glu Val Gly Tyr Cys
            20                  25                  30

<210> 26
<211> 31
<212> PRT
<213> Homo sapiens

<400> 26
Ile Phe Phe Arg Asp Arg Tyr Gly Thr Lys Gln Arg Glu Leu Leu Tyr
1               5                   10                  15

Ile Leu Leu Ala Tyr Glu Glu Tyr Asn Pro Glu Val Gly Tyr Cys
            20                  25                  30

<210> 27
<211> 31
<212> PRT
<213> Homo sapiens

<400> 27
Pro Phe Gln Cys Lys Thr Cys Gln Arg Lys Phe Ser Arg Ser Asp His
1               5                   10                  15

Leu Lys Thr His Thr Arg Thr His Thr Gly Glu Lys Pro Phe Ser
            20                  25                  30

<210> 28
<211> 31
<212> PRT
<213> Homo sapiens

<400> 28
Pro Phe Gln Cys Lys Thr Cys Gln Arg Lys Phe Ser Arg Ser Asp Tyr
1               5                   10                  15

Leu Lys Thr His Thr Arg Thr His Thr Gly Glu Lys Pro Phe Ser
            20                  25                  30

<210> 29
<211> 31
<212> PRT
<213> Homo sapiens

<400> 29
Trp Thr Ser Asn Phe Leu Val Gly Leu Leu Phe Pro Ser Ala Ala His
1               5                   10                  15

Tyr Leu Gly Ala Tyr Val Phe Ile Ile Phe Thr Gly Phe Leu Ile
            20                  25                  30

<210> 30
<211> 31
<212> PRT
<213> Homo sapiens

<400> 30
Trp Thr Ser Asn Phe Leu Val Gly Leu Leu Phe Pro Ser Ala Ala Tyr
1               5                   10                  15

Tyr Leu Gly Ala Tyr Val Phe Ile Ile Phe Thr Gly Phe Leu Ile
            20                  25                  30

<210> 31
<211> 31
<212> PRT

<213> Homo sapiens

<400> 31
Phe Gly Lys Gly Thr Gln Phe Leu Ala Ala Val Leu Trp Gln Leu Asn
1               5                   10                  15

Gly Thr Lys Ile Thr Asp Phe Gly Glu Pro Arg Ile Gln Gln Glu
            20                  25                  30

<210> 32
<211> 31
<212> PRT
<213> Homo sapiens

<400> 32
Phe Gly Lys Gly Thr Gln Phe Leu Ala Ala Val Leu Trp Gln Leu Tyr
1               5                   10                  15

Gly Thr Lys Ile Thr Asp Phe Gly Glu Pro Arg Ile Gln Gln Glu
            20                  25                  30

<210> 33
<211> 31
<212> PRT
<213> Homo sapiens

<400> 33
Pro Ile Lys Glu Thr Val Val Glu Glu Pro Val Asp Ile Thr Pro Tyr
1               5                   10                  15

Leu Asp Gln Leu Asp Glu Ser Leu Arg Asp Lys Val Leu Gln Leu
            20                  25                  30

<210> 34
<211> 31
<212> PRT
<213> Homo sapiens

<400> 34
Pro Ile Lys Glu Thr Val Val Glu Glu Pro Val Asp Ile Thr Pro Cys
1               5                   10                  15

Leu Asp Gln Leu Asp Glu Ser Leu Arg Asp Lys Val Leu Gln Leu
            20                  25                  30

<210> 35
<211> 28
<212> PRT
<213> Homo sapiens

<400> 35
Pro Tyr Glu Cys Asn Tyr Cys Gly Lys Ser Phe Thr Ser Asn Ser Tyr
1               5                   10                  15

Leu Ser Val His Thr Arg Met His Asn Arg Gln Met
            20                  25

<210> 36
<211> 28
<212> PRT
<213> Homo sapiens

<400> 36
Pro Tyr Glu Cys Asn Tyr Cys Gly Lys Ser Phe Thr Ser Asn Ser Cys
1               5                   10                  15

Leu Ser Val His Thr Arg Met His Asn Arg Gln Met
            20                  25

<210> 37
<211> 31
<212> PRT
<213> Homo sapiens

<400> 37
Thr Asn Met Lys Thr Ser Lys Pro Ile Val His Ser Arg Lys Lys Tyr
1               5                   10                  15

Arg Phe His Lys Thr Arg Ser Arg Met Thr His Arg Thr Pro Lys
            20                  25                  30

<210> 38
<211> 31
<212> PRT
<213> Homo sapiens

<400> 38
Thr Asn Met Lys Thr Ser Lys Pro Ile Val His Ser Arg Lys Lys Cys
1               5                   10                  15

Arg Phe His Lys Thr Arg Ser Arg Met Thr His Arg Thr Pro Lys
            20                  25                  30

<210> 39
<211> 31
<212> PRT
<213> Homo sapiens

<400> 39
Tyr Ala Val Leu Val His Ala Gly Trp Ser Cys His Asn Gly Tyr Cys
1               5                   10                  15

Phe Ser Tyr Val Lys Ala Gln Glu Gly Gln Trp Tyr Lys Met Asp
            20                  25                  30

<210> 40
<211> 31
<212> PRT

<213> Homo sapiens

<400> 40
Tyr Ala Val Leu Val His Ala Gly Trp Ser Cys His Asn Gly Cys Cys
1               5                   10                  15

Phe Ser Tyr Val Lys Ala Gln Glu Gly Gln Trp Tyr Lys Met Asp
            20                  25                  30

<210> 41
<211> 31
<212> PRT
<213> Homo sapiens

<400> 41
Ala Ala Ala Ala Ala Ala Thr Pro Ala Val Arg Thr Val Pro Arg Tyr
1               5                   10                  15

Lys Tyr Ala Ala Gly Val Arg Asn Pro Gln Gln His Arg Asn Ala
            20                  25                  30

<210> 42
<211> 31
<212> PRT
<213> Homo sapiens

<400> 42
Ala Ala Ala Ala Ala Ala Thr Pro Ala Val Arg Thr Val Pro Arg Asp
1               5                   10                  15

Lys Tyr Ala Ala Gly Val Arg Asn Pro Gln Gln His Arg Asn Ala
            20                  25                  30

<210> 43
<211> 31
<212> PRT
<213> Homo sapiens

<400> 43
Ala Ala Pro Trp Cys Tyr Thr Thr Asp Pro Ser Val Arg Trp Glu Tyr
1               5                   10                  15

Cys Asn Leu Thr Arg Cys Ser Asp Ala Glu Trp Thr Ala Phe Val
            20                  25                  30

<210> 44
<211> 31
<212> PRT
<213> Homo sapiens

<400> 44
Ala Ala Pro Trp Cys Tyr Thr Thr Asp Pro Ser Val Arg Trp Glu Asp
1               5                   10                  15

Cys Asn Leu Thr Arg Cys Ser Asp Ala Glu Trp Thr Ala Phe Val
            20                  25                  30

<210> 45
<211> 31
<212> PRT
<213> Homo sapiens

<400> 45
Asp Ala Pro Gly Gln Gln Asp Gly Ala Ala Ser Phe Pro Ala Gly Tyr
1               5                   10                  15

Met Tyr Pro Ser Met Asp Gln Leu Ala Glu Met Leu Pro Gly Val
            20                  25                  30

<210> 46
<211> 31
<212> PRT
<213> Homo sapiens

<400> 46
Asp Ala Pro Gly Gln Gln Asp Gly Ala Ala Ser Phe Pro Ala Gly Asp
1               5                   10                  15

Met Tyr Pro Ser Met Asp Gln Leu Ala Glu Met Leu Pro Gly Val
            20                  25                  30

<210> 47
<211> 31
<212> PRT
<213> Homo sapiens

<400> 47
Glu Val Gly Gly Leu Glu Phe Pro Gly Cys Pro Phe Asn Gly Trp Tyr
1               5                   10                  15

Met Gly Thr Glu Ile Gly Val Arg Asp Phe Cys Asp Val Gln Arg
            20                  25                  30

<210> 48
<211> 31
<212> PRT
<213> Homo sapiens

<400> 48
Glu Val Gly Gly Leu Glu Phe Pro Gly Cys Pro Phe Asn Gly Trp Asp
1               5                   10                  15

Met Gly Thr Glu Ile Gly Val Arg Asp Phe Cys Asp Val Gln Arg
            20                  25                  30

<210> 49
<211> 31
<212> PRT

<213> Homo sapiens

<400> 49
Phe Phe Tyr Lys Gln Asn Glu Asp Ser Lys Arg Leu Ile Trp Leu Tyr
1               5                   10                  15

Gln Asn Leu Ile Lys His Ser Ser Leu Phe Val Lys Gln Leu Asp
            20                  25                  30

<210> 50
<211> 31
<212> PRT
<213> Homo sapiens

<400> 50
Phe Phe Tyr Lys Gln Asn Glu Asp Ser Lys Arg Leu Ile Trp Leu Asp
1               5                   10                  15

Gln Asn Leu Ile Lys His Ser Ser Leu Phe Val Lys Gln Leu Asp
            20                  25                  30

<210> 51
<211> 31
<212> PRT
<213> Homo sapiens

<400> 51
Gly Trp Leu Gln Tyr Val Met Gly Trp Asn Leu Arg Lys Val Gln Tyr
1               5                   10                  15

Phe Leu Met Ala Lys Arg Pro Ala Val Tyr Ile Asp Glu Glu Ala
            20                  25                  30

<210> 52
<211> 31
<212> PRT
<213> Homo sapiens

<400> 52
Gly Trp Leu Gln Tyr Val Met Gly Trp Asn Leu Arg Lys Val Gln Asp
1               5                   10                  15

Phe Leu Met Ala Lys Arg Pro Ala Val Tyr Ile Asp Glu Glu Ala
            20                  25                  30

<210> 53
<211> 31
<212> PRT
<213> Homo sapiens

<400> 53
His Gly Gln Ala Ser Ser Ala Val Arg Asp Ser Gly His Arg Gly Tyr
1               5                   10                  15

Ser Gly Ser Gln Ala Ser Asp Asn Glu Gly His Ser Glu Asp Ser
            20                  25                  30

<210> 54
<211> 31
<212> PRT
<213> Homo sapiens

<400> 54
His Gly Gln Ala Ser Ser Ala Val Arg Asp Ser Gly His Arg Gly Asp
1               5                   10                  15

Ser Gly Ser Gln Ala Ser Asp Asn Glu Gly His Ser Glu Asp Ser
            20                  25                  30

<210> 55
<211> 31
<212> PRT
<213> Homo sapiens

<400> 55
His Asn Ala His Asn Trp Arg Leu Gly Gln Ala Pro Ala Asn Trp Tyr
1               5                   10                  15

Asn Asp Thr Tyr Pro Leu Ser Pro Pro Gln Arg Thr Pro Ala Gly
            20                  25                  30

<210> 56
<211> 31
<212> PRT
<213> Homo sapiens

<400> 56
His Asn Ala His Asn Trp Arg Leu Gly Gln Ala Pro Ala Asn Trp Asp
1               5                   10                  15

Asn Asp Thr Tyr Pro Leu Ser Pro Pro Gln Arg Thr Pro Ala Gly
            20                  25                  30

<210> 57
<211> 31
<212> PRT
<213> Homo sapiens

<400> 57
His Asn Lys Asn Asp His Leu Gln Glu Glu Asp His Ser Gly Trp Tyr
1               5                   10                  15

His Arg Ile Glu Asn Asn Gly Trp Arg Pro Val Ser Asp Thr Phe
            20                  25                  30

<210> 58
<211> 31
<212> PRT

<213> Homo sapiens

<400> 58
His Asn Lys Asn Asp His Leu Gln Glu Glu Asp His Ser Gly Trp Asp
1               5                   10                  15

His Arg Ile Glu Asn Asn Gly Trp Arg Pro Val Ser Asp Thr Phe
            20                  25                  30

<210> 59
<211> 31
<212> PRT
<213> Homo sapiens

<400> 59
His Tyr Leu Leu Ser Leu Trp Gln Arg Leu Ala Ala Ser Val Pro Tyr
1               5                   10                  15

Val Lys Ala Thr Glu Pro His Met Leu Glu Thr Tyr Thr Pro Glu
            20                  25                  30

<210> 60
<211> 31
<212> PRT
<213> Homo sapiens

<400> 60
His Tyr Leu Leu Ser Leu Trp Gln Arg Leu Ala Ala Ser Val Pro Asp
1               5                   10                  15

Val Lys Ala Thr Glu Pro His Met Leu Glu Thr Tyr Thr Pro Glu
            20                  25                  30

<210> 61
<211> 31
<212> PRT
<213> Homo sapiens

<400> 61
Ile Gln Leu Lys Asp Ala Leu Glu Lys Asn Gln Gln Trp Leu Val Tyr
1               5                   10                  15

Asp Gln Gln Arg Glu Val Tyr Val Lys Gly Leu Leu Ala Lys Ile
            20                  25                  30

<210> 62
<211> 31
<212> PRT
<213> Homo sapiens

<400> 62
Ile Gln Leu Lys Asp Ala Leu Glu Lys Asn Gln Gln Trp Leu Val Asp
1               5                   10                  15

Asp Gln Gln Arg Glu Val Tyr Val Lys Gly Leu Leu Ala Lys Ile
            20                  25                  30

<210> 63
<211> 31
<212> PRT
<213> Homo sapiens

<400> 63
Lys Arg Gln Leu Trp Asp Arg Thr Arg Pro Glu Val Gln Gln Trp Tyr
1               5                   10                  15

Gln Gln Phe Leu Tyr Met Gly Phe Asp Glu Ala Lys Phe Glu Asp
            20                  25                  30

<210> 64
<211> 31
<212> PRT
<213> Homo sapiens

<400> 64
Lys Arg Gln Leu Trp Asp Arg Thr Arg Pro Glu Val Gln Gln Trp Asp
1               5                   10                  15

Gln Gln Phe Leu Tyr Met Gly Phe Asp Glu Ala Lys Phe Glu Asp
            20                  25                  30

<210> 65
<211> 31
<212> PRT
<213> Homo sapiens

<400> 65
Pro Ser Met Ala Ser Met Ala Ala Ile Gly Ser Cys Ser Lys Glu Tyr
1               5                   10                  15

Arg Val Leu Leu Gly Gln Leu Gln Lys Gln Thr Asp Leu Met Gln
            20                  25                  30

<210> 66
<211> 31
<212> PRT
<213> Homo sapiens

<400> 66
Pro Ser Met Ala Ser Met Ala Ala Ile Gly Ser Cys Ser Lys Glu Asp
1               5                   10                  15

Arg Val Leu Leu Gly Gln Leu Gln Lys Gln Thr Asp Leu Met Gln
            20                  25                  30

<210> 67
<211> 31
<212> PRT

<213> Homo sapiens

<400> 67
Gln Glu Arg Lys Val Asn Ser Arg Ala Glu Met Glu Ile Gly Arg Tyr
1               5                   10                  15

His Trp Met Tyr Pro Gly Ser Lys Asn His Gln Tyr His Pro Val
            20                  25                  30

<210> 68
<211> 31
<212> PRT
<213> Homo sapiens

<400> 68
Gln Glu Arg Lys Val Asn Ser Arg Ala Glu Met Glu Ile Gly Arg Asp
1               5                   10                  15

His Trp Met Tyr Pro Gly Ser Lys Asn His Gln Tyr His Pro Val
            20                  25                  30

<210> 69
<211> 31
<212> PRT
<213> Homo sapiens

<400> 69
Arg Asp Asn Thr Ala Gly Pro His Cys Glu Lys Cys Ser Asp Gly Tyr
1               5                   10                  15

Tyr Gly Asp Ser Thr Ala Gly Thr Ser Ser Asp Cys Gln Pro Cys
            20                  25                  30

<210> 70
<211> 31
<212> PRT
<213> Homo sapiens

<400> 70
Arg Asp Asn Thr Ala Gly Pro His Cys Glu Lys Cys Ser Asp Gly Asp
1               5                   10                  15

Tyr Gly Asp Ser Thr Ala Gly Thr Ser Ser Asp Cys Gln Pro Cys
            20                  25                  30

<210> 71
<211> 31
<212> PRT
<213> Homo sapiens

<400> 71
Arg Pro Ile Pro Glu Glu Asn Phe Leu Ala Ala Gln Ala Ser Arg Tyr
1               5                   10                  15

Ser Ser Asn Gly Ile Phe Gly Phe Leu Pro His His Pro Phe Leu
            20                  25                  30

<210> 72
<211> 31
<212> PRT
<213> Homo sapiens

<400> 72
Arg Pro Ile Pro Glu Glu Asn Phe Leu Ala Ala Gln Ala Ser Arg Asp
1               5                   10                  15

Ser Ser Asn Gly Ile Phe Gly Phe Leu Pro His His Pro Phe Leu
            20                  25                  30

<210> 73
<211> 31
<212> PRT
<213> Homo sapiens

<400> 73
Arg Val Gly Ala Gly Ala Pro Val Tyr Met Ala Ala Val Leu Glu Tyr
1               5                   10                  15

Leu Thr Ala Glu Ile Leu Glu Leu Ala Gly Asn Ala Ala Arg Asp
            20                  25                  30

<210> 74
<211> 31
<212> PRT
<213> Homo sapiens

<400> 74
Arg Val Gly Ala Gly Ala Pro Val Tyr Met Ala Ala Val Leu Glu Asp
1               5                   10                  15

Leu Thr Ala Glu Ile Leu Glu Leu Ala Gly Asn Ala Ala Arg Asp
            20                  25                  30

<210> 75
<211> 31
<212> PRT
<213> Homo sapiens

<400> 75
Ser Phe Leu Asn Leu Tyr Ser Val Asp Ala Ser Lys Thr Gly Gln Tyr
1               5                   10                  15

Thr Cys His Val Thr Asn Asp Val Gly Ser Asp Ser Cys Thr Thr
            20                  25                  30

<210> 76
<211> 31
<212> PRT

<213> Homo sapiens

<400> 76
Ser Phe Leu Asn Leu Tyr Ser Val Asp Ala Ser Lys Thr Gly Gln Asp
1               5                   10                  15

Thr Cys His Val Thr Asn Asp Val Gly Ser Asp Ser Cys Thr Thr
            20                  25                  30

<210> 77
<211> 31
<212> PRT
<213> Homo sapiens

<400> 77
Thr Pro Trp Arg Glu Leu Gln Leu His Asp Trp Met Ser Glu Glu Tyr
1               5                   10                  15

Ala Asp Leu Arg Asp Pro Phe Leu Lys Leu Ser Gly Phe Pro Cys
            20                  25                  30

<210> 78
<211> 31
<212> PRT
<213> Homo sapiens

<400> 78
Thr Pro Trp Arg Glu Leu Gln Leu His Asp Trp Met Ser Glu Glu Asp
1               5                   10                  15

Ala Asp Leu Arg Asp Pro Phe Leu Lys Leu Ser Gly Phe Pro Cys
            20                  25                  30

<210> 79
<211> 31
<212> PRT
<213> Homo sapiens

<400> 79
Val Asp Lys Leu Ile Asp Asp Val His Arg Leu Phe Arg Asp Lys Tyr
1               5                   10                  15

Arg Thr Glu Ile Gln Gln Gln Ser Ala Leu Ser Leu Leu Asn Gly
            20                  25                  30

<210> 80
<211> 31
<212> PRT
<213> Homo sapiens

<400> 80
Val Asp Lys Leu Ile Asp Asp Val His Arg Leu Phe Arg Asp Lys Asp
1               5                   10                  15

Arg Thr Glu Ile Gln Gln Gln Ser Ala Leu Ser Leu Leu Asn Gly
            20                  25                  30

<210> 81
<211> 31
<212> PRT
<213> Homo sapiens

<400> 81
Val Thr Ser Asp Lys Leu Lys Asp Trp Leu Ile Ser Arg Gln Arg Tyr
1               5                   10                  15

Trp Gly Thr Pro Ile Pro Ile Val His Cys Pro Val Cys Gly Pro
            20                  25                  30

<210> 82
<211> 31
<212> PRT
<213> Homo sapiens

<400> 82
Val Thr Ser Asp Lys Leu Lys Asp Trp Leu Ile Ser Arg Gln Arg Asp
1               5                   10                  15

Trp Gly Thr Pro Ile Pro Ile Val His Cys Pro Val Cys Gly Pro
            20                  25                  30

<210> 83
<211> 31
<212> PRT
<213> Homo sapiens

<400> 83
Ala Trp Phe Met Gly Phe Gly Tyr Gly Phe Val Phe Thr Phe Leu Tyr
1               5                   10                  15

Trp His Leu Glu Asp Leu Asn Gly Thr Thr Thr Leu Phe Gly Val
            20                  25                  30

<210> 84
<211> 31
<212> PRT
<213> Homo sapiens

<400> 84
Ala Trp Phe Met Gly Phe Gly Tyr Gly Phe Val Phe Thr Phe Leu Phe
1               5                   10                  15

Trp His Leu Glu Asp Leu Asn Gly Thr Thr Thr Leu Phe Gly Val
            20                  25                  30

<210> 85
<211> 31
<212> PRT

<213> Homo sapiens

<400> 85
Cys Phe His Lys His Val Arg Ile Glu Arg Leu Val Ile Gln Ser Tyr
1               5                   10                  15

Phe Val Gln Thr Leu Lys Ile Glu Lys Ser Thr Ser Lys Glu Pro
            20                  25                  30

<210> 86
<211> 31
<212> PRT
<213> Homo sapiens

<400> 86
Cys Phe His Lys His Val Arg Ile Glu Arg Leu Val Ile Gln Ser Phe
1               5                   10                  15

Phe Val Gln Thr Leu Lys Ile Glu Lys Ser Thr Ser Lys Glu Pro
            20                  25                  30

<210> 87
<211> 31
<212> PRT
<213> Homo sapiens

<400> 87
Phe Thr Leu Gly Tyr Ala Ala Asp Arg Tyr Gly Arg Ile Val Ile Tyr
1               5                   10                  15

Leu Leu Ser Cys Leu Gly Val Gly Val Thr Gly Val Val Val Ala
            20                  25                  30

<210> 88
<211> 31
<212> PRT
<213> Homo sapiens

<400> 88
Phe Thr Leu Gly Tyr Ala Ala Asp Arg Tyr Gly Arg Ile Val Ile Phe
1               5                   10                  15

Leu Leu Ser Cys Leu Gly Val Gly Val Thr Gly Val Val Val Ala
            20                  25                  30

<210> 89
<211> 31
<212> PRT
<213> Homo sapiens

<400> 89
Leu Leu Pro Ser Val Phe Gly Ser Leu Arg Asp Ser Gly Tyr Phe Tyr
1               5                   10                  15

Asp Pro Ile Glu Arg Asp Ile Glu Ile Gly Phe Leu Pro Trp Leu
            20                  25                  30

<210> 90
<211> 31
<212> PRT
<213> Homo sapiens

<400> 90
Leu Leu Pro Ser Val Phe Gly Ser Leu Arg Asp Ser Gly Tyr Phe Phe
1               5                   10                  15

Asp Pro Ile Glu Arg Asp Ile Glu Ile Gly Phe Leu Pro Trp Leu
            20                  25                  30

<210> 91
<211> 31
<212> PRT
<213> Homo sapiens

<400> 91
Met Asn Tyr Cys Arg Asn Pro Asp Ala Val Ala Ala Pro Tyr Cys Tyr
1               5                   10                  15

Thr Arg Asp Pro Gly Val Arg Trp Glu Tyr Cys Asn Leu Thr Gln
            20                  25                  30

<210> 92
<211> 31
<212> PRT
<213> Homo sapiens

<400> 92
Met Asn Tyr Cys Arg Asn Pro Asp Ala Val Ala Ala Pro Tyr Cys Phe
1               5                   10                  15

Thr Arg Asp Pro Gly Val Arg Trp Glu Tyr Cys Asn Leu Thr Gln
            20                  25                  30

<210> 93
<211> 31
<212> PRT
<213> Homo sapiens

<400> 93
Pro Arg Ser Val Val Gly Thr Ala Cys Met Tyr Phe Lys Arg Phe Tyr
1               5                   10                  15

Leu Asn Asn Ser Val Met Glu Tyr His Pro Arg Ile Ile Met Leu
            20                  25                  30

<210> 94
<211> 31
<212> PRT

<213> Homo sapiens

<400> 94
Pro Arg Ser Val Val Gly Thr Ala Cys Met Tyr Phe Lys Arg Phe Phe
1               5                   10                  15

Leu Asn Asn Ser Val Met Glu Tyr His Pro Arg Ile Ile Met Leu
            20                  25                  30

<210> 95
<211> 31
<212> PRT
<213> Homo sapiens

<400> 95
Gln Ser Arg Tyr Phe Glu Ser Val Leu Asp Arg Glu Trp Gln Phe Tyr
1               5                   10                  15

Cys Cys Arg Tyr Ser Lys Arg Cys Pro Tyr Ser Cys Trp Leu Thr
            20                  25                  30

<210> 96
<211> 31
<212> PRT
<213> Homo sapiens

<400> 96
Gln Ser Arg Tyr Phe Glu Ser Val Leu Asp Arg Glu Trp Gln Phe Phe
1               5                   10                  15

Cys Cys Arg Tyr Ser Lys Arg Cys Pro Tyr Ser Cys Trp Leu Thr
            20                  25                  30

<210> 97
<211> 16
<212> PRT
<213> Homo sapiens

<400> 97
Ser Cys Arg Pro Ser Cys Tyr Gly Gly Tyr Gly Phe Ser Gly Phe Tyr
1               5                   10                  15

<210> 98
<211> 16
<212> PRT
<213> Homo sapiens

<400> 98
Ser Cys Arg Pro Ser Cys Tyr Gly Gly Tyr Gly Phe Ser Gly Phe Phe
1               5                   10                  15

<210> 99
<211> 31
<212> PRT
<213> Homo sapiens

<400> 99
Val Gln Ala Asn Leu Ser Leu Phe Leu Glu Ala Ala Ser Gly Phe Tyr
1               5                   10                  15

Thr Gln Leu Leu Gln Glu Leu Cys Thr Val Phe Asn Val Asp Leu
            20                  25                  30

<210> 100
<211> 31
<212> PRT
<213> Homo sapiens

<400> 100
Val Gln Ala Asn Leu Ser Leu Phe Leu Glu Ala Ala Ser Gly Phe Phe
1               5                   10                  15

Thr Gln Leu Leu Gln Glu Leu Cys Thr Val Phe Asn Val Asp Leu
            20                  25                  30

<210> 101
<211> 31
<212> PRT
<213> Homo sapiens

<400> 101
Ala Ser Ala Ser Gly Ser Phe Pro Asn Ser Gly Leu Tyr Gly Ser Tyr
1               5                   10                  15

Pro Gln Gly Gln Ala Pro Pro Leu Ser Gln Ala Gln Gly His Pro
            20                  25                  30

<210> 102
<211> 31
<212> PRT
<213> Homo sapiens

<400> 102
Ala Ser Ala Ser Gly Ser Phe Pro Asn Ser Gly Leu Tyr Gly Ser His
1               5                   10                  15

Pro Gln Gly Gln Ala Pro Pro Leu Ser Gln Ala Gln Gly His Pro
            20                  25                  30

<210> 103
<211> 31
<212> PRT
<213> Homo sapiens

<400> 103
Asp Glu Lys Glu Pro Glu Val Leu Gln Asp Ser Leu Asp Arg Cys Tyr
1               5                   10                  15

Ser Thr Pro Ser Gly Tyr Leu Glu Leu Pro Asp Leu Gly Gln Pro

20                  25                  30


<210> 104
<211> 31
<212> PRT
<213> Homo sapiens

<400> 104
Asp Glu Lys Glu Pro Glu Val Leu Gln Asp Ser Leu Asp Arg Cys His
1               5                   10                  15


Ser Thr Pro Ser Gly Tyr Leu Glu Leu Pro Asp Leu Gly Gln Pro
            20                  25                  30


<210> 105
<211> 31
<212> PRT
<213> Homo sapiens

<400> 105
Ile Tyr Val Ile Gly Gly His Cys Gly Tyr Arg Gly Ser Cys Thr Tyr
1               5                   10                  15


Asp Lys Val Gln Ser Tyr Asn Ser Asp Ile Asn Glu Trp Ser Leu
            20                  25                  30


<210> 106
<211> 31
<212> PRT
<213> Homo sapiens

<400> 106
Ile Tyr Val Ile Gly Gly His Cys Gly Tyr Arg Gly Ser Cys Thr His
1               5                   10                  15


Asp Lys Val Gln Ser Tyr Asn Ser Asp Ile Asn Glu Trp Ser Leu
            20                  25                  30


<210> 107
<211> 31
<212> PRT
<213> Homo sapiens

<400> 107
Arg Tyr Leu Ala Cys Lys Gln Arg Ser His Ser Leu Val Ala Thr Tyr
1               5                   10                  15


Leu Leu Arg Asn Ser Leu Leu Leu Ile Phe Thr Ser Ala Thr Tyr
            20                  25                  30


<210> 108
<211> 31
<212> PRT
<213> Homo sapiens

<400> 108
Arg Tyr Leu Ala Cys Lys Gln Arg Ser His Ser Leu Val Ala Thr His
1               5                   10                  15

Leu Leu Arg Asn Ser Leu Leu Leu Ile Phe Thr Ser Ala Thr Tyr
            20                  25                  30

<210> 109
<211> 31
<212> PRT
<213> Homo sapiens

<400> 109
Ser Glu Leu Ser Gly Lys Phe Glu Arg Leu Ile Val Ala Leu Met Tyr
1               5                   10                  15

Pro Pro Tyr Arg Tyr Glu Ala Lys Glu Leu His Asp Ala Met Lys
            20                  25                  30

<210> 110
<211> 31
<212> PRT
<213> Homo sapiens

<400> 110
Ser Glu Leu Ser Gly Lys Phe Glu Arg Leu Ile Val Ala Leu Met His
1               5                   10                  15

Pro Pro Tyr Arg Tyr Glu Ala Lys Glu Leu His Asp Ala Met Lys
            20                  25                  30

<210> 111
<211> 31
<212> PRT
<213> Homo sapiens

<400> 111
Ser Thr Ser Leu Ser Leu Phe Tyr Lys Lys Val Tyr Arg Leu Ala Tyr
1               5                   10                  15

Leu Arg Leu Asn Thr Leu Cys Glu Arg Leu Leu Ser Glu His Pro
            20                  25                  30

<210> 112
<211> 31
<212> PRT
<213> Homo sapiens

<400> 112
Ser Thr Ser Leu Ser Leu Phe Tyr Lys Lys Val Tyr Arg Leu Ala His
1               5                   10                  15

Leu Arg Leu Asn Thr Leu Cys Glu Arg Leu Leu Ser Glu His Pro

20                  25                  30

<210> 113
<211> 31
<212> PRT
<213> Homo sapiens

<400> 113
Ser Val Val Thr Ala Asp Leu His Ala Arg Tyr Gly Ser Pro Thr Tyr
1               5                   10                  15

Phe Tyr Ala Phe Tyr His His Cys Gln Ser Leu Met Lys Pro Ala
            20                  25                  30

<210> 114
<211> 31
<212> PRT
<213> Homo sapiens

<400> 114
Ser Val Val Thr Ala Asp Leu His Ala Arg Tyr Gly Ser Pro Thr His
1               5                   10                  15

Phe Tyr Ala Phe Tyr His His Cys Gln Ser Leu Met Lys Pro Ala
            20                  25                  30

<210> 115
<211> 31
<212> PRT
<213> Homo sapiens

<400> 115
Thr Leu Ser Gly Ile Cys Ala Phe Ile Ser Gly Arg Phe Pro Tyr Tyr
1               5                   10                  15

Arg Arg Lys Phe Pro Ala Trp Gln Asn Ser Ile Arg His Asn Leu
            20                  25                  30

<210> 116
<211> 31
<212> PRT
<213> Homo sapiens

<400> 116
Thr Leu Ser Gly Ile Cys Ala Phe Ile Ser Gly Arg Phe Pro Tyr His
1               5                   10                  15

Arg Arg Lys Phe Pro Ala Trp Gln Asn Ser Ile Arg His Asn Leu
            20                  25                  30

<210> 117
<211> 31
<212> PRT
<213> Homo sapiens

<400> 117
Val Ser Ala Thr Asp Arg Asp Ser Gly Thr Asn Ala Gln Val Thr Tyr
1               5                   10                  15

Ser Leu Leu Pro Pro Gln Asp Pro His Leu Pro Leu Ala Ser Leu
            20                  25                  30

<210> 118
<211> 31
<212> PRT
<213> Homo sapiens

<400> 118
Val Ser Ala Thr Asp Arg Asp Ser Gly Thr Asn Ala Gln Val Thr His
1               5                   10                  15

Ser Leu Leu Pro Pro Gln Asp Pro His Leu Pro Leu Ala Ser Leu
            20                  25                  30

<210> 119
<211> 31
<212> PRT
<213> Homo sapiens

<400> 119
Ala Gln Phe Asn Tyr Cys Phe Asp Val Asp Trp Leu Val Lys Gln Tyr
1               5                   10                  15

Pro Pro Glu Phe Arg Lys Lys Pro Ile Leu Leu Val His Gly Asp
            20                  25                  30

<210> 120
<211> 31
<212> PRT
<213> Homo sapiens

<400> 120
Ala Gln Phe Asn Tyr Cys Phe Asp Val Asp Trp Leu Val Lys Gln Asn
1               5                   10                  15

Pro Pro Glu Phe Arg Lys Lys Pro Ile Leu Leu Val His Gly Asp
            20                  25                  30

<210> 121
<211> 31
<212> PRT
<213> Homo sapiens

<400> 121
Ala Ala Phe Met Ile Ile Leu Thr Ile Asp Arg Ile Gly Arg Arg Tyr
1               5                   10                  15

Pro Trp Ala Ala Ser Asn Met Val Ala Gly Ala Ala Cys Leu Ala

20                  25                  30


<210> 122
<211> 31
<212> PRT
<213> Homo sapiens

<400> 122
Ala Ala Phe Met Ile Ile Leu Thr Ile Asp Arg Ile Gly Arg Arg Ser
1               5                   10                  15


Pro Trp Ala Ala Ser Asn Met Val Ala Gly Ala Ala Cys Leu Ala
            20                  25                  30


<210> 123
<211> 31
<212> PRT
<213> Homo sapiens

<400> 123
Ala Asp Asp Lys Met Ala Ala Phe His Gly Ala Gly Leu Lys Arg Tyr
1               5                   10                  15


Leu Leu Thr Val Met Ala Ala Ala Ala Lys Ala Phe Lys His Pro
            20                  25                  30


<210> 124
<211> 31
<212> PRT
<213> Homo sapiens

<400> 124
Ala Asp Asp Lys Met Ala Ala Phe His Gly Ala Gly Leu Lys Arg Ser
1               5                   10                  15


Leu Leu Thr Val Met Ala Ala Ala Ala Lys Ala Phe Lys His Pro
            20                  25                  30


<210> 125
<211> 25
<212> PRT
<213> Homo sapiens

<400> 125
Ala Glu Ser Gly Ser Cys Cys Thr Thr His Ile Ala Asn His Ser Tyr
1               5                   10                  15


Leu Pro Leu Ser Tyr Trp Gln Gln Pro
            20                  25


<210> 126
<211> 25
<212> PRT
<213> Homo sapiens

<400> 126
Ala Glu Ser Gly Ser Cys Cys Thr Thr His Ile Ala Asn His Ser Ser
1               5                   10                  15

Leu Pro Leu Ser Tyr Trp Gln Gln Pro
            20                  25

<210> 127
<211> 31
<212> PRT
<213> Homo sapiens

<400> 127
Ala Ile His Ser Ser Gly Leu Gly His Val Glu Asn Glu Glu Gln Tyr
1               5                   10                  15

Arg Glu Ala Val Glu Ser Leu Gly Asn Ser His Leu Ser Gln Asn
            20                  25                  30

<210> 128
<211> 31
<212> PRT
<213> Homo sapiens

<400> 128
Ala Ile His Ser Ser Gly Leu Gly His Val Glu Asn Glu Glu Gln Ser
1               5                   10                  15

Arg Glu Ala Val Glu Ser Leu Gly Asn Ser His Leu Ser Gln Asn
            20                  25                  30

<210> 129
<211> 31
<212> PRT
<213> Homo sapiens

<400> 129
Ala Lys Asn Leu Ser His Ala Lys Gly Gly Ala Leu Met Ala Ala Tyr
1               5                   10                  15

Leu Lys Val Leu Pro Leu Phe Ile Met Val Phe Pro Gly Met Val
            20                  25                  30

<210> 130
<211> 31
<212> PRT
<213> Homo sapiens

<400> 130
Ala Lys Asn Leu Ser His Ala Lys Gly Gly Ala Leu Met Ala Ala Ser
1               5                   10                  15

Leu Lys Val Leu Pro Leu Phe Ile Met Val Phe Pro Gly Met Val

20                  25                  30


<210> 131
<211> 31
<212> PRT
<213> Homo sapiens

<400> 131
Ala Lys Ser Ile Leu Lys Ser Ser Lys Leu Ser Asp Thr Thr Glu Tyr
1               5                   10                  15


Gln Pro Ile Leu Ser Ser Tyr Ser His Arg Ala Gln Glu Phe Gly
            20                  25                  30


<210> 132
<211> 31
<212> PRT
<213> Homo sapiens

<400> 132
Ala Lys Ser Ile Leu Lys Ser Ser Lys Leu Ser Asp Thr Thr Glu Ser
1               5                   10                  15


Gln Pro Ile Leu Ser Ser Tyr Ser His Arg Ala Gln Glu Phe Gly
            20                  25                  30


<210> 133
<211> 31
<212> PRT
<213> Homo sapiens

<400> 133
Ala Leu Met Leu Ile Val Asp Ala Tyr Ser Glu His Ala Gly Gln Tyr
1               5                   10                  15


Ser Cys Lys Ala Ala Asn Ser Ala Gly Glu Ala Thr Cys Ala Ala
            20                  25                  30


<210> 134
<211> 31
<212> PRT
<213> Homo sapiens

<400> 134
Ala Leu Met Leu Ile Val Asp Ala Tyr Ser Glu His Ala Gly Gln Ser
1               5                   10                  15


Ser Cys Lys Ala Ala Asn Ser Ala Gly Glu Ala Thr Cys Ala Ala
            20                  25                  30


<210> 135
<211> 31
<212> PRT
<213> Homo sapiens

<400> 135
Ala Leu Asn Val Asp Leu Thr Glu Phe Gln Thr Asn Leu Val Pro Tyr
1               5                   10                  15

Pro Arg Ile His Phe Pro Leu Ala Thr Tyr Ala Pro Val Ile Ser
            20                  25                  30

<210> 136
<211> 31
<212> PRT
<213> Homo sapiens

<400> 136
Ala Leu Asn Val Asp Leu Thr Glu Phe Gln Thr Asn Leu Val Pro Ser
1               5                   10                  15

Pro Arg Ile His Phe Pro Leu Ala Thr Tyr Ala Pro Val Ile Ser
            20                  25                  30

<210> 137
<211> 31
<212> PRT
<213> Homo sapiens

<400> 137
Ala Met Val Trp Pro Pro Thr Tyr Ser Arg Asn Glu Arg Trp Glu Tyr
1               5                   10                  15

Pro His Ser Glu Val Thr Gln Gly Pro Leu Pro Pro Ser Ala His
            20                  25                  30

<210> 138
<211> 31
<212> PRT
<213> Homo sapiens

<400> 138
Ala Met Val Trp Pro Pro Thr Tyr Ser Arg Asn Glu Arg Trp Glu Ser
1               5                   10                  15

Pro His Ser Glu Val Thr Gln Gly Pro Leu Pro Pro Ser Ala His
            20                  25                  30

<210> 139
<211> 31
<212> PRT
<213> Homo sapiens

<400> 139
Ala Gln Ala Val Val Ala Gly Ser Asp Pro Leu Gly Leu Ile Ala Tyr
1               5                   10                  15

Leu Ser His Phe His Ser Ala Phe Lys Ser Met Ala His Ser Pro

20                  25                  30

<210> 140
<211> 31
<212> PRT
<213> Homo sapiens

<400> 140
Ala Gln Ala Val Val Ala Gly Ser Asp Pro Leu Gly Leu Ile Ala Ser
1               5                   10                  15

Leu Ser His Phe His Ser Ala Phe Lys Ser Met Ala His Ser Pro
            20                  25                  30

<210> 141
<211> 31
<212> PRT
<213> Homo sapiens

<400> 141
Ala Val Thr Ile Asn Leu Val Pro Thr Glu Glu Gln Ala Lys Pro Tyr
1               5                   10                  15

Arg Val Val Asn Leu Glu Gln Pro Leu Cys Lys Pro Tyr Thr Val
            20                  25                  30

<210> 142
<211> 31
<212> PRT
<213> Homo sapiens

<400> 142
Ala Val Thr Ile Asn Leu Val Pro Thr Glu Glu Gln Ala Lys Pro Ser
1               5                   10                  15

Arg Val Val Asn Leu Glu Gln Pro Leu Cys Lys Pro Tyr Thr Val
            20                  25                  30

<210> 143
<211> 31
<212> PRT
<213> Homo sapiens

<400> 143
Ala Trp Arg Ser Ser Gln Arg Ser Thr Gln Lys Asp Pro Val Pro Tyr
1               5                   10                  15

Gln Pro Pro Phe Leu Cys Gln Trp Gly Arg His Gln Pro Ser Trp
            20                  25                  30

<210> 144
<211> 31
<212> PRT
<213> Homo sapiens

<400> 144
Ala Trp Arg Ser Ser Gln Arg Ser Thr Gln Lys Asp Pro Val Pro Ser
1               5                   10                  15

Gln Pro Pro Phe Leu Cys Gln Trp Gly Arg His Gln Pro Ser Trp
            20                  25                  30

<210> 145
<211> 31
<212> PRT
<213> Homo sapiens

<400> 145
Cys Cys Arg Trp Arg Lys His Glu Ser Asp Pro Asp Glu Arg Asp Tyr
1               5                   10                  15

Gly Leu Lys Leu Phe Ile Thr Asp Asp Glu Leu Lys Lys Val His
            20                  25                  30

<210> 146
<211> 31
<212> PRT
<213> Homo sapiens

<400> 146
Cys Cys Arg Trp Arg Lys His Glu Ser Asp Pro Asp Glu Arg Asp Ser
1               5                   10                  15

Gly Leu Lys Leu Phe Ile Thr Asp Asp Glu Leu Lys Lys Val His
            20                  25                  30

<210> 147
<211> 31
<212> PRT
<213> Homo sapiens

<400> 147
Cys Asp Tyr Ala Ser Tyr Trp Thr Ser Ser Pro Lys Pro Ser Ser Tyr
1               5                   10                  15

Pro Ser Thr Gly Ser Ser Ser Asn Asp Ala Ala Gln Val Gly Lys
            20                  25                  30

<210> 148
<211> 31
<212> PRT
<213> Homo sapiens

<400> 148
Cys Asp Tyr Ala Ser Tyr Trp Thr Ser Ser Pro Lys Pro Ser Ser Ser
1               5                   10                  15

Pro Ser Thr Gly Ser Ser Ser Asn Asp Ala Ala Gln Val Gly Lys

20                  25                  30

<210> 149
<211> 31
<212> PRT
<213> Homo sapiens

<400> 149
Cys Glu Cys Tyr Asp Glu Leu Gly Asn Arg Tyr Gln Leu Pro Ile Tyr
1               5                   10                  15

Cys Leu Ser Pro Pro Val Asn Leu Leu Leu Glu His Thr Glu Glu
            20                  25                  30

<210> 150
<211> 31
<212> PRT
<213> Homo sapiens

<400> 150
Cys Glu Cys Tyr Asp Glu Leu Gly Asn Arg Tyr Gln Leu Pro Ile Ser
1               5                   10                  15

Cys Leu Ser Pro Pro Val Asn Leu Leu Leu Glu His Thr Glu Glu
            20                  25                  30

<210> 151
<211> 31
<212> PRT
<213> Homo sapiens

<400> 151
Cys Phe Asp His Ala Phe Asp Asp Lys Ala Ser Asn Glu Leu Val Tyr
1               5                   10                  15

Gln Phe Thr Ala Gln Pro Leu Val Glu Ser Ile Phe Arg Lys Gly
            20                  25                  30

<210> 152
<211> 31
<212> PRT
<213> Homo sapiens

<400> 152
Cys Phe Asp His Ala Phe Asp Asp Lys Ala Ser Asn Glu Leu Val Ser
1               5                   10                  15

Gln Phe Thr Ala Gln Pro Leu Val Glu Ser Ile Phe Arg Lys Gly
            20                  25                  30

<210> 153
<211> 31
<212> PRT
<213> Homo sapiens

<400> 153
Cys Leu Leu Ser Glu Tyr Thr Glu Asn Tyr Pro Phe Tyr His Ser Tyr
1               5                   10                  15

Leu Pro Arg Glu Ser Phe Lys Pro Arg Arg Glu Tyr Gln Lys Gly
            20                  25                  30

<210> 154
<211> 31
<212> PRT
<213> Homo sapiens

<400> 154
Cys Leu Leu Ser Glu Tyr Thr Glu Asn Tyr Pro Phe Tyr His Ser Ser
1               5                   10                  15

Leu Pro Arg Glu Ser Phe Lys Pro Arg Arg Glu Tyr Gln Lys Gly
            20                  25                  30

<210> 155
<211> 31
<212> PRT
<213> Homo sapiens

<400> 155
Cys Tyr Leu His Ser Leu Pro Asp Leu Phe Asn Ser Thr Leu Leu Tyr
1               5                   10                  15

Arg Arg Ser Ser Tyr Arg Gln Lys Pro Tyr Gln Gln Leu Glu Ser
            20                  25                  30

<210> 156
<211> 31
<212> PRT
<213> Homo sapiens

<400> 156
Cys Tyr Leu His Ser Leu Pro Asp Leu Phe Asn Ser Thr Leu Leu Ser
1               5                   10                  15

Arg Arg Ser Ser Tyr Arg Gln Lys Pro Tyr Gln Gln Leu Glu Ser
            20                  25                  30

<210> 157
<211> 31
<212> PRT
<213> Homo sapiens

<400> 157
Asp Ala Lys Asn Met Met Ala Ala Cys Asp Pro Arg His Gly Arg Tyr
1               5                   10                  15

Leu Thr Val Ala Thr Val Phe Arg Gly Arg Met Ser Met Lys Glu

20                  25                  30

<210> 158
<211> 31
<212> PRT
<213> Homo sapiens

<400> 158
Asp Ala Lys Asn Met Met Ala Ala Cys Asp Pro Arg His Gly Arg Ser
1               5                   10                  15

Leu Thr Val Ala Thr Val Phe Arg Gly Arg Met Ser Met Lys Glu
            20                  25                  30

<210> 159
<211> 31
<212> PRT
<213> Homo sapiens

<400> 159
Asp Glu Lys Gly Pro Glu Val Leu Gln Asp Ser Leu Asp Arg Cys Tyr
1               5                   10                  15

Ser Thr Pro Ser Gly Cys Leu Glu Leu Thr Asp Ser Cys Gln Pro
            20                  25                  30

<210> 160
<211> 31
<212> PRT
<213> Homo sapiens

<400> 160
Asp Glu Lys Gly Pro Glu Val Leu Gln Asp Ser Leu Asp Arg Cys Ser
1               5                   10                  15

Ser Thr Pro Ser Gly Cys Leu Glu Leu Thr Asp Ser Cys Gln Pro
            20                  25                  30

<210> 161
<211> 31
<212> PRT
<213> Homo sapiens

<400> 161
Asp Gly Glu Gly Asn Phe Asn Trp Arg Phe Val Phe Pro Phe Asp Tyr
1               5                   10                  15

Leu Pro Ala Glu Gln Leu Cys Ile Val Ala Lys Lys Glu His Phe
            20                  25                  30

<210> 162
<211> 31
<212> PRT
<213> Homo sapiens

<400> 162
Asp Gly Glu Gly Asn Phe Asn Trp Arg Phe Val Phe Pro Phe Asp Ser
1               5                   10                  15

Leu Pro Ala Glu Gln Leu Cys Ile Val Ala Lys Lys Glu His Phe
            20                  25                  30

<210> 163
<211> 31
<212> PRT
<213> Homo sapiens

<400> 163
Asp Ile Ala Lys Leu Pro Ala Ile Ser Asp Gln Asp Met Asn Ala Tyr
1               5                   10                  15

Leu Ala Glu Gln Ser Arg Leu His Ala Val Glu Phe Asn Met Leu
            20                  25                  30

<210> 164
<211> 31
<212> PRT
<213> Homo sapiens

<400> 164
Asp Ile Ala Lys Leu Pro Ala Ile Ser Asp Gln Asp Met Asn Ala Ser
1               5                   10                  15

Leu Ala Glu Gln Ser Arg Leu His Ala Val Glu Phe Asn Met Leu
            20                  25                  30

<210> 165
<211> 31
<212> PRT
<213> Homo sapiens

<400> 165
Asp Leu Pro Val Phe Thr Leu Pro Phe Asn Ile Ala Val Thr Leu Tyr
1               5                   10                  15

Leu Ala Ala Thr Gly His Tyr Asn Leu Phe Phe Pro Thr Thr Leu
            20                  25                  30

<210> 166
<211> 31
<212> PRT
<213> Homo sapiens

<400> 166
Asp Leu Pro Val Phe Thr Leu Pro Phe Asn Ile Ala Val Thr Leu Ser
1               5                   10                  15

Leu Ala Ala Thr Gly His Tyr Asn Leu Phe Phe Pro Thr Thr Leu

20                  25                  30

<210> 167
<211> 31
<212> PRT
<213> Homo sapiens

<400> 167
Asp Thr Lys Gly Phe Phe Asp Pro Asn Thr His Glu Asn Leu Thr Tyr
1               5                   10                  15

Leu Gln Leu Leu Glu Arg Cys Val Arg Asp Pro Glu Thr Gly Leu
            20                  25                  30

<210> 168
<211> 31
<212> PRT
<213> Homo sapiens

<400> 168
Asp Thr Lys Gly Phe Phe Asp Pro Asn Thr His Glu Asn Leu Thr Ser
1               5                   10                  15

Leu Gln Leu Leu Glu Arg Cys Val Arg Asp Pro Glu Thr Gly Leu
            20                  25                  30

<210> 169
<211> 31
<212> PRT
<213> Homo sapiens

<400> 169
Glu Asp Ala Leu Arg Lys Ile Arg Ala Val Glu Glu Gln Ile Glu Tyr
1               5                   10                  15

Leu Gln Lys Lys Leu Ala Met Ala Lys Gln Glu Glu Glu Ala Leu
            20                  25                  30

<210> 170
<211> 31
<212> PRT
<213> Homo sapiens

<400> 170
Glu Asp Ala Leu Arg Lys Ile Arg Ala Val Glu Glu Gln Ile Glu Ser
1               5                   10                  15

Leu Gln Lys Lys Leu Ala Met Ala Lys Gln Glu Glu Glu Ala Leu
            20                  25                  30

<210> 171
<211> 31
<212> PRT
<213> Homo sapiens

<400> 171
Glu Ile Asn Asn Pro Lys Phe Asn Phe Leu Asn Pro Asn Asp Pro Tyr
1               5                   10                  15

His Ala Tyr Tyr Arg His Lys Val Ser Glu Phe Lys Glu Gly Lys
            20                  25                  30

<210> 172
<211> 31
<212> PRT
<213> Homo sapiens

<400> 172
Glu Ile Asn Asn Pro Lys Phe Asn Phe Leu Asn Pro Asn Asp Pro Ser
1               5                   10                  15

His Ala Tyr Tyr Arg His Lys Val Ser Glu Phe Lys Glu Gly Lys
            20                  25                  30

<210> 173
<211> 31
<212> PRT
<213> Homo sapiens

<400> 173
Glu Thr Asp Ile Ile Val Ala Asp Phe Ser Ser Gly Arg Glu Ile Tyr
1               5                   10                  15

Leu Pro Ile Arg Glu Ala Leu Lys Asp Lys Asp Val Gly Ile Leu
            20                  25                  30

<210> 174
<211> 31
<212> PRT
<213> Homo sapiens

<400> 174
Glu Thr Asp Ile Ile Val Ala Asp Phe Ser Ser Gly Arg Glu Ile Ser
1               5                   10                  15

Leu Pro Ile Arg Glu Ala Leu Lys Asp Lys Asp Val Gly Ile Leu
            20                  25                  30

<210> 175
<211> 31
<212> PRT
<213> Homo sapiens

<400> 175
Phe His Pro Thr Asn His Ala Tyr Ile Gln Ser Leu Leu Lys Arg Tyr
1               5                   10                  15

Gln Pro His Arg Val Pro Ser Thr Cys Cys Ala Pro Val Lys Thr

20                  25                  30

<210> 176
<211> 31
<212> PRT
<213> Homo sapiens

<400> 176
Phe His Pro Thr Asn His Ala Tyr Ile Gln Ser Leu Leu Lys Arg Ser
1               5                   10                  15

Gln Pro His Arg Val Pro Ser Thr Cys Cys Ala Pro Val Lys Thr
            20                  25                  30

<210> 177
<211> 31
<212> PRT
<213> Homo sapiens

<400> 177
Gly Ala Phe Ser Met Leu Glu Thr Val Gly Ile Asn Leu Phe Leu Tyr
1               5                   10                  15

Pro Trp Lys Lys Glu Phe Arg Ser Ile Lys Thr Tyr Thr Gly Pro
            20                  25                  30

<210> 178
<211> 31
<212> PRT
<213> Homo sapiens

<400> 178
Gly Ala Phe Ser Met Leu Glu Thr Val Gly Ile Asn Leu Phe Leu Ser
1               5                   10                  15

Pro Trp Lys Lys Glu Phe Arg Ser Ile Lys Thr Tyr Thr Gly Pro
            20                  25                  30

<210> 179
<211> 31
<212> PRT
<213> Homo sapiens

<400> 179
Gly Phe Leu Ala Phe Met Ile Phe Met Cys Trp Val Gly Asp Val Tyr
1               5                   10                  15

Pro Val Tyr Gln Pro Val Gly Pro Lys Gln Tyr Pro Tyr Asn Asn
            20                  25                  30

<210> 180
<211> 31
<212> PRT
<213> Homo sapiens

<400> 180
Gly Phe Leu Ala Phe Met Ile Phe Met Cys Trp Val Gly Asp Val Ser
1               5                   10                  15

Pro Val Tyr Gln Pro Val Gly Pro Lys Gln Tyr Pro Tyr Asn Asn
            20                  25                  30

<210> 181
<211> 18
<212> PRT
<213> Homo sapiens

<400> 181
Gly Gly Met Leu Leu Thr Cys Val Val Met Phe Leu Val Val Gln Tyr
1               5                   10                  15

Leu Thr

<210> 182
<211> 18
<212> PRT
<213> Homo sapiens

<400> 182
Gly Gly Met Leu Leu Thr Cys Val Val Met Phe Leu Val Val Gln Ser
1               5                   10                  15

Leu Thr

<210> 183
<211> 31
<212> PRT
<213> Homo sapiens

<400> 183
Gly Lys Gln His Thr Phe Val Glu Thr Glu Ser Val Arg Tyr Val Tyr
1               5                   10                  15

Gln Pro Met Glu Lys Leu Tyr Met Val Leu Ile Thr Thr Lys Asn
            20                  25                  30

<210> 184
<211> 31
<212> PRT
<213> Homo sapiens

<400> 184
Gly Lys Gln His Thr Phe Val Glu Thr Glu Ser Val Arg Tyr Val Ser
1               5                   10                  15

Gln Pro Met Glu Lys Leu Tyr Met Val Leu Ile Thr Thr Lys Asn

20                  25                  30

<210> 185
<211> 31
<212> PRT
<213> Homo sapiens

<400> 185
Gly Leu Ile Ala Asp Ser Gln Ile Ser Ala Ser Ser Thr Gln Glu Tyr
1               5                   10                  15

Leu Trp Ser Pro Ser Ala Ala Arg Leu Val Ser Ser Arg Ser Gly
            20                  25                  30

<210> 186
<211> 31
<212> PRT
<213> Homo sapiens

<400> 186
Gly Leu Ile Ala Asp Ser Gln Ile Ser Ala Ser Ser Thr Gln Glu Ser
1               5                   10                  15

Leu Trp Ser Pro Ser Ala Ala Arg Leu Val Ser Ser Arg Ser Gly
            20                  25                  30

<210> 187
<211> 31
<212> PRT
<213> Homo sapiens

<400> 187
Gly Gln Gly His Gln Tyr Glu Leu Asn Ser Lys Lys His His Gln Tyr
1               5                   10                  15

Gln Pro His Ser Lys Glu Arg Ala Gly Lys Pro Pro Pro Pro Gly
            20                  25                  30

<210> 188
<211> 31
<212> PRT
<213> Homo sapiens

<400> 188
Gly Gln Gly His Gln Tyr Glu Leu Asn Ser Lys Lys His His Gln Ser
1               5                   10                  15

Gln Pro His Ser Lys Glu Arg Ala Gly Lys Pro Pro Pro Pro Gly
            20                  25                  30

<210> 189
<211> 31
<212> PRT
<213> Homo sapiens

<400> 189
Gly Tyr Thr Pro Gln Ser Pro Ser Tyr Ser Pro Thr Ser Pro Ser Tyr
1               5                   10                  15

Ser Pro Thr Ser Pro Ser Tyr Ser Pro Thr Ser Pro Asn Tyr Ser
            20                  25                  30

<210> 190
<211> 31
<212> PRT
<213> Homo sapiens

<400> 190
Gly Tyr Thr Pro Gln Ser Pro Ser Tyr Ser Pro Thr Ser Pro Ser Ser
1               5                   10                  15

Ser Pro Thr Ser Pro Ser Tyr Ser Pro Thr Ser Pro Asn Tyr Ser
            20                  25                  30

<210> 191
<211> 31
<212> PRT
<213> Homo sapiens

<400> 191
His Leu Leu Gln His Lys Ala Pro Val Asp Asp Val Thr Leu Asp Tyr
1               5                   10                  15

Leu Thr Ala Leu His Val Ala Ala His Cys Gly His Tyr Arg Val
            20                  25                  30

<210> 192
<211> 31
<212> PRT
<213> Homo sapiens

<400> 192
His Leu Leu Gln His Lys Ala Pro Val Asp Asp Val Thr Leu Asp Ser
1               5                   10                  15

Leu Thr Ala Leu His Val Ala Ala His Cys Gly His Tyr Arg Val
            20                  25                  30

<210> 193
<211> 31
<212> PRT
<213> Homo sapiens

<400> 193
His Arg Tyr Arg Pro Gly Thr Val Ala Leu Arg Glu Ile Arg Arg Tyr
1               5                   10                  15

Gln Lys Ser Thr Glu Leu Leu Ile Arg Lys Leu Pro Phe Gln Arg

20                  25                  30


<210> 194
<211> 31
<212> PRT
<213> Homo sapiens

<400> 194
His Arg Tyr Arg Pro Gly Thr Val Ala Leu Arg Glu Ile Arg Arg Ser
1               5                   10                  15


Gln Lys Ser Thr Glu Leu Leu Ile Arg Lys Leu Pro Phe Gln Arg
            20                  25                  30


<210> 195
<211> 31
<212> PRT
<213> Homo sapiens

<400> 195
His Ser Thr Asn Arg Leu Thr Leu Ala Val Ala Trp Val Pro Lys Tyr
1               5                   10                  15


Ser Gly Val Ser Pro Arg Asp Lys Cys Lys Leu Ile Cys Arg Ala
            20                  25                  30


<210> 196
<211> 31
<212> PRT
<213> Homo sapiens

<400> 196
His Ser Thr Asn Arg Leu Thr Leu Ala Val Ala Trp Val Pro Lys Ser
1               5                   10                  15


Ser Gly Val Ser Pro Arg Asp Lys Cys Lys Leu Ile Cys Arg Ala
            20                  25                  30


<210> 197
<211> 31
<212> PRT
<213> Homo sapiens

<400> 197
Ile Pro Gly Thr Arg Leu Pro Pro Pro Thr His Gly Pro Gln Glu Tyr
1               5                   10                  15


Pro Pro Pro Pro Ala Val Arg Asp Leu Leu Pro Ser Gly Ser Arg
            20                  25                  30


<210> 198
<211> 31
<212> PRT
<213> Homo sapiens

<400> 198
Ile Pro Gly Thr Arg Leu Pro Pro Pro Thr His Gly Pro Gln Glu Ser
1               5                   10                  15

Pro Pro Pro Pro Ala Val Arg Asp Leu Leu Pro Ser Gly Ser Arg
            20                  25                  30

<210> 199
<211> 31
<212> PRT
<213> Homo sapiens

<400> 199
Ile Pro Trp Gln Asn Tyr His Leu Asn Asp Trp Met Glu Glu Glu Tyr
1               5                   10                  15

Arg His Ile Pro Gly Glu Tyr Val Arg Phe Thr Gly Tyr Pro Cys
            20                  25                  30

<210> 200
<211> 31
<212> PRT
<213> Homo sapiens

<400> 200
Ile Pro Trp Gln Asn Tyr His Leu Asn Asp Trp Met Glu Glu Glu Ser
1               5                   10                  15

Arg His Ile Pro Gly Glu Tyr Val Arg Phe Thr Gly Tyr Pro Cys
            20                  25                  30

<210> 201
<211> 31
<212> PRT
<213> Homo sapiens

<400> 201
Ile Val Gly His Tyr Phe Leu Tyr Gly Phe Arg Ile Leu Pro Leu Tyr
1               5                   10                  15

Arg Cys Ser Arg Trp Pro Cys Pro Asn Val Val Asp Cys Phe Val
            20                  25                  30

<210> 202
<211> 31
<212> PRT
<213> Homo sapiens

<400> 202
Ile Val Gly His Tyr Phe Leu Tyr Gly Phe Arg Ile Leu Pro Leu Ser
1               5                   10                  15

Arg Cys Ser Arg Trp Pro Cys Pro Asn Val Val Asp Cys Phe Val

20                  25                  30

<210> 203
<211> 31
<212> PRT
<213> Homo sapiens

<400> 203
Ile Tyr Pro Leu Ser Gly Ser Asp Arg Lys Lys Val Leu Asp Phe Tyr
1               5                   10                  15

Gln Arg Ala Cys Leu Ser Gly Tyr Cys Ser Ala Phe Ala Tyr Lys
            20                  25                  30

<210> 204
<211> 31
<212> PRT
<213> Homo sapiens

<400> 204
Ile Tyr Pro Leu Ser Gly Ser Asp Arg Lys Lys Val Leu Asp Phe Ser
1               5                   10                  15

Gln Arg Ala Cys Leu Ser Gly Tyr Cys Ser Ala Phe Ala Tyr Lys
            20                  25                  30

<210> 205
<211> 31
<212> PRT
<213> Homo sapiens

<400> 205
Lys Cys Met Thr Asp Ser Glu Ser Ala Pro Pro Asp Trp Pro Tyr Tyr
1               5                   10                  15

Leu Ala Ile Asp Gly Ile Leu Ala Lys Val Pro Glu Ser Cys Asp
            20                  25                  30

<210> 206
<211> 31
<212> PRT
<213> Homo sapiens

<400> 206
Lys Cys Met Thr Asp Ser Glu Ser Ala Pro Pro Asp Trp Pro Tyr Ser
1               5                   10                  15

Leu Ala Ile Asp Gly Ile Leu Ala Lys Val Pro Glu Ser Cys Asp
            20                  25                  30

<210> 207
<211> 31
<212> PRT
<213> Homo sapiens

<400> 207
Lys Phe Arg Ser Lys Lys Tyr Phe Ala Lys His Pro Arg Leu Gly Tyr
1               5                   10                  15

Leu Pro Val Gln Thr Val Leu Glu Gly Asp Asn Leu Glu Thr Pro
            20                  25                  30

<210> 208
<211> 31
<212> PRT
<213> Homo sapiens

<400> 208
Lys Phe Arg Ser Lys Lys Tyr Phe Ala Lys His Pro Arg Leu Gly Ser
1               5                   10                  15

Leu Pro Val Gln Thr Val Leu Glu Gly Asp Asn Leu Glu Thr Pro
            20                  25                  30

<210> 209
<211> 31
<212> PRT
<213> Homo sapiens

<400> 209
Lys Ile Arg Glu Arg Gln Arg Ala Gln Ala Arg Pro Leu Thr Arg Tyr
1               5                   10                  15

Leu Pro Val Arg Lys Glu Asp Phe Asp Leu Arg Ser His Val Glu
            20                  25                  30

<210> 210
<211> 31
<212> PRT
<213> Homo sapiens

<400> 210
Lys Ile Arg Glu Arg Gln Arg Ala Gln Ala Arg Pro Leu Thr Arg Ser
1               5                   10                  15

Leu Pro Val Arg Lys Glu Asp Phe Asp Leu Arg Ser His Val Glu
            20                  25                  30

<210> 211
<211> 31
<212> PRT
<213> Homo sapiens

<400> 211
Lys Thr Leu Phe Glu Val Gly Phe Ile Val Gly His Tyr Phe Leu Tyr
1               5                   10                  15

Gly Phe Arg Ile Leu Pro Leu Tyr Arg Cys Ser Arg Trp Pro Cys

20                  25                  30

<210> 212
<211> 31
<212> PRT
<213> Homo sapiens

<400> 212
Lys Thr Leu Phe Glu Val Gly Phe Ile Val Gly His Tyr Phe Leu Ser
1               5                   10                  15

Gly Phe Arg Ile Leu Pro Leu Tyr Arg Cys Ser Arg Trp Pro Cys
            20                  25                  30

<210> 213
<211> 31
<212> PRT
<213> Homo sapiens

<400> 213
Lys Thr Thr Lys Pro Arg Tyr Pro Ile Phe Met Ala Pro Gln Lys Tyr
1               5                   10                  15

Leu Pro Pro Leu Arg Ile Val Gln Ala Ile Lys Ala Pro Arg Tyr
            20                  25                  30

<210> 214
<211> 31
<212> PRT
<213> Homo sapiens

<400> 214
Lys Thr Thr Lys Pro Arg Tyr Pro Ile Phe Met Ala Pro Gln Lys Ser
1               5                   10                  15

Leu Pro Pro Leu Arg Ile Val Gln Ala Ile Lys Ala Pro Arg Tyr
            20                  25                  30

<210> 215
<211> 31
<212> PRT
<213> Homo sapiens

<400> 215
Leu Ala Gln Thr Val Leu Ala Glu Val Pro Thr Gln Leu Val Ser Tyr
1               5                   10                  15

Phe Arg Ala Gln Gly Trp Ala Pro Leu Lys Pro Leu Pro Pro Ser
            20                  25                  30

<210> 216
<211> 31
<212> PRT
<213> Homo sapiens

<400> 216
Leu Ala Gln Thr Val Leu Ala Glu Val Pro Thr Gln Leu Val Ser Ser
1               5                   10                  15

Phe Arg Ala Gln Gly Trp Ala Pro Leu Lys Pro Leu Pro Pro Ser
            20                  25                  30

<210> 217
<211> 31
<212> PRT
<213> Homo sapiens

<400> 217
Leu Glu Val Cys Gln Cys Asp Asn Arg Gly Ile Cys Gly Thr Ser Tyr
1               5                   10                  15

Pro Thr Thr Ser Pro Gly Thr Arg Tyr Gly Arg Pro His Ser Gly
            20                  25                  30

<210> 218
<211> 31
<212> PRT
<213> Homo sapiens

<400> 218
Leu Glu Val Cys Gln Cys Asp Asn Arg Gly Ile Cys Gly Thr Ser Ser
1               5                   10                  15

Pro Thr Thr Ser Pro Gly Thr Arg Tyr Gly Arg Pro His Ser Gly
            20                  25                  30

<210> 219
<211> 31
<212> PRT
<213> Homo sapiens

<400> 219
Leu Glu Trp Asp Gly Pro Met Ala Val Thr Glu Tyr Val Ile Ser Tyr
1               5                   10                  15

Gln Pro Thr Ala Leu Gly Gly Leu Gln Leu Gln Gln Arg Val Pro
            20                  25                  30

<210> 220
<211> 31
<212> PRT
<213> Homo sapiens

<400> 220
Leu Glu Trp Asp Gly Pro Met Ala Val Thr Glu Tyr Val Ile Ser Ser
1               5                   10                  15

Gln Pro Thr Ala Leu Gly Gly Leu Gln Leu Gln Gln Arg Val Pro

20                  25                  30

<210> 221
<211> 31
<212> PRT
<213> Homo sapiens

<400> 221
Leu Gly Ala Glu Lys Gln Gly Thr Phe Cys Val Asp Cys Glu Thr Tyr
1               5                   10                  15

His Thr Ala Ala Ser Thr Leu Gly Ser Gln Gly Gln Thr Gly Lys
            20                  25                  30

<210> 222
<211> 31
<212> PRT
<213> Homo sapiens

<400> 222
Leu Gly Ala Glu Lys Gln Gly Thr Phe Cys Val Asp Cys Glu Thr Ser
1               5                   10                  15

His Thr Ala Ala Ser Thr Leu Gly Ser Gln Gly Gln Thr Gly Lys
            20                  25                  30

<210> 223
<211> 22
<212> PRT
<213> Homo sapiens

<400> 223
Leu Gly Ile Leu Val Val Ala Gly Cys Ser Phe Ala Ile Arg Arg Tyr
1               5                   10                  15

Gln Lys Lys Ala Thr Ala
            20

<210> 224
<211> 22
<212> PRT
<213> Homo sapiens

<400> 224
Leu Gly Ile Leu Val Val Ala Gly Cys Ser Phe Ala Ile Arg Arg Ser
1               5                   10                  15

Gln Lys Lys Ala Thr Ala
            20

<210> 225
<211> 31
<212> PRT
<213> Homo sapiens

<400> 225
Leu Leu Ile Gln Gly Ser Ala Cys Val Tyr Ser Lys Lys Val Glu Tyr
1               5                   10                  15

Leu Tyr Ser Leu Val Tyr Gln Ala Leu Asp Phe Ile Ser Gly Lys
            20                  25                  30

<210> 226
<211> 31
<212> PRT
<213> Homo sapiens

<400> 226
Leu Leu Ile Gln Gly Ser Ala Cys Val Tyr Ser Lys Lys Val Glu Ser
1               5                   10                  15

Leu Tyr Ser Leu Val Tyr Gln Ala Leu Asp Phe Ile Ser Gly Lys
            20                  25                  30

<210> 227
<211> 31
<212> PRT
<213> Homo sapiens

<400> 227
Leu Asn Trp Ala Val Asp Arg Thr Gly Lys Trp Gln Glu Leu Glu Tyr
1               5                   10                  15

Pro Ser Pro Ala Tyr Pro Ala Phe Ala Cys Gly Ser Gly Tyr Val
            20                  25                  30

<210> 228
<211> 31
<212> PRT
<213> Homo sapiens

<400> 228
Leu Asn Trp Ala Val Asp Arg Thr Gly Lys Trp Gln Glu Leu Glu Ser
1               5                   10                  15

Pro Ser Pro Ala Tyr Pro Ala Phe Ala Cys Gly Ser Gly Tyr Val
            20                  25                  30

<210> 229
<211> 31
<212> PRT
<213> Homo sapiens

<400> 229
Leu Ser Pro Met Glu Pro His Ala Leu Val Gln Leu Cys Gly Thr Tyr
1               5                   10                  15

Pro Pro Ser Tyr Asn Leu Thr Phe His Ser Ser Gln Asn Val Leu

20                  25                  30

<210> 230
<211> 31
<212> PRT
<213> Homo sapiens

<400> 230
Leu Ser Pro Met Glu Pro His Ala Leu Val Gln Leu Cys Gly Thr Ser
1               5                   10                  15

Pro Pro Ser Tyr Asn Leu Thr Phe His Ser Ser Gln Asn Val Leu
            20                  25                  30

<210> 231
<211> 31
<212> PRT
<213> Homo sapiens

<400> 231
Leu Ser Arg Tyr Ala His Trp Val Val Ser Gln Pro Pro Asp Thr Tyr
1               5                   10                  15

Leu Lys Pro Leu Met Thr Glu Leu Leu Lys Arg Ile Leu Asp Ser
            20                  25                  30

<210> 232
<211> 31
<212> PRT
<213> Homo sapiens

<400> 232
Leu Ser Arg Tyr Ala His Trp Val Val Ser Gln Pro Pro Asp Thr Ser
1               5                   10                  15

Leu Lys Pro Leu Met Thr Glu Leu Leu Lys Arg Ile Leu Asp Ser
            20                  25                  30

<210> 233
<211> 31
<212> PRT
<213> Homo sapiens

<400> 233
Leu Thr Glu Lys Ser Val Gln Glu Asp Trp Gln His Phe Pro Arg Tyr
1               5                   10                  15

Arg Thr Ala Ser Gln Gly Pro Gln Thr Asp Ser Val Ile Gln Asn
            20                  25                  30

<210> 234
<211> 31
<212> PRT
<213> Homo sapiens

<400> 234
Leu Thr Glu Lys Ser Val Gln Glu Asp Trp Gln His Phe Pro Arg Ser
1               5                   10                  15

Arg Thr Ala Ser Gln Gly Pro Gln Thr Asp Ser Val Ile Gln Asn
            20                  25                  30

<210> 235
<211> 31
<212> PRT
<213> Homo sapiens

<400> 235
Leu Thr Leu Ser Gly Ile Cys Ala Phe Ile Ser Gly Arg Phe Pro Tyr
1               5                   10                  15

Tyr Arg Arg Lys Phe Pro Ala Trp Gln Asn Ser Ile Arg His Asn
            20                  25                  30

<210> 236
<211> 31
<212> PRT
<213> Homo sapiens

<400> 236
Leu Thr Leu Ser Gly Ile Cys Ala Phe Ile Ser Gly Arg Phe Pro Ser
1               5                   10                  15

Tyr Arg Arg Lys Phe Pro Ala Trp Gln Asn Ser Ile Arg His Asn
            20                  25                  30

<210> 237
<211> 31
<212> PRT
<213> Homo sapiens

<400> 237
Leu Thr Gln Met Tyr Phe Phe Leu Ala Phe Gly Asn Thr Asp Ser Tyr
1               5                   10                  15

Leu Leu Ala Ala Met Ala Ile Asp Arg Tyr Val Ala Ile Cys Asn
            20                  25                  30

<210> 238
<211> 31
<212> PRT
<213> Homo sapiens

<400> 238
Leu Thr Gln Met Tyr Phe Phe Leu Ala Phe Gly Asn Thr Asp Ser Ser
1               5                   10                  15

Leu Leu Ala Ala Met Ala Ile Asp Arg Tyr Val Ala Ile Cys Asn

20                  25                  30

<210> 239
<211> 31
<212> PRT
<213> Homo sapiens

<400> 239
Leu Val Pro Ser Gly Ile Leu Ile Ala Ile Asp Ala Leu Ser Phe Tyr
1               5                   10                  15

Leu Pro Leu Glu Ser Gly Asn Cys Ala Pro Phe Lys Met Thr Val
            20                  25                  30

<210> 240
<211> 31
<212> PRT
<213> Homo sapiens

<400> 240
Leu Val Pro Ser Gly Ile Leu Ile Ala Ile Asp Ala Leu Ser Phe Ser
1               5                   10                  15

Leu Pro Leu Glu Ser Gly Asn Cys Ala Pro Phe Lys Met Thr Val
            20                  25                  30

<210> 241
<211> 31
<212> PRT
<213> Homo sapiens

<400> 241
Met Ala Ser Gln Val His His Ser Arg Ser Asn Pro Thr Asp Ile Tyr
1               5                   10                  15

Pro Ser Lys Trp Ile Ala Arg Leu Arg His Ile Lys Arg Leu Arg
            20                  25                  30

<210> 242
<211> 31
<212> PRT
<213> Homo sapiens

<400> 242
Met Ala Ser Gln Val His His Ser Arg Ser Asn Pro Thr Asp Ile Ser
1               5                   10                  15

Pro Ser Lys Trp Ile Ala Arg Leu Arg His Ile Lys Arg Leu Arg
            20                  25                  30

<210> 243
<211> 18
<212> PRT
<213> Homo sapiens

<400> 243
Met Asp Tyr Ser Leu Ser Ser Ser Phe Pro Thr Ser Pro Val Asn Ser
1               5                   10                  15

Asp Phe

<210> 244
<211> 18
<212> PRT
<213> Homo sapiens

<400> 244
Met Asp Ser Ser Leu Ser Ser Ser Phe Pro Thr Ser Pro Val Asn Ser
1               5                   10                  15

Asp Phe

<210> 245
<211> 31
<212> PRT
<213> Homo sapiens

<400> 245
Met Ile Ala Ile Val Leu Ala Ile Phe Leu Val Cys Phe Val Pro Tyr
1               5                   10                  15

His Val Asn Arg Ser Val Tyr Val Leu His Tyr Arg Ser His Gly
            20                  25                  30

<210> 246
<211> 31
<212> PRT
<213> Homo sapiens

<400> 246
Met Ile Ala Ile Val Leu Ala Ile Phe Leu Val Cys Phe Val Pro Ser
1               5                   10                  15

His Val Asn Arg Ser Val Tyr Val Leu His Tyr Arg Ser His Gly
            20                  25                  30

<210> 247
<211> 31
<212> PRT
<213> Homo sapiens

<400> 247
Met Ile His Arg Ser Thr Ser Gln Gly Ser Ile Asn Ser Pro Val Tyr
1               5                   10                  15

Ser Arg His Ser Tyr Thr Pro Thr Thr Ser Arg Ser Pro Gln His

20                  25                  30

<210> 248
<211> 31
<212> PRT
<213> Homo sapiens

<400> 248
Met Ile His Arg Ser Thr Ser Gln Gly Ser Ile Asn Ser Pro Val Ser
1               5                   10                  15

Ser Arg His Ser Tyr Thr Pro Thr Thr Ser Arg Ser Pro Gln His
            20                  25                  30

<210> 249
<211> 31
<212> PRT
<213> Homo sapiens

<400> 249
Asn Ala Glu Lys Lys Gly Val Tyr Gln Thr Leu Ser Trp Lys Arg Tyr
1               5                   10                  15

Gln Pro Cys Trp Val Leu Met Val Ser Val Leu Leu His His Trp
            20                  25                  30

<210> 250
<211> 31
<212> PRT
<213> Homo sapiens

<400> 250
Asn Ala Glu Lys Lys Gly Val Tyr Gln Thr Leu Ser Trp Lys Arg Ser
1               5                   10                  15

Gln Pro Cys Trp Val Leu Met Val Ser Val Leu Leu His His Trp
            20                  25                  30

<210> 251
<211> 31
<212> PRT
<213> Homo sapiens

<400> 251
Asn Leu Gln Lys Lys Asp Ala Asp Leu Arg Ala Met Glu Glu Arg Tyr
1               5                   10                  15

Arg Arg Tyr Val Asp Lys Ala Arg Met Val Met Gln Thr Met Glu
            20                  25                  30

<210> 252
<211> 31
<212> PRT
<213> Homo sapiens

<400> 252
Asn Leu Gln Lys Lys Asp Ala Asp Leu Arg Ala Met Glu Glu Arg Ser
1               5                   10                  15

Arg Arg Tyr Val Asp Lys Ala Arg Met Val Met Gln Thr Met Glu
            20                  25                  30

<210> 253
<211> 31
<212> PRT
<213> Homo sapiens

<400> 253
Pro Ala Gln Phe Gln Val Arg Pro Ile Pro Gln His Tyr Gln His Tyr
1               5                   10                  15

Leu Ala Thr Pro Arg Met His His Phe Pro Arg Asn Ser Ser Ser
            20                  25                  30

<210> 254
<211> 31
<212> PRT
<213> Homo sapiens

<400> 254
Pro Ala Gln Phe Gln Val Arg Pro Ile Pro Gln His Tyr Gln His Ser
1               5                   10                  15

Leu Ala Thr Pro Arg Met His His Phe Pro Arg Asn Ser Ser Ser
            20                  25                  30

<210> 255
<211> 31
<212> PRT
<213> Homo sapiens

<400> 255
Pro Cys Glu Ile Pro Phe Glu Ser Leu Thr Thr Gln Lys Gln Ser Tyr
1               5                   10                  15

Arg Gly Leu Met Gly Glu Pro Ala Lys Ser Leu Lys Pro Leu Ala
            20                  25                  30

<210> 256
<211> 31
<212> PRT
<213> Homo sapiens

<400> 256
Pro Cys Glu Ile Pro Phe Glu Ser Leu Thr Thr Gln Lys Gln Ser Ser
1               5                   10                  15

Arg Gly Leu Met Gly Glu Pro Ala Lys Ser Leu Lys Pro Leu Ala

20                  25                  30

<210> 257
<211> 31
<212> PRT
<213> Homo sapiens

<400> 257
Pro Arg Ser Gly Cys Gly Leu Thr Arg Ser Asn Arg Asn Asp Asp Tyr
1               5                   10                  15

Thr Leu Ser Val Arg Pro Pro Val Tyr Leu His Asp Val Ile Leu
            20                  25                  30

<210> 258
<211> 31
<212> PRT
<213> Homo sapiens

<400> 258
Pro Arg Ser Gly Cys Gly Leu Thr Arg Ser Asn Arg Asn Asp Asp Ser
1               5                   10                  15

Thr Leu Ser Val Arg Pro Pro Val Tyr Leu His Asp Val Ile Leu
            20                  25                  30

<210> 259
<211> 31
<212> PRT
<213> Homo sapiens

<400> 259
Gln Ala Glu Ile Leu Lys Arg Leu Ser Cys Ser Glu Leu Ser Leu Tyr
1               5                   10                  15

Gln Pro Leu Gln Asn Ser Ser Lys Glu Lys Asn Asp Lys Ala Ser
            20                  25                  30

<210> 260
<211> 31
<212> PRT
<213> Homo sapiens

<400> 260
Gln Ala Glu Ile Leu Lys Arg Leu Ser Cys Ser Glu Leu Ser Leu Ser
1               5                   10                  15

Gln Pro Leu Gln Asn Ser Ser Lys Glu Lys Asn Asp Lys Ala Ser
            20                  25                  30

<210> 261
<211> 31
<212> PRT
<213> Homo sapiens

<400> 261
Gln Ala Pro Val Cys Gly Phe Pro Cys Asn His Arg His Leu Phe Tyr
1               5                   10                  15

Ser Glu His Gly Arg Ile Leu Ser Tyr Ala Ala Ser Ser Gln Gln
            20                  25                  30

<210> 262
<211> 31
<212> PRT
<213> Homo sapiens

<400> 262
Gln Ala Pro Val Cys Gly Phe Pro Cys Asn His Arg His Leu Phe Ser
1               5                   10                  15

Ser Glu His Gly Arg Ile Leu Ser Tyr Ala Ala Ser Ser Gln Gln
            20                  25                  30

<210> 263
<211> 31
<212> PRT
<213> Homo sapiens

<400> 263
Gln Cys Leu Gln Arg Trp Asp Asp Thr Thr Cys Pro Val Cys Arg Tyr
1               5                   10                  15

Cys Gln Thr Pro Glu Pro Val Glu Glu Asn Lys Cys Phe Glu Cys
            20                  25                  30

<210> 264
<211> 31
<212> PRT
<213> Homo sapiens

<400> 264
Gln Cys Leu Gln Arg Trp Asp Asp Thr Thr Cys Pro Val Cys Arg Ser
1               5                   10                  15

Cys Gln Thr Pro Glu Pro Val Glu Glu Asn Lys Cys Phe Glu Cys
            20                  25                  30

<210> 265
<211> 31
<212> PRT
<213> Homo sapiens

<400> 265
Gln Glu Cys Gln Arg Lys Leu Asp His Lys Leu Ser Leu Asp Ser Tyr
1               5                   10                  15

Leu Leu Lys Pro Val Gln Arg Ile Thr Lys Tyr Gln Leu Leu Leu

20                  25                  30

<210> 266
<211> 31
<212> PRT
<213> Homo sapiens

<400> 266
Gln Glu Cys Gln Arg Lys Leu Asp His Lys Leu Ser Leu Asp Ser Ser
1               5                   10                  15

Leu Leu Lys Pro Val Gln Arg Ile Thr Lys Tyr Gln Leu Leu Leu
            20                  25                  30

<210> 267
<211> 31
<212> PRT
<213> Homo sapiens

<400> 267
Gln Glu Asn Pro Pro Asp His Asp Pro Ile His Asp Gln Ser Trp Tyr
1               5                   10                  15

Leu Asp Gln Thr Leu Arg Lys Arg Leu Tyr Glu Glu Tyr Gly Val
            20                  25                  30

<210> 268
<211> 31
<212> PRT
<213> Homo sapiens

<400> 268
Gln Glu Asn Pro Pro Asp His Asp Pro Ile His Asp Gln Ser Trp Ser
1               5                   10                  15

Leu Asp Gln Thr Leu Arg Lys Arg Leu Tyr Glu Glu Tyr Gly Val
            20                  25                  30

<210> 269
<211> 31
<212> PRT
<213> Homo sapiens

<400> 269
Gln Gln Glu Asp Asp Glu Phe Val Cys Gln Ile Ile Tyr Val Phe Tyr
1               5                   10                  15

Gln Met Val Phe His Gln Ala Thr Arg Asp Val Ile Ile Lys Glu
            20                  25                  30

<210> 270
<211> 31
<212> PRT
<213> Homo sapiens

<400> 270
Gln Gln Glu Asp Asp Glu Phe Val Cys Gln Ile Ile Tyr Val Phe Ser
1               5                   10                  15

Gln Met Val Phe His Gln Ala Thr Arg Asp Val Ile Ile Lys Glu
            20                  25                  30

<210> 271
<211> 31
<212> PRT
<213> Homo sapiens

<400> 271
Gln Arg Met Pro Gln Met Leu Pro Gln Cys Cys His Pro Cys Pro Tyr
1               5                   10                  15

His His Pro Leu Thr Ser His Ser Ser His Gln Glu Cys His Pro
            20                  25                  30

<210> 272
<211> 31
<212> PRT
<213> Homo sapiens

<400> 272
Gln Arg Met Pro Gln Met Leu Pro Gln Cys Cys His Pro Cys Pro Ser
1               5                   10                  15

His His Pro Leu Thr Ser His Ser Ser His Gln Glu Cys His Pro
            20                  25                  30

<210> 273
<211> 31
<212> PRT
<213> Homo sapiens

<400> 273
Arg Cys His Thr Ser Val Lys Pro His Lys Cys His Leu Cys Asp Tyr
1               5                   10                  15

Ala Ala Val Asp Ser Ser Ser Leu Lys Lys His Leu Arg Ile His
            20                  25                  30

<210> 274
<211> 31
<212> PRT
<213> Homo sapiens

<400> 274
Arg Cys His Thr Ser Val Lys Pro His Lys Cys His Leu Cys Asp Ser
1               5                   10                  15

Ala Ala Val Asp Ser Ser Ser Leu Lys Lys His Leu Arg Ile His

20                  25                  30


<210> 275
<211> 31
<212> PRT
<213> Homo sapiens

<400> 275
Arg Ile His Ser Asp Glu Arg Pro Phe Lys Cys Gln Ile Cys Pro Tyr
1               5                   10                  15


Ala Ser Arg Asn Ser Ser Gln Leu Thr Val His Leu Arg Ser His
            20                  25                  30


<210> 276
<211> 31
<212> PRT
<213> Homo sapiens

<400> 276
Arg Ile His Ser Asp Glu Arg Pro Phe Lys Cys Gln Ile Cys Pro Ser
1               5                   10                  15


Ala Ser Arg Asn Ser Ser Gln Leu Thr Val His Leu Arg Ser His
            20                  25                  30


<210> 277
<211> 31
<212> PRT
<213> Homo sapiens

<400> 277
Arg Lys Ser Ala Pro Ala Thr Gly Gly Val Lys Lys Pro His Arg Tyr
1               5                   10                  15


Arg Pro Gly Thr Val Ala Leu Arg Glu Ile Arg Arg Tyr Gln Lys
            20                  25                  30


<210> 278
<211> 31
<212> PRT
<213> Homo sapiens

<400> 278
Arg Lys Ser Ala Pro Ala Thr Gly Gly Val Lys Lys Pro His Arg Ser
1               5                   10                  15


Arg Pro Gly Thr Val Ala Leu Arg Glu Ile Arg Arg Tyr Gln Lys
            20                  25                  30


<210> 279
<211> 31
<212> PRT
<213> Homo sapiens

<400> 279
Arg Thr Glu Gly Ser His Ile Thr Ile Trp Cys Asn Val Ser Gly Tyr
1               5                   10                  15

Gln Gly Pro Ser Glu Gln Asn Phe Gln Trp Ser Ile Tyr Leu Pro
            20                  25                  30

<210> 280
<211> 31
<212> PRT
<213> Homo sapiens

<400> 280
Arg Thr Glu Gly Ser His Ile Thr Ile Trp Cys Asn Val Ser Gly Ser
1               5                   10                  15

Gln Gly Pro Ser Glu Gln Asn Phe Gln Trp Ser Ile Tyr Leu Pro
            20                  25                  30

<210> 281
<211> 31
<212> PRT
<213> Homo sapiens

<400> 281
Ser Lys Met Leu Arg Ala Thr Ala Pro Cys Trp Phe Arg Pro Gly Tyr
1               5                   10                  15

Pro Glu Ala Lys Lys Val Ala Lys Glu Ala Ala Pro Glu Ala Ser
            20                  25                  30

<210> 282
<211> 31
<212> PRT
<213> Homo sapiens

<400> 282
Ser Lys Met Leu Arg Ala Thr Ala Pro Cys Trp Phe Arg Pro Gly Ser
1               5                   10                  15

Pro Glu Ala Lys Lys Val Ala Lys Glu Ala Ala Pro Glu Ala Ser
            20                  25                  30

<210> 283
<211> 31
<212> PRT
<213> Homo sapiens

<400> 283
Ser Leu Ala Lys Leu Leu Gln Glu Arg Gly Ile Ser Ala Lys Val Tyr
1               5                   10                  15

His Ser Pro Ile Ser Glu Asn Pro Leu Gln Pro Leu Pro Lys Ser

20                  25                  30

<210> 284
<211> 31
<212> PRT
<213> Homo sapiens

<400> 284
Ser Leu Ala Lys Leu Leu Gln Glu Arg Gly Ile Ser Ala Lys Val Ser
1               5                   10                  15

His Ser Pro Ile Ser Glu Asn Pro Leu Gln Pro Leu Pro Lys Ser
            20                  25                  30

<210> 285
<211> 31
<212> PRT
<213> Homo sapiens

<400> 285
Ser Leu Glu Ile Val Leu Lys Asn Ile Ser His Leu Ile Ser Ala Tyr
1               5                   10                  15

Leu Pro Lys Ile Leu Gln Ile Leu Leu Cys Met Thr Ala Thr Val
            20                  25                  30

<210> 286
<211> 31
<212> PRT
<213> Homo sapiens

<400> 286
Ser Leu Glu Ile Val Leu Lys Asn Ile Ser His Leu Ile Ser Ala Ser
1               5                   10                  15

Leu Pro Lys Ile Leu Gln Ile Leu Leu Cys Met Thr Ala Thr Val
            20                  25                  30

<210> 287
<211> 31
<212> PRT
<213> Homo sapiens

<400> 287
Ser Gln Pro Gln Ser Ala Ala Thr Ala Pro Ser Ala Met Phe Pro Tyr
1               5                   10                  15

Pro Gly Gln His Gly Pro Ala His Pro Ala Phe Ser Ile Gly Ser
            20                  25                  30

<210> 288
<211> 31
<212> PRT
<213> Homo sapiens

<400> 288
Ser Gln Pro Gln Ser Ala Ala Thr Ala Pro Ser Ala Met Phe Pro Ser
1               5                   10                  15

Pro Gly Gln His Gly Pro Ala His Pro Ala Phe Ser Ile Gly Ser
            20                  25                  30

<210> 289
<211> 31
<212> PRT
<213> Homo sapiens

<400> 289
Ser Trp Asp Val Pro Glu Asn Asp Gly Gly Ala Pro Val Lys Asn Tyr
1               5                   10                  15

His Ile Glu Lys Arg Glu Ala Ser Lys Lys Ala Trp Val Ser Val
            20                  25                  30

<210> 290
<211> 31
<212> PRT
<213> Homo sapiens

<400> 290
Ser Trp Asp Val Pro Glu Asn Asp Gly Gly Ala Pro Val Lys Asn Ser
1               5                   10                  15

His Ile Glu Lys Arg Glu Ala Ser Lys Lys Ala Trp Val Ser Val
            20                  25                  30

<210> 291
<211> 31
<212> PRT
<213> Homo sapiens

<400> 291
Thr Glu Gln Leu Ile Ile Arg Ala Pro Leu Thr Asp Lys His Ile Tyr
1               5                   10                  15

Gln Pro Tyr Pro Ser Gly Ala Asp Val Pro Phe Gly Pro Pro Leu
            20                  25                  30

<210> 292
<211> 31
<212> PRT
<213> Homo sapiens

<400> 292
Thr Glu Gln Leu Ile Ile Arg Ala Pro Leu Thr Asp Lys His Ile Ser
1               5                   10                  15

Gln Pro Tyr Pro Ser Gly Ala Asp Val Pro Phe Gly Pro Pro Leu

20                  25                  30


<210> 293
<211> 31
<212> PRT
<213> Homo sapiens

<400> 293
Thr Phe Phe Cys Pro Asp Cys Ser His Ala Pro Leu Trp Leu Met Tyr
1               5                   10                  15


Leu Ala Ile Val Leu Ser His Thr Asn Ser Val Val Asn Pro Phe
            20                  25                  30


<210> 294
<211> 31
<212> PRT
<213> Homo sapiens

<400> 294
Thr Phe Phe Cys Pro Asp Cys Ser His Ala Pro Leu Trp Leu Met Ser
1               5                   10                  15


Leu Ala Ile Val Leu Ser His Thr Asn Ser Val Val Asn Pro Phe
            20                  25                  30


<210> 295
<211> 31
<212> PRT
<213> Homo sapiens

<400> 295
Thr His Lys Asn Leu Ser Asp Met Glu Asn Glu Phe Tyr Tyr Arg Tyr
1               5                   10                  15


Pro Ser Phe Gln Asp Val His Val Met Val Phe Val Gly Phe Gly
            20                  25                  30


<210> 296
<211> 31
<212> PRT
<213> Homo sapiens

<400> 296
Thr His Lys Asn Leu Ser Asp Met Glu Asn Glu Phe Tyr Tyr Arg Ser
1               5                   10                  15


Pro Ser Phe Gln Asp Val His Val Met Val Phe Val Gly Phe Gly
            20                  25                  30


<210> 297
<211> 31
<212> PRT
<213> Homo sapiens

<400> 297
Thr Leu Lys Pro Gln Asp Gln Leu Ser Ala Leu Gln Leu Leu Val Tyr
1               5                   10                  15

Leu Met Pro Pro Cys His Ser Asp Thr Leu Glu Arg Leu Leu Lys
            20                  25                  30

<210> 298
<211> 31
<212> PRT
<213> Homo sapiens

<400> 298
Thr Leu Lys Pro Gln Asp Gln Leu Ser Ala Leu Gln Leu Leu Val Ser
1               5                   10                  15

Leu Met Pro Pro Cys His Ser Asp Thr Leu Glu Arg Leu Leu Lys
            20                  25                  30

<210> 299
<211> 31
<212> PRT
<213> Homo sapiens

<400> 299
Thr Leu Asn Gly Ile Tyr Gln Phe Ile Met Asp Arg Phe Pro Phe Tyr
1               5                   10                  15

Arg Asp Asn Lys Gln Gly Trp Gln Asn Ser Ile Arg His Asn Leu
            20                  25                  30

<210> 300
<211> 31
<212> PRT
<213> Homo sapiens

<400> 300
Thr Leu Asn Gly Ile Tyr Gln Phe Ile Met Asp Arg Phe Pro Phe Ser
1               5                   10                  15

Arg Asp Asn Lys Gln Gly Trp Gln Asn Ser Ile Arg His Asn Leu
            20                  25                  30

<210> 301
<211> 31
<212> PRT
<213> Homo sapiens

<400> 301
Thr Leu Asn Gly Ile Tyr Gln Phe Ile Met Asp Arg Phe Pro Phe Tyr
1               5                   10                  15

His Asp Asn Arg Gln Gly Trp Gln Asn Ser Ile Arg His Asn Leu

20                  25                  30


<210> 302
<211> 31
<212> PRT
<213> Homo sapiens

<400> 302
Thr Leu Asn Gly Ile Tyr Gln Phe Ile Met Asp Arg Phe Pro Phe Ser
1               5                   10                  15


His Asp Asn Arg Gln Gly Trp Gln Asn Ser Ile Arg His Asn Leu
            20                  25                  30


<210> 303
<211> 31
<212> PRT
<213> Homo sapiens

<400> 303
Val Phe Pro Tyr Thr Ser Lys Ser Cys Met Arg Ala Val Leu Glu Tyr
1               5                   10                  15


Leu Tyr Thr Gly Met Phe Thr Ser Ser Pro Asp Leu Asp Asp Met
            20                  25                  30


<210> 304
<211> 31
<212> PRT
<213> Homo sapiens

<400> 304
Val Phe Pro Tyr Thr Ser Lys Ser Cys Met Arg Ala Val Leu Glu Ser
1               5                   10                  15


Leu Tyr Thr Gly Met Phe Thr Ser Ser Pro Asp Leu Asp Asp Met
            20                  25                  30


<210> 305
<211> 31
<212> PRT
<213> Homo sapiens

<400> 305
Val Gly Phe Met Leu Tyr Arg Met Lys Lys Lys Asp Glu Gly Ser Tyr
1               5                   10                  15


Ser Leu Glu Glu Pro Lys Gln Ala Asn Gly Gly Ala Tyr Gln Lys
            20                  25                  30


<210> 306
<211> 31
<212> PRT
<213> Homo sapiens

<400> 306
Val Gly Phe Met Leu Tyr Arg Met Lys Lys Lys Asp Glu Gly Ser Ser
1               5                   10                  15

Ser Leu Glu Glu Pro Lys Gln Ala Asn Gly Gly Ala Tyr Gln Lys
            20                  25                  30

<210> 307
<211> 31
<212> PRT
<213> Homo sapiens

<400> 307
Val Gly Thr Tyr Asn Thr Arg Lys Tyr Glu Cys Cys Ala Glu Ile Tyr
1               5                   10                  15

Pro Asp Ile Thr Tyr Ala Phe Val Ile Arg Arg Leu Pro Leu Phe
            20                  25                  30

<210> 308
<211> 31
<212> PRT
<213> Homo sapiens

<400> 308
Val Gly Thr Tyr Asn Thr Arg Lys Tyr Glu Cys Cys Ala Glu Ile Ser
1               5                   10                  15

Pro Asp Ile Thr Tyr Ala Phe Val Ile Arg Arg Leu Pro Leu Phe
            20                  25                  30

<210> 309
<211> 31
<212> PRT
<213> Homo sapiens

<400> 309
Val Arg Ser Thr Ala Pro Ala Val Ala Tyr Asp Ser Lys Gln Tyr Tyr
1               5                   10                  15

Gln Gln Pro Thr Ala Thr Ala Ala Ala Val Ala Ala Ala Ala Gln
            20                  25                  30

<210> 310
<211> 31
<212> PRT
<213> Homo sapiens

<400> 310
Val Arg Ser Thr Ala Pro Ala Val Ala Tyr Asp Ser Lys Gln Tyr Ser
1               5                   10                  15

Gln Gln Pro Thr Ala Thr Ala Ala Ala Val Ala Ala Ala Ala Gln

20                  25                  30


<210> 311
<211> 31
<212> PRT
<213> Homo sapiens

<400> 311
Val Ser Ala Thr Asp Arg Asp Ser Gly Thr Asn Ala Gln Val Thr Tyr
1               5                   10                  15


Ser Leu Leu Pro Pro Gln Asp Pro His Leu Pro Leu Thr Ser Leu
            20                  25                  30


<210> 312
<211> 31
<212> PRT
<213> Homo sapiens

<400> 312
Val Ser Ala Thr Asp Arg Asp Ser Gly Thr Asn Ala Gln Val Thr Ser
1               5                   10                  15


Ser Leu Leu Pro Pro Gln Asp Pro His Leu Pro Leu Thr Ser Leu
            20                  25                  30


<210> 313
<211> 31
<212> PRT
<213> Homo sapiens

<400> 313
Val Ser Phe Trp Asp Leu Asn Tyr Gly Asp Leu Leu Gln Thr Val Tyr
1               5                   10                  15


Leu Gly Lys Asn Ser Glu Ala Gln Pro Ala Arg Gln Ile Leu Val
            20                  25                  30


<210> 314
<211> 31
<212> PRT
<213> Homo sapiens

<400> 314
Val Ser Phe Trp Asp Leu Asn Tyr Gly Asp Leu Leu Gln Thr Val Ser
1               5                   10                  15


Leu Gly Lys Asn Ser Glu Ala Gln Pro Ala Arg Gln Ile Leu Val
            20                  25                  30


<210> 315
<211> 31
<212> PRT
<213> Homo sapiens

<400> 315
Val Thr Trp Tyr Lys Gly Pro Thr Glu Leu Thr Glu Ser Gln Lys Tyr
1               5                   10                  15

Asn Phe Arg Asn Asp Gly Arg Cys His Tyr Met Thr Ile His Asn
            20                  25                  30

<210> 316
<211> 31
<212> PRT
<213> Homo sapiens

<400> 316
Val Thr Trp Tyr Lys Gly Pro Thr Glu Leu Thr Glu Ser Gln Lys Ser
1               5                   10                  15

Asn Phe Arg Asn Asp Gly Arg Cys His Tyr Met Thr Ile His Asn
            20                  25                  30

<210> 317
<211> 31
<212> PRT
<213> Homo sapiens

<400> 317
Val Val Gly Phe His His Lys Lys Gly Cys Gln Val Glu Phe Ser Tyr
1               5                   10                  15

Pro Pro Leu Ile Pro Gly Asp Gly His Asp Ser His Thr Leu Pro
            20                  25                  30

<210> 318
<211> 31
<212> PRT
<213> Homo sapiens

<400> 318
Val Val Gly Phe His His Lys Lys Gly Cys Gln Val Glu Phe Ser Ser
1               5                   10                  15

Pro Pro Leu Ile Pro Gly Asp Gly His Asp Ser His Thr Leu Pro
            20                  25                  30

<210> 319
<211> 31
<212> PRT
<213> Homo sapiens

<400> 319
Trp Leu Leu Lys Asp Gly Leu Lys Asp Ser Gln Phe Ser Ile Arg Tyr
1               5                   10                  15

Gln Tyr Leu Leu Ala Ala Leu Leu Cys Cys Cys Gly Lys Gly Leu

20                  25                  30


<210> 320
<211> 31
<212> PRT
<213> Homo sapiens

<400> 320
Trp Leu Leu Lys Asp Gly Leu Lys Asp Ser Gln Phe Ser Ile Arg Ser
1               5                   10                  15


Gln Tyr Leu Leu Ala Ala Leu Leu Cys Cys Cys Gly Lys Gly Leu
            20                  25                  30


<210> 321
<211> 31
<212> PRT
<213> Homo sapiens

<400> 321
Trp Pro Cys Ser Phe Ser Pro Ser Gln Asn Ser Ser Glu Pro Phe Tyr
1               5                   10                  15


Gln Gln Leu Pro Leu Glu Pro Pro Ala Ala Lys Thr Gly Cys Pro
            20                  25                  30


<210> 322
<211> 31
<212> PRT
<213> Homo sapiens

<400> 322
Trp Pro Cys Ser Phe Ser Pro Ser Gln Asn Ser Ser Glu Pro Phe Ser
1               5                   10                  15


Gln Gln Leu Pro Leu Glu Pro Pro Ala Ala Lys Thr Gly Cys Pro
            20                  25                  30


<210> 323
<211> 31
<212> PRT
<213> Homo sapiens

<400> 323
Trp Thr Phe Pro Ile Met Ala Leu Pro Ile Asn Ala Tyr Ile Ser Tyr
1               5                   10                  15


Leu Gly Phe Arg Phe Tyr Val Asp Ala Asp Arg Arg Ser Ser Arg
            20                  25                  30


<210> 324
<211> 31
<212> PRT
<213> Homo sapiens

<400> 324
Trp Thr Phe Pro Ile Met Ala Leu Pro Ile Asn Ala Tyr Ile Ser Ser
1               5                   10                  15

Leu Gly Phe Arg Phe Tyr Val Asp Ala Asp Arg Arg Ser Ser Arg
            20                  25                  30

<210> 325
<211> 31
<212> PRT
<213> Homo sapiens

<400> 325
Tyr Gly Ala Ala Phe Thr Ala Glu Lys Pro Asn Ser Pro Met Met Tyr
1               5                   10                  15

Pro Gln Ala Phe Asn Asn Gln Asn Pro Ile Val Pro Pro Met Ala
            20                  25                  30

<210> 326
<211> 31
<212> PRT
<213> Homo sapiens

<400> 326
Tyr Gly Ala Ala Phe Thr Ala Glu Lys Pro Asn Ser Pro Met Met Ser
1               5                   10                  15

Pro Gln Ala Phe Asn Asn Gln Asn Pro Ile Val Pro Pro Met Ala
            20                  25                  30

<210> 327
<211> 31
<212> PRT
<213> Homo sapiens

<400> 327
Tyr Gly Glu Ile Cys Gly Phe Ser Asp Thr Asn Leu Gln Lys Leu Tyr
1               5                   10                  15

Phe Gln Leu Arg Leu Asn Gln Pro Tyr Cys Gly Tyr Ala Val Gly
            20                  25                  30

<210> 328
<211> 31
<212> PRT
<213> Homo sapiens

<400> 328
Tyr Gly Glu Ile Cys Gly Phe Ser Asp Thr Asn Leu Gln Lys Leu Ser
1               5                   10                  15

Phe Gln Leu Arg Leu Asn Gln Pro Tyr Cys Gly Tyr Ala Val Gly

20                  25                  30


<210> 329
<211> 31
<212> PRT
<213> Homo sapiens

<400> 329
Tyr His His Gln Met Pro Pro Pro His Ser Asp Thr Val Glu Phe Tyr
1               5                   10                  15


Gln Arg Leu Ser Thr Glu Thr Leu Phe Phe Ile Phe Tyr Tyr Leu
            20                  25                  30


<210> 330
<211> 31
<212> PRT
<213> Homo sapiens

<400> 330
Tyr His His Gln Met Pro Pro Pro His Ser Asp Thr Val Glu Phe Ser
1               5                   10                  15


Gln Arg Leu Ser Thr Glu Thr Leu Phe Phe Ile Phe Tyr Tyr Leu
            20                  25                  30


<210> 331
<211> 31
<212> PRT
<213> Homo sapiens

<400> 331
Tyr Gln Val Thr Asn Leu Val Pro Gly Thr Lys Phe Tyr Ile Ser Tyr
1               5                   10                  15


Leu Val Lys Lys Gly Thr Ala Thr Glu Ser Ser Arg Glu Ile Pro
            20                  25                  30


<210> 332
<211> 31
<212> PRT
<213> Homo sapiens

<400> 332
Tyr Gln Val Thr Asn Leu Val Pro Gly Thr Lys Phe Tyr Ile Ser Ser
1               5                   10                  15


Leu Val Lys Lys Gly Thr Ala Thr Glu Ser Ser Arg Glu Ile Pro
            20                  25                  30


<210> 333
<211> 31
<212> PRT
<213> Homo sapiens

<400> 333
Tyr Ser Ser Asp Thr Glu Ser Leu Asn Gln Ala Asp Leu Pro Pro Tyr
1               5                   10                  15

Arg Ser Arg Ser Gly Ser Ala Asn Ser Ala Ser Ser Gln Ala Ala
            20                  25                  30

<210> 334
<211> 31
<212> PRT
<213> Homo sapiens

<400> 334
Tyr Ser Ser Asp Thr Glu Ser Leu Asn Gln Ala Asp Leu Pro Pro Ser
1               5                   10                  15

Arg Ser Arg Ser Gly Ser Ala Asn Ser Ala Ser Ser Gln Ala Ala
            20                  25                  30

<210> 335
<211> 31
<212> PRT
<213> Homo sapiens

<400> 335
Tyr Val Gly Leu Phe Gly His Pro Gly Met Leu His Arg Ala Lys Tyr
1               5                   10                  15

Ser Arg Phe Arg Asn Glu Ser Ile Thr Ser Leu Asp Glu Gly Ser
            20                  25                  30

<210> 336
<211> 31
<212> PRT
<213> Homo sapiens

<400> 336
Tyr Val Gly Leu Phe Gly His Pro Gly Met Leu His Arg Ala Lys Ser
1               5                   10                  15

Ser Arg Phe Arg Asn Glu Ser Ile Thr Ser Leu Asp Glu Gly Ser
            20                  25                  30

<210> 337
<211> 31
<212> PRT
<213> Homo sapiens

<400> 337
Leu Leu Glu Arg Leu Arg His Pro Asn Val Leu Gln Leu Tyr Gly Tyr
1               5                   10                  15

Cys Tyr Gln Asp Ser Glu Asp Ile Pro Asp Thr Leu Thr Thr Ile

20                  25                  30

<210> 338
<211> 31
<212> PRT
<213> Homo sapiens

<220>
<221> MOD_RES
<222> (16)..(16)
<223> Any amino acid

<400> 338
Leu Leu Glu Arg Leu Arg His Pro Asn Val Leu Gln Leu Tyr Gly Xaa
1               5                   10                  15

Cys Tyr Gln Asp Ser Glu Asp Ile Pro Asp Thr Leu Thr Thr Ile
            20                  25                  30

<210> 339
<211> 31
<212> PRT
<213> Homo sapiens

<400> 339
Ile Thr Lys Met Ala Leu Tyr Lys Asn Ala Ser Tyr Lys His Pro Tyr
1               5                   10                  15

Arg Gln Gly Glu Val Val Leu Thr Thr Arg Asp Val Leu Tyr Val
            20                  25                  30

<210> 340
<211> 31
<212> PRT
<213> Homo sapiens

<220>
<221> MOD_RES
<222> (16)..(16)
<223> Any amino acid

<400> 340
Ile Thr Lys Met Ala Leu Tyr Lys Asn Ala Ser Tyr Lys His Pro Xaa
1               5                   10                  15

Arg Gln Gly Glu Val Val Leu Thr Thr Arg Asp Val Leu Tyr Val
            20                  25                  30

<210> 341
<211> 31
<212> PRT
<213> Homo sapiens

<400> 341

Arg Pro Glu Ser Ala Leu Ala Gln Ala Gln Lys Cys Phe Ala Leu Tyr
1               5                   10                  15

Arg Gln Ala Tyr Thr Gly Asn Asn Ser Ser Gln Ile Gln Ala Ala
            20                  25                  30

<210> 342
<211> 31
<212> PRT
<213> Homo sapiens

<220>
<221> MOD_RES
<222> (16)..(16)
<223> Any amino acid

<400> 342
Arg Pro Glu Ser Ala Leu Ala Gln Ala Gln Lys Cys Phe Ala Leu Xaa
1               5                   10                  15

Arg Gln Ala Tyr Thr Gly Asn Asn Ser Ser Gln Ile Gln Ala Ala
            20                  25                  30

<210> 343
<211> 31
<212> PRT
<213> Homo sapiens

<400> 343
Leu Phe Gly Gly Tyr Gly Tyr Ser Lys Thr Thr Asp Thr Tyr Gly Tyr
1               5                   10                  15

Ser Thr Pro His Gln Pro Tyr Pro Pro Pro Ala Ala Ala Ser Ser
            20                  25                  30

<210> 344
<211> 31
<212> PRT
<213> Homo sapiens

<220>
<221> MOD_RES
<222> (16)..(16)
<223> Any amino acid

<400> 344
Leu Phe Gly Gly Tyr Gly Tyr Ser Lys Thr Thr Asp Thr Tyr Gly Xaa
1               5                   10                  15

Ser Thr Pro His Gln Pro Tyr Pro Pro Pro Ala Ala Ala Ser Ser
            20                  25                  30

<210> 345

<211> 31
<212> PRT
<213> Homo sapiens

<400> 345
Trp Leu His Asn Gly Arg Ser Cys Phe Gly Val Asn Arg Ser Gly Tyr
1               5                   10                  15

Thr Leu Ile Arg Lys Asp Ser Glu Glu Glu Val Ser Leu Leu Gly
            20                  25                  30

<210> 346
<211> 31
<212> PRT
<213> Homo sapiens

<220>
<221> MOD_RES
<222> (16)..(16)
<223> Any amino acid

<400> 346
Trp Leu His Asn Gly Arg Ser Cys Phe Gly Val Asn Arg Ser Gly Xaa
1               5                   10                  15

Thr Leu Ile Arg Lys Asp Ser Glu Glu Glu Val Ser Leu Leu Gly
            20                  25                  30

<210> 347
<211> 31
<212> PRT
<213> Homo sapiens

<400> 347
Val Ala Asp Phe Gly Leu Ser Lys Lys Ile Tyr Asn Gly Asp Tyr Tyr
1               5                   10                  15

Arg Gln Gly Arg Ile Ala Lys Met Pro Val Lys Trp Ile Ala Ile
            20                  25                  30

<210> 348
<211> 31
<212> PRT
<213> Homo sapiens

<220>
<221> MOD_RES
<222> (16)..(16)
<223> Any amino acid

<400> 348
Val Ala Asp Phe Gly Leu Ser Lys Lys Ile Tyr Asn Gly Asp Tyr Xaa
1               5                   10                  15

Arg Gln Gly Arg Ile Ala Lys Met Pro Val Lys Trp Ile Ala Ile
            20                  25                  30

<210> 349
<211> 32
<212> PRT
<213> Homo sapiens

<400> 349
Lys Ile Val Ser Ser Ser Trp Asp Gly Asn Leu Arg Leu Trp Gln Tyr
1               5                   10                  15

Arg Gln Ala Glu Tyr Glu Phe Gln Asp Asp Met Pro Glu Ser Glu Glu
            20                  25                  30

<210> 350
<211> 32
<212> PRT
<213> Homo sapiens

<220>
<221> MOD_RES
<222> (16)..(16)
<223> Any amino acid

<400> 350
Lys Ile Val Ser Ser Ser Trp Asp Gly Asn Leu Arg Leu Trp Gln Xaa
1               5                   10                  15

Arg Gln Ala Glu Tyr Glu Phe Gln Asp Asp Met Pro Glu Ser Glu Glu
            20                  25                  30

<210> 351
<211> 31
<212> PRT
<213> Homo sapiens

<400> 351
Cys Glu Glu Glu Gly Asn Gly Ala Asp Asn Val Gln Tyr Cys Gly Tyr
1               5                   10                  15

Cys Lys Tyr His Phe Ser Lys Leu Lys Lys Ser Lys Arg Gly Ser
            20                  25                  30

<210> 352
<211> 31
<212> PRT
<213> Homo sapiens

<220>
<221> MOD_RES
<222> (16)..(16)
<223> Any amino acid

<400> 352
Cys Glu Glu Glu Gly Asn Gly Ala Asp Asn Val Gln Tyr Cys Gly Xaa
1               5                   10                  15

Cys Lys Tyr His Phe Ser Lys Leu Lys Lys Ser Lys Arg Gly Ser
            20                  25                  30

<210> 353
<211> 31
<212> PRT
<213> Homo sapiens

<400> 353
Cys Leu Cys Gly Ser Lys Leu Val Ile Asp Trp His Asn Tyr Gly Tyr
1               5                   10                  15

Ser Ile Met Gly Leu Val His Gly Pro Asn His Pro Leu Val Leu
            20                  25                  30

<210> 354
<211> 31
<212> PRT
<213> Homo sapiens

<220>
<221> MOD_RES
<222> (16)..(16)
<223> Any amino acid

<400> 354
Cys Leu Cys Gly Ser Lys Leu Val Ile Asp Trp His Asn Tyr Gly Xaa
1               5                   10                  15

Ser Ile Met Gly Leu Val His Gly Pro Asn His Pro Leu Val Leu
            20                  25                  30

<210> 355
<211> 31
<212> PRT
<213> Homo sapiens

<400> 355
Asp Asp Phe Glu Phe Thr Gly Ser His Leu Thr Val Arg Asn Gly Tyr
1               5                   10                  15

Ser Cys Val Pro Val Ala Leu Ala Glu Gly Leu Asp Ile Lys Leu
            20                  25                  30

<210> 356
<211> 31
<212> PRT
<213> Homo sapiens

<220>
<221> MOD_RES
<222> (16)..(16)
<223> Any amino acid

<400> 356
Asp Asp Phe Glu Phe Thr Gly Ser His Leu Thr Val Arg Asn Gly Xaa
1               5                   10                  15

Ser Cys Val Pro Val Ala Leu Ala Glu Gly Leu Asp Ile Lys Leu
            20                  25                  30

<210> 357
<211> 31
<212> PRT
<213> Homo sapiens

<400> 357
Asp Gly Trp Ser Glu Thr Phe Pro Asp Phe Val Asp Ala Cys Gly Tyr
1               5                   10                  15

Ser Asp Pro Glu Asp Glu Ser Lys Ile Thr Phe Tyr Ile Leu Val
            20                  25                  30

<210> 358
<211> 31
<212> PRT
<213> Homo sapiens

<220>
<221> MOD_RES
<222> (16)..(16)
<223> Any amino acid

<400> 358
Asp Gly Trp Ser Glu Thr Phe Pro Asp Phe Val Asp Ala Cys Gly Xaa
1               5                   10                  15

Ser Asp Pro Glu Asp Glu Ser Lys Ile Thr Phe Tyr Ile Leu Val
            20                  25                  30

<210> 359
<211> 31
<212> PRT
<213> Homo sapiens

<400> 359
Glu Lys Ala Val Val Trp Ala Lys His Val Ala Glu Lys Asn Gly Tyr
1               5                   10                  15

Leu Gly His Val Ile Arg Lys Gly Leu Asn Ala Tyr Leu Glu Gly
            20                  25                  30

<210> 360

<211> 31
<212> PRT
<213> Homo sapiens

<220>
<221> MOD_RES
<222> (16)..(16)
<223> Any amino acid

<400> 360
Glu Lys Ala Val Val Trp Ala Lys His Val Ala Glu Lys Asn Gly Xaa
1               5                   10                  15

Leu Gly His Val Ile Arg Lys Gly Leu Asn Ala Tyr Leu Glu Gly
            20                  25                  30

<210> 361
<211> 31
<212> PRT
<213> Homo sapiens

<400> 361
Phe Glu Asp Arg Val Tyr Trp Ile Asp Gly Glu Asn Glu Ala Val Tyr
1               5                   10                  15

Gly Ala Asn Lys Phe Thr Gly Ser Glu Leu Ala Thr Leu Val Asn
            20                  25                  30

<210> 362
<211> 31
<212> PRT
<213> Homo sapiens

<220>
<221> MOD_RES
<222> (16)..(16)
<223> Any amino acid

<400> 362
Phe Glu Asp Arg Val Tyr Trp Ile Asp Gly Glu Asn Glu Ala Val Xaa
1               5                   10                  15

Gly Ala Asn Lys Phe Thr Gly Ser Glu Leu Ala Thr Leu Val Asn
            20                  25                  30

<210> 363
<211> 31
<212> PRT
<213> Homo sapiens

<400> 363
Phe Arg Leu Ile Gly Trp Asn Asp Trp Ile Ile Ala Pro Thr Gly Tyr
1               5                   10                  15

Tyr Gly Asn Tyr Cys Glu Gly Ser Cys Pro Ala Tyr Leu Ala Gly
            20                  25                  30

<210> 364
<211> 31
<212> PRT
<213> Homo sapiens

<220>
<221> MOD_RES
<222> (16)..(16)
<223> Any amino acid

<400> 364
Phe Arg Leu Ile Gly Trp Asn Asp Trp Ile Ile Ala Pro Thr Gly Xaa
1               5                   10                  15

Tyr Gly Asn Tyr Cys Glu Gly Ser Cys Pro Ala Tyr Leu Ala Gly
            20                  25                  30

<210> 365
<211> 30
<212> PRT
<213> Homo sapiens

<400> 365
Gly Thr Met Ile Glu Trp Gly Asn Trp Ala Arg Ala Ile Lys Tyr Arg
1               5                   10                  15

Gln Glu Asn Gln Glu Ala Val Gly Gly Phe Phe Ser Gln Ile
            20                  25                  30

<210> 366
<211> 30
<212> PRT
<213> Homo sapiens

<220>
<221> MOD_RES
<222> (15)..(15)
<223> Any amino acid

<400> 366
Gly Thr Met Ile Glu Trp Gly Asn Trp Ala Arg Ala Ile Lys Xaa Arg
1               5                   10                  15

Gln Glu Asn Gln Glu Ala Val Gly Gly Phe Phe Ser Gln Ile
            20                  25                  30

<210> 367
<211> 31
<212> PRT
<213> Homo sapiens

<400> 367
His Leu Thr Lys Lys Leu Leu Asp Leu Val Gln Gln Ser Cys Asn Tyr
1               5                   10                  15

Lys Gln Leu Arg Lys Gly Ala Asn Glu Ala Thr Lys Thr Leu Asn
            20                  25                  30

<210> 368
<211> 31
<212> PRT
<213> Homo sapiens

<220>
<221> MOD_RES
<222> (16)..(16)
<223> Any amino acid

<400> 368
His Leu Thr Lys Lys Leu Leu Asp Leu Val Gln Gln Ser Cys Asn Xaa
1               5                   10                  15

Lys Gln Leu Arg Lys Gly Ala Asn Glu Ala Thr Lys Thr Leu Asn
            20                  25                  30

<210> 369
<211> 31
<212> PRT
<213> Homo sapiens

<400> 369
Leu Ala Gly Gly Arg His Cys Cys Pro Val Cys Arg Trp Pro Ser Tyr
1               5                   10                  15

Lys Lys Lys Gln Pro Tyr Ala Gln His Gln Pro Leu Ser Asn Asp
            20                  25                  30

<210> 370
<211> 31
<212> PRT
<213> Homo sapiens

<220>
<221> MOD_RES
<222> (16)..(16)
<223> Any amino acid

<400> 370
Leu Ala Gly Gly Arg His Cys Cys Pro Val Cys Arg Trp Pro Ser Xaa
1               5                   10                  15

Lys Lys Lys Gln Pro Tyr Ala Gln His Gln Pro Leu Ser Asn Asp
            20                  25                  30

<210> 371
<211> 31
<212> PRT
<213> Homo sapiens

<400> 371
Leu Ala Leu Leu Ala Val Pro Asp Met Ser Ser Leu Ala Cys Gly Tyr
1               5                   10                  15

Leu Arg Asn Leu Thr Trp Thr Leu Ser Asn Leu Cys Arg Asn Lys
            20                  25                  30

<210> 372
<211> 31
<212> PRT
<213> Homo sapiens

<220>
<221> MOD_RES
<222> (16)..(16)
<223> Any amino acid

<400> 372
Leu Ala Leu Leu Ala Val Pro Asp Met Ser Ser Leu Ala Cys Gly Xaa
1               5                   10                  15

Leu Arg Asn Leu Thr Trp Thr Leu Ser Asn Leu Cys Arg Asn Lys
            20                  25                  30

<210> 373
<211> 31
<212> PRT
<213> Homo sapiens

<400> 373
Met Ala Lys Glu Tyr Lys Asp Ala Phe Met Lys Ala Asn Pro Gly Tyr
1               5                   10                  15

Lys Trp Cys Pro Thr Thr Asn Lys Pro Val Lys Ser Pro Thr Pro
            20                  25                  30

<210> 374
<211> 31
<212> PRT
<213> Homo sapiens

<220>
<221> MOD_RES
<222> (16)..(16)
<223> Any amino acid

<400> 374
Met Ala Lys Glu Tyr Lys Asp Ala Phe Met Lys Ala Asn Pro Gly Xaa
1               5                   10                  15

Lys Trp Cys Pro Thr Thr Asn Lys Pro Val Lys Ser Pro Thr Pro
            20                  25                  30

<210> 375
<211> 26
<212> PRT
<213> Homo sapiens

<400> 375
Met Cys Tyr Gly Tyr Gly Cys Gly Cys Gly Tyr Gly Cys Gly Cys Gly
1               5                   10                  15

Ser Phe Cys Arg Leu Gly Tyr Gly Cys Gly
            20                  25

<210> 376
<211> 26
<212> PRT
<213> Homo sapiens

<220>
<221> MOD_RES
<222> (11)..(11)
<223> Any amino acid

<400> 376
Met Cys Tyr Gly Tyr Gly Cys Gly Cys Gly Xaa Gly Cys Gly Cys Gly
1               5                   10                  15

Ser Phe Cys Arg Leu Gly Tyr Gly Cys Gly
            20                  25

<210> 377
<211> 22
<212> PRT
<213> Homo sapiens

<400> 377
Met Trp Val Leu Leu Arg Tyr Pro Leu Arg Ile Leu Leu Pro Leu Arg
1               5                   10                  15

Gly Glu Trp Met Gly Arg
            20

<210> 378
<211> 22
<212> PRT
<213> Homo sapiens

<220>
<221> MOD_RES
<222> (7)..(7)
<223> Any amino acid

<400> 378
Met Trp Val Leu Leu Arg Xaa Pro Leu Arg Ile Leu Leu Pro Leu Arg
1               5                   10                  15

Gly Glu Trp Met Gly Arg
            20

<210> 379
<211> 31
<212> PRT
<213> Homo sapiens

<400> 379
Asn Gly Val Gly Pro Glu Glu Glu Ser Val Asp Pro Asn Gln Tyr Tyr
1               5                   10                  15

Lys Ile Arg Ser Gln Ala Ile His Gln Leu Lys Val Asn Gly Glu
            20                  25                  30

<210> 380
<211> 31
<212> PRT
<213> Homo sapiens

<220>
<221> MOD_RES
<222> (16)..(16)
<223> Any amino acid

<400> 380
Asn Gly Val Gly Pro Glu Glu Glu Ser Val Asp Pro Asn Gln Tyr Xaa
1               5                   10                  15

Lys Ile Arg Ser Gln Ala Ile His Gln Leu Lys Val Asn Gly Glu
            20                  25                  30

<210> 381
<211> 31
<212> PRT
<213> Homo sapiens

<400> 381
Gln Ile Glu Gly Gln Met Tyr Gln Gln Tyr Gln Gln Gln Ala Gly Tyr
1               5                   10                  15

Gly Ala Gln Gln Pro Gln Ala Pro Pro Gln Gln Pro Gln Gln Tyr
            20                  25                  30

<210> 382
<211> 31
<212> PRT
<213> Homo sapiens

<220>
<221> MOD_RES
<222> (16)..(16)
<223> Any amino acid

<400> 382
Gln Ile Glu Gly Gln Met Tyr Gln Gln Tyr Gln Gln Gln Ala Gly Xaa
1               5                   10                  15

Gly Ala Gln Gln Pro Gln Ala Pro Pro Gln Gln Pro Gln Gln Tyr
            20                  25                  30

<210> 383
<211> 31
<212> PRT
<213> Homo sapiens

<400> 383
Arg Cys Leu Gln Asn Pro Leu Glu Asn Leu Glu Leu Thr Tyr Gly Tyr
1               5                   10                  15

Leu Leu Glu Glu Asp Met Lys Cys Leu Ser Gln Tyr Pro Ser Leu
            20                  25                  30

<210> 384
<211> 31
<212> PRT
<213> Homo sapiens

<220>
<221> MOD_RES
<222> (16)..(16)
<223> Any amino acid

<400> 384
Arg Cys Leu Gln Asn Pro Leu Glu Asn Leu Glu Leu Thr Tyr Gly Xaa
1               5                   10                  15

Leu Leu Glu Glu Asp Met Lys Cys Leu Ser Gln Tyr Pro Ser Leu
            20                  25                  30

<210> 385
<211> 31
<212> PRT
<213> Homo sapiens

<400> 385
Arg Lys Lys Ser Ala Asp Val Leu Ala Leu Ala Asp Glu Ala Gly Tyr
1               5                   10                  15

Tyr Phe Phe Asn Gly Asn Tyr Lys Val Asp Ser Pro Lys Asn Phe
            20                  25                  30

<210> 386
<211> 31
<212> PRT
<213> Homo sapiens

<220>
<221> MOD_RES
<222> (16)..(16)
<223> Any amino acid

<400> 386
Arg Lys Lys Ser Ala Asp Val Leu Ala Leu Ala Asp Glu Ala Gly Xaa
1               5                   10                  15

Tyr Phe Phe Asn Gly Asn Tyr Lys Val Asp Ser Pro Lys Asn Phe
            20                  25                  30

<210> 387
<211> 31
<212> PRT
<213> Homo sapiens

<400> 387
Arg Lys Leu Leu Met Gln Asp Leu Val Gln Glu Asn Tyr Leu Glu Tyr
1               5                   10                  15

Arg Gln Val Pro Gly Ser Asp Pro Ala Cys Tyr Glu Phe Leu Trp
            20                  25                  30

<210> 388
<211> 31
<212> PRT
<213> Homo sapiens

<220>
<221> MOD_RES
<222> (16)..(16)
<223> Any amino acid

<400> 388
Arg Lys Leu Leu Met Gln Asp Leu Val Gln Glu Asn Tyr Leu Glu Xaa
1               5                   10                  15

Arg Gln Val Pro Gly Ser Asp Pro Ala Cys Tyr Glu Phe Leu Trp
            20                  25                  30

<210> 389
<211> 31
<212> PRT
<213> Homo sapiens

<400> 389
Arg Lys Leu Leu Thr Gln Asp Trp Val Gln Glu Asn Tyr Leu Glu Tyr
1               5                   10                  15

Arg Gln Val Pro Gly Ser Asn Pro Ala Arg Tyr Glu Phe Leu Trp
            20                  25                  30

<210> 390
<211> 31
<212> PRT
<213> Homo sapiens

<220>
<221> MOD_RES
<222> (16)..(16)
<223> Any amino acid

<400> 390
Arg Lys Leu Leu Thr Gln Asp Trp Val Gln Glu Asn Tyr Leu Glu Xaa
1               5                   10                  15

Arg Gln Val Pro Gly Ser Asn Pro Ala Arg Tyr Glu Phe Leu Trp
            20                  25                  30

<210> 391
<211> 31
<212> PRT
<213> Homo sapiens

<400> 391
Ser Ser Asn Thr Ala Gly Lys Thr Leu Phe Gly Lys Met Met Asp Tyr
1               5                   10                  15

Leu Gln Gly Ser Gly Glu Thr Pro Gln Thr Asp Val Arg Trp Met
            20                  25                  30

<210> 392
<211> 31
<212> PRT
<213> Homo sapiens

<220>
<221> MOD_RES
<222> (16)..(16)
<223> Any amino acid

<400> 392
Ser Ser Asn Thr Ala Gly Lys Thr Leu Phe Gly Lys Met Met Asp Xaa
1               5                   10                  15

Leu Gln Gly Ser Gly Glu Thr Pro Gln Thr Asp Val Arg Trp Met
            20                  25                  30

<210> 393
<211> 31
<212> PRT
<213> Homo sapiens

<400> 393
Thr Ala Gly Gly Arg Ala Gln Glu Val Lys Ala Arg Phe Ala Pro Tyr
1               5                   10                  15

Lys Pro Gln Asp Ile Leu Leu Lys Pro Leu Leu Phe Glu Val Pro
            20                  25                  30

<210> 394
<211> 31
<212> PRT
<213> Homo sapiens

<220>
<221> MOD_RES
<222> (16)..(16)
<223> Any amino acid

<400> 394
Thr Ala Gly Gly Arg Ala Gln Glu Val Lys Ala Arg Phe Ala Pro Xaa
1               5                   10                  15

Lys Pro Gln Asp Ile Leu Leu Lys Pro Leu Leu Phe Glu Val Pro
            20                  25                  30

<210> 395
<211> 31
<212> PRT
<213> Homo sapiens

<400> 395
Thr Ala Tyr Ile Arg Val Lys Val Asp Gly Pro Arg Ser Pro Ser Tyr
1               5                   10                  15

Gly Arg Ser Arg Ser Arg Ser Arg Ser Arg Ser Arg Ser Arg Ser
            20                  25                  30

<210> 396
<211> 31
<212> PRT
<213> Homo sapiens

<220>
<221> MOD_RES
<222> (16)..(16)
<223> Any amino acid

<400> 396
Thr Ala Tyr Ile Arg Val Lys Val Asp Gly Pro Arg Ser Pro Ser Xaa
1               5                   10                  15

Gly Arg Ser Arg Ser Arg Ser Arg Ser Arg Ser Arg Ser Arg Ser
            20                  25                  30

<210> 397
<211> 31
<212> PRT
<213> Homo sapiens

<400> 397
Val Phe Leu Val Ser Thr Arg Lys Ile His Thr Thr Met Ser Gly Tyr
1               5                   10                  15

Gln Val Leu Arg Ser Val Leu Gln Phe Leu Ala Thr Thr Asp Leu
            20                  25                  30

<210> 398
<211> 31
<212> PRT
<213> Homo sapiens

<220>
<221> MOD_RES
<222> (16)..(16)
<223> Any amino acid

<400> 398
Val Phe Leu Val Ser Thr Arg Lys Ile His Thr Thr Met Ser Gly Xaa
1               5                   10                  15

Gln Val Leu Arg Ser Val Leu Gln Phe Leu Ala Thr Thr Asp Leu
            20                  25                  30

<210> 399
<211> 31
<212> PRT
<213> Homo sapiens

<400> 399
Tyr Gln Val Ser Gly Ile Leu Asp Phe Gly Asp Met Ser Tyr Gly Tyr
1               5                   10                  15

Tyr Val Phe Glu Val Ala Ile Thr Ile Met Tyr Met Met Ile Glu
            20                  25                  30

<210> 400
<211> 31
<212> PRT
<213> Homo sapiens

<220>
<221> MOD_RES
<222> (16)..(16)
<223> Any amino acid

<400> 400
Tyr Gln Val Ser Gly Ile Leu Asp Phe Gly Asp Met Ser Tyr Gly Xaa
1               5                   10                  15

Tyr Val Phe Glu Val Ala Ile Thr Ile Met Tyr Met Met Ile Glu
            20                  25                  30

<210> 401
<211> 11
<212> PRT
<213> Homo sapiens

<400> 401
Gln Leu Asp Gln Glu Ser Gly Ala Val Ile Pro
1               5                   10

<210> 402
<211> 11
<212> PRT
<213> Homo sapiens

<400> 402
Gln Leu Asp Gln Glu Ser Gly Ala Val Ile His
1               5                   10

<210> 403
<211> 8
<212> PRT
<213> Homo sapiens

<400> 403
Tyr Leu Ala Asn Gln Trp Gln Gly
1               5

<210> 404
<211> 8
<212> PRT
<213> Homo sapiens

<400> 404
Tyr Leu Ala Asn Gln Trp Gln Val
1               5

<210> 405
<211> 10
<212> PRT
<213> Homo sapiens

<400> 405
Ser Pro Arg Arg Cys Gln Pro Ile Glu Phe
1               5                   10

<210> 406
<211> 10
<212> PRT
<213> Homo sapiens

<400> 406
Ser Thr Arg Arg Cys Gln Pro Ile Glu Phe

1                 5                    10


<210> 407
<211> 9
<212> PRT
<213> Homo sapiens

<400> 407
Cys Arg Gln Thr Gln Leu Leu Pro Tyr
1               5


<210> 408
<211> 9
<212> PRT
<213> Homo sapiens

<400> 408
Cys Leu Gln Thr Gln Leu Leu Pro Tyr
1               5


<210> 409
<211> 11
<212> PRT
<213> Homo sapiens

<400> 409
Cys Arg Gln Thr Gln Leu Leu Pro Tyr Leu Ala
1               5                   10


<210> 410
<211> 11
<212> PRT
<213> Homo sapiens

<400> 410
Cys Leu Gln Thr Gln Leu Leu Pro Tyr Leu Ala
1               5                   10


<210> 411
<211> 11
<212> PRT
<213> Homo sapiens

<400> 411
Phe His Glu Lys His Glu Ser Glu Asn Leu Ser
1               5                   10


<210> 412
<211> 11
<212> PRT
<213> Homo sapiens

<400> 412
Phe His Glu Lys His Glu Ser Glu Asn Leu Tyr
1               5                   10

<210> 413
<211> 8
<212> PRT
<213> Homo sapiens

<400> 413
Lys His Glu Ser Glu Asn Leu Ser
1               5


<210> 414
<211> 8
<212> PRT
<213> Homo sapiens

<400> 414
Lys His Glu Ser Glu Asn Leu Tyr
1               5


<210> 415
<211> 11
<212> PRT
<213> Homo sapiens

<400> 415
Asn Ser Asp Ile Asn Glu Trp Ser Leu Ile Ala
1               5                   10


<210> 416
<211> 11
<212> PRT
<213> Homo sapiens

<400> 416
Asn Ser Asp Ile Asn Glu Trp Ser Leu Ile Thr
1               5                   10


<210> 417
<211> 9
<212> PRT
<213> Homo sapiens

<400> 417
Ile Ala Ser Ser Pro His Pro Glu Tyr
1               5


<210> 418
<211> 9
<212> PRT
<213> Homo sapiens

<400> 418
Ile Thr Ser Ser Pro His Pro Glu Tyr
1               5


<210> 419
<211> 9
<212> PRT

<213> Homo sapiens

<400> 419
Ile Ser Ala Phe Leu Val Met Gly Val
1               5


<210> 420
<211> 9
<212> PRT
<213> Homo sapiens

<400> 420
Ile Cys Ala Phe Leu Val Met Gly Val
1               5


<210> 421
<211> 8
<212> PRT
<213> Homo sapiens

<400> 421
Trp Leu Gly Phe Gly His Lys Phe
1               5


<210> 422
<211> 8
<212> PRT
<213> Homo sapiens

<400> 422
Trp Leu Asp Phe Gly His Lys Phe
1               5


<210> 423
<211> 8
<212> PRT
<213> Homo sapiens

<400> 423
Thr Leu Asp Arg Val Leu Pro Arg
1               5


<210> 424
<211> 8
<212> PRT
<213> Homo sapiens

<400> 424
Thr Leu Glu Arg Val Leu Pro Arg
1               5


<210> 425
<211> 9
<212> PRT
<213> Homo sapiens

<400> 425

Thr Leu Asp Arg Val Leu Pro Arg Val
1               5

<210> 426
<211> 9
<212> PRT
<213> Homo sapiens

<400> 426
Thr Leu Glu Arg Val Leu Pro Arg Val
1               5

<210> 427
<211> 11
<212> PRT
<213> Homo sapiens

<400> 427
Thr Leu Asp Arg Val Leu Pro Arg Val Arg Phe
1               5                   10

<210> 428
<211> 11
<212> PRT
<213> Homo sapiens

<400> 428
Thr Leu Glu Arg Val Leu Pro Arg Val Arg Phe
1               5                   10

<210> 429
<211> 9
<212> PRT
<213> Homo sapiens

<400> 429
Asn Phe Ser His Arg Gln Val Arg Asp
1               5

<210> 430
<211> 9
<212> PRT
<213> Homo sapiens

<400> 430
Asn Phe Ser His Arg Gln Val Arg Tyr
1               5

<210> 431
<211> 8
<212> PRT
<213> Homo sapiens

<400> 431
Phe Ser His Arg Gln Val Arg Asp
1               5

<210> 432
<211> 8
<212> PRT
<213> Homo sapiens

<400> 432
Phe Ser His Arg Gln Val Arg Tyr
1               5


<210> 433
<211> 11
<212> PRT
<213> Homo sapiens

<400> 433
Gly Ser Glu Ser Glu Asn Ser Asn Arg Glu His
1               5                   10


<210> 434
<211> 11
<212> PRT
<213> Homo sapiens

<400> 434
Gly Ser Glu Ser Glu Asn Ser Asn Arg Glu Tyr
1               5                   10


<210> 435
<211> 10
<212> PRT
<213> Homo sapiens

<400> 435
Ser Glu Ser Glu Asn Ser Asn Arg Glu His
1               5                   10


<210> 436
<211> 10
<212> PRT
<213> Homo sapiens

<400> 436
Ser Glu Ser Glu Asn Ser Asn Arg Glu Tyr
1               5                   10


<210> 437
<211> 9
<212> PRT
<213> Homo sapiens

<400> 437
Glu Ser Glu Asn Ser Asn Arg Glu His
1               5


<210> 438
<211> 9

<212> PRT
<213> Homo sapiens

<400> 438
Glu Ser Glu Asn Ser Asn Arg Glu Tyr
1               5


<210> 439
<211> 9
<212> PRT
<213> Homo sapiens

<400> 439
Thr Ala Asp Ala Ala Arg Gln Ile Cys
1               5


<210> 440
<211> 9
<212> PRT
<213> Homo sapiens

<400> 440
Thr Thr Asp Ala Ala Arg Gln Ile Cys
1               5


<210> 441
<211> 10
<212> PRT
<213> Homo sapiens

<400> 441
Thr Ala Asp Ala Ala Arg Gln Ile Cys Glu
1               5                   10


<210> 442
<211> 10
<212> PRT
<213> Homo sapiens

<400> 442
Thr Thr Asp Ala Ala Arg Gln Ile Cys Glu
1               5                   10


<210> 443
<211> 11
<212> PRT
<213> Homo sapiens

<400> 443
Thr Ala Asp Ala Ala Arg Gln Ile Cys Glu Met
1               5                   10


<210> 444
<211> 11
<212> PRT
<213> Homo sapiens

<400> 444
Thr Thr Asp Ala Ala Arg Gln Ile Cys Glu Met
1               5                   10

<210> 445
<211> 9
<212> PRT
<213> Homo sapiens

<400> 445
Asn Leu Gln Asp Leu Ala His Ile Tyr
1               5

<210> 446
<211> 9
<212> PRT
<213> Homo sapiens

<400> 446
Asn Leu Gln Asp Leu Ala Arg Ile Tyr
1               5

<210> 447
<211> 8
<212> PRT
<213> Homo sapiens

<400> 447
Leu Gln Asp Leu Ala His Ile Tyr
1               5

<210> 448
<211> 8
<212> PRT
<213> Homo sapiens

<400> 448
Leu Gln Asp Leu Ala Arg Ile Tyr
1               5

<210> 449
<211> 9
<212> PRT
<213> Homo sapiens

<400> 449
Pro Leu Gly Glu Gly Thr Glu Gln Cys
1               5

<210> 450
<211> 9
<212> PRT
<213> Homo sapiens

<400> 450
Pro Leu Gly Glu Gly Thr Glu Gln Tyr
1               5

<210> 451
<211> 8
<212> PRT
<213> Homo sapiens

<400> 451
Leu Gly Glu Gly Thr Glu Gln Cys
1               5


<210> 452
<211> 8
<212> PRT
<213> Homo sapiens

<400> 452
Leu Gly Glu Gly Thr Glu Gln Tyr
1               5


<210> 453
<211> 11
<212> PRT
<213> Homo sapiens

<400> 453
His Thr Asn Pro Val Gly Thr Glu Trp Arg Gly
1               5                   10


<210> 454
<211> 11
<212> PRT
<213> Homo sapiens

<400> 454
His Thr Asn Pro Val Gly Thr Glu Trp Arg Trp
1               5                   10


<210> 455
<211> 11
<212> PRT
<213> Homo sapiens

<400> 455
His Thr Gly Ile His Ala Gly Glu Lys Pro Phe
1               5                   10


<210> 456
<211> 11
<212> PRT
<213> Homo sapiens

<400> 456
His Thr Gly Ile His Ala Gly Glu Lys Pro Tyr
1               5                   10


<210> 457

<211> 10
<212> PRT
<213> Homo sapiens

<400> 457
Arg Ile Gln Pro Glu Asp Met Phe Val Tyr
1               5                   10


<210> 458
<211> 10
<212> PRT
<213> Homo sapiens

<400> 458
Arg Ile Gln Pro Glu Asp Met Phe Val Cys
1               5                   10


<210> 459
<211> 8
<212> PRT
<213> Homo sapiens

<400> 459
Gln Pro Glu Asp Met Phe Val Tyr
1               5


<210> 460
<211> 8
<212> PRT
<213> Homo sapiens

<400> 460
Gln Pro Glu Asp Met Phe Val Cys
1               5


<210> 461
<211> 11
<212> PRT
<213> Homo sapiens

<400> 461
Thr Gly Ser Val Leu Gly Phe Phe Ile Gln Ser
1               5                   10


<210> 462
<211> 11
<212> PRT
<213> Homo sapiens

<400> 462
Thr Gly Ser Val Leu Gly Phe Phe Ile Gln Tyr
1               5                   10


<210> 463
<211> 10
<212> PRT
<213> Homo sapiens

<400> 463
Gly Ser Val Leu Gly Phe Phe Ile Gln Ser
1               5                   10

<210> 464
<211> 10
<212> PRT
<213> Homo sapiens

<400> 464
Gly Ser Val Leu Gly Phe Phe Ile Gln Tyr
1               5                   10

<210> 465
<211> 9
<212> PRT
<213> Homo sapiens

<400> 465
Ser Val Leu Gly Phe Phe Ile Gln Ser
1               5

<210> 466
<211> 9
<212> PRT
<213> Homo sapiens

<400> 466
Ser Val Leu Gly Phe Phe Ile Gln Tyr
1               5

<210> 467
<211> 8
<212> PRT
<213> Homo sapiens

<400> 467
Val Leu Gly Phe Phe Ile Gln Ser
1               5

<210> 468
<211> 8
<212> PRT
<213> Homo sapiens

<400> 468
Val Leu Gly Phe Phe Ile Gln Tyr
1               5

<210> 469
<211> 9
<212> PRT
<213> Homo sapiens

<400> 469
Tyr Pro Ala Thr Leu Ser Ile Asn Thr

1               5


<210> 470
<211> 9
<212> PRT
<213> Homo sapiens

<400> 470
Tyr Thr Ala Thr Leu Ser Ile Asn Thr
1               5


<210> 471
<211> 11
<212> PRT
<213> Homo sapiens

<400> 471
Ala Phe Glu Asp Thr Ser Phe Ala Ser Leu Tyr
1               5                   10


<210> 472
<211> 11
<212> PRT
<213> Homo sapiens

<400> 472
Ala Phe Glu Asp Thr Ser Phe Ala Ser Leu Cys
1               5                   10


<210> 473
<211> 10
<212> PRT
<213> Homo sapiens

<400> 473
Phe Glu Asp Thr Ser Phe Ala Ser Leu Tyr
1               5                   10


<210> 474
<211> 10
<212> PRT
<213> Homo sapiens

<400> 474
Phe Glu Asp Thr Ser Phe Ala Ser Leu Cys
1               5                   10


<210> 475
<211> 8
<212> PRT
<213> Homo sapiens

<400> 475
Asp Thr Ser Phe Ala Ser Leu Tyr
1               5

<210> 476
<211> 8
<212> PRT
<213> Homo sapiens

<400> 476
Asp Thr Ser Phe Ala Ser Leu Cys
1               5


<210> 477
<211> 9
<212> PRT
<213> Homo sapiens

<400> 477
Lys Lys Asp Asp Ile Pro Glu Glu Tyr
1               5


<210> 478
<211> 9
<212> PRT
<213> Homo sapiens

<400> 478
Lys Lys Asp Asp Ile Pro Glu Glu Asp
1               5


<210> 479
<211> 8
<212> PRT
<213> Homo sapiens

<400> 479
Lys Asp Asp Ile Pro Glu Glu Tyr
1               5


<210> 480
<211> 8
<212> PRT
<213> Homo sapiens

<400> 480
Lys Asp Asp Ile Pro Glu Glu Asp
1               5


<210> 481
<211> 9
<212> PRT
<213> Homo sapiens

<400> 481
Pro Ser Ser Val Ala Ala Leu Arg Phe
1               5


<210> 482
<211> 9
<212> PRT

<213> Homo sapiens

<400> 482
Pro Ser Ser Val Ala Ala Leu Arg Asp
1               5


<210> 483
<211> 8
<212> PRT
<213> Homo sapiens

<400> 483
Ser Ser Val Ala Ala Leu Arg Phe
1               5


<210> 484
<211> 8
<212> PRT
<213> Homo sapiens

<400> 484
Ser Ser Val Ala Ala Leu Arg Asp
1               5


<210> 485
<211> 10
<212> PRT
<213> Homo sapiens

<400> 485
Thr Leu Ala Leu Ser Gly Ala Leu Cys Phe
1               5                   10


<210> 486
<211> 10
<212> PRT
<213> Homo sapiens

<400> 486
Thr Leu Ala Leu Ser Gly Ala Leu Cys Val
1               5                   10


<210> 487
<211> 11
<212> PRT
<213> Homo sapiens

<400> 487
Leu Leu Lys Lys Lys Tyr Glu Gly His Trp Ser
1               5                   10


<210> 488
<211> 11
<212> PRT
<213> Homo sapiens

<400> 488

Leu Leu Lys Lys Lys Tyr Glu Gly His Trp Tyr
1               5                   10

<210> 489
<211> 9
<212> PRT
<213> Homo sapiens

<400> 489
Leu Leu Asp Ala Leu Tyr Gly Lys Gly
1               5

<210> 490
<211> 9
<212> PRT
<213> Homo sapiens

<400> 490
Leu Leu Asp Ala Leu Tyr Gly Lys Val
1               5

<210> 491
<211> 10
<212> PRT
<213> Homo sapiens

<400> 491
Gly Ala Met Thr Ile Thr Gly Asn Ile Tyr
1               5                   10

<210> 492
<211> 10
<212> PRT
<213> Homo sapiens

<400> 492
Gly Ser Met Thr Ile Thr Gly Asn Ile Tyr
1               5                   10

<210> 493
<211> 11
<212> PRT
<213> Homo sapiens

<400> 493
Asp Ala Glu Asp Leu Ser Pro Asn Trp Pro Leu
1               5                   10

<210> 494
<211> 11
<212> PRT
<213> Homo sapiens

<400> 494
Asp Thr Glu Asp Leu Ser Pro Asn Trp Pro Leu
1               5                   10

<210> 495
<211> 11
<212> PRT
<213> Homo sapiens

<400> 495
Gln Leu Asn Asp Val Lys Thr Thr Val Val Ser
1               5                   10


<210> 496
<211> 11
<212> PRT
<213> Homo sapiens

<400> 496
Gln Leu Asn Asp Val Lys Thr Thr Val Val Tyr
1               5                   10


<210> 497
<211> 10
<212> PRT
<213> Homo sapiens

<400> 497
Leu Asn Asp Val Lys Thr Thr Val Val Ser
1               5                   10


<210> 498
<211> 10
<212> PRT
<213> Homo sapiens

<400> 498
Leu Asn Asp Val Lys Thr Thr Val Val Tyr
1               5                   10


<210> 499
<211> 11
<212> PRT
<213> Homo sapiens

<400> 499
Trp Ala Cys Ser His Ser Met Arg Tyr Phe Tyr
1               5                   10


<210> 500
<211> 11
<212> PRT
<213> Homo sapiens

<400> 500
Trp Ala Cys Ser His Ser Met Arg Tyr Phe Asp
1               5                   10


<210> 501
<211> 10

<212> PRT
<213> Homo sapiens

<400> 501
Ala Cys Ser His Ser Met Arg Tyr Phe Tyr
1               5                   10


<210> 502
<211> 10
<212> PRT
<213> Homo sapiens

<400> 502
Ala Cys Ser His Ser Met Arg Tyr Phe Asp
1               5                   10


<210> 503
<211> 9
<212> PRT
<213> Homo sapiens

<400> 503
Cys Ser His Ser Met Arg Tyr Phe Tyr
1               5


<210> 504
<211> 9
<212> PRT
<213> Homo sapiens

<400> 504
Cys Ser His Ser Met Arg Tyr Phe Asp
1               5


<210> 505
<211> 8
<212> PRT
<213> Homo sapiens

<400> 505
Ser His Ser Met Arg Tyr Phe Tyr
1               5


<210> 506
<211> 8
<212> PRT
<213> Homo sapiens

<400> 506
Ser His Ser Met Arg Tyr Phe Asp
1               5


<210> 507
<211> 11
<212> PRT
<213> Homo sapiens

<400> 507
Ala Ile Asn Gly Met Asp Val Asn Gly Arg Asp
1               5                   10


<210> 508
<211> 11
<212> PRT
<213> Homo sapiens

<400> 508
Ala Ile Asn Gly Met Asp Val Asn Gly Arg Tyr
1               5                   10


<210> 509
<211> 9
<212> PRT
<213> Homo sapiens

<400> 509
Asn Gly Met Asp Val Asn Gly Arg Asp
1               5


<210> 510
<211> 9
<212> PRT
<213> Homo sapiens

<400> 510
Asn Gly Met Asp Val Asn Gly Arg Tyr
1               5


<210> 511
<211> 8
<212> PRT
<213> Homo sapiens

<400> 511
Gly Met Asp Val Asn Gly Arg Asp
1               5


<210> 512
<211> 8
<212> PRT
<213> Homo sapiens

<400> 512
Gly Met Asp Val Asn Gly Arg Tyr
1               5


<210> 513
<211> 8
<212> PRT
<213> Homo sapiens

<400> 513
Asp Thr Ala Asp Gly Lys Glu Val
1               5

<210> 514
<211> 8
<212> PRT
<213> Homo sapiens

<400> 514
Tyr Thr Ala Asp Gly Lys Glu Val
1               5


<210> 515
<211> 9
<212> PRT
<213> Homo sapiens

<400> 515
Asp Thr Ala Asp Gly Lys Glu Val Leu
1               5


<210> 516
<211> 9
<212> PRT
<213> Homo sapiens

<400> 516
Tyr Thr Ala Asp Gly Lys Glu Val Leu
1               5


<210> 517
<211> 11
<212> PRT
<213> Homo sapiens

<400> 517
Asp Thr Ala Asp Gly Lys Glu Val Leu Glu Tyr
1               5                   10


<210> 518
<211> 11
<212> PRT
<213> Homo sapiens

<400> 518
Tyr Thr Ala Asp Gly Lys Glu Val Leu Glu Tyr
1               5                   10